(19) Europäisches Patentamt
European Patent Office
Office européen des brevets

(11) EP 1 658 067 B1

(12) **EUROPEAN PATENT SPECIFICATION**

(45) Date of publication and mention of the grant of the patent:
**12.11.2008 Bulletin 2008/46**

(21) Application number: **04743655.5**

(22) Date of filing: **04.08.2004**

(51) Int Cl.:
***A61K 31/404*** *(2006.01)* ***C07D 209/42*** *(2006.01)*
***A61P 3/12*** *(2006.01)*

(86) International application number:
**PCT/GB2004/003364**

(87) International publication number:
**WO 2005/013975 (17.02.2005 Gazette 2005/07)**

(54) **INDOLAMIDE DERIVATIVES WHICH POSSESS GLYCOGEN PHOSPHORYLASE INHIBITORY ACTIVITY**

INDOLAMID-DERIVATE, DIE GLYCOGEN PHOSPHORYLASE INHIBIERENDE AKTIVITÄT BESITZEN

DERIVES D'INDOLAMIDE POSSEDANT UNE ACTIVITE INHIBITRICE DE LA GLYCOGENE PHOSPHORYLASE

(84) Designated Contracting States:
**AT BE BG CH CY CZ DE DK EE ES FI FR GB GR HU IE IT LI LU MC NL PL PT RO SE SI SK TR**

(30) Priority: **07.08.2003 GB 0318464**

(43) Date of publication of application:
**24.05.2006 Bulletin 2006/21**

(73) Proprietor: **AstraZeneca AB**
**151 85 Södertälje (SE)**

(72) Inventors:
- **BIRCH, Alan, Martin**
  **Macclesfield, Cheshire SK10 4TG (GB)**
- **BENNETT, Stuart, Norman, Lile**
  **Macclesfield, Cheshire SK10 4TG (GB)**
- **GODFREY, Linda**
  **Macclesfield, Cheshire SK10 4TG (GB)**
- **SIMPSON, Iain**
  **Macclesfield, Cheshire SK10 4TG (GB)**
- **WHITTAMORE, Paul, Robert, Owen**
  **Macclesfield, Cheshire SK10 4TG (GB)**

(56) References cited:
**EP-A- 1 136 071**
**WO-A-02/20530**
**WO-A-03/037864**
**WO-A-03/074484**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 658 067 B1

Printed by Jouve, 75001 PARIS (FR)

## Description

The present invention relates to heterocyclic amide derivatives, pharmaceutically acceptable salts and in-vivo hydrolysable esters thereof. These heterocyclic amide possess glycogen phosphorylase inhibitory activity and accordingly have value in the treatment of disease states associated with increased glycogen phosphorylase activity and thus are potentially useful in methods of treatment of a warm-blooded animal such as man. The invention also relates to processes for the manufacture of said heterocyclic amide derivatives, to pharmaceutical compositions containing them and to their use in the manufacture of medicaments to inhibit glycogen phosphorylase activity in a warm-blooded animal such as man.

The liver is the major organ regulating glycaemia in the post-absorptive state. Additionally, although having a smaller role in the contribution to post-prandial blood glucose levels, the response of the liver to exogenous sources of plasma glucose is key to an ability to maintain euglycaemia. An increased hepatic glucose output (HGO) is considered to play an important role in maintaining the elevated fasting plasma glucose (FPG) levels seen in type 2 diabetics; particularly those with a FPG >140mg/dl (7.8mM). (Weyer et al, (1999), J Clin Invest 104: 787-794; Clore & Blackgard (1994), Diabetes 43: 256-262; De Fronzo, R. A., et al, (1992) Diabetes Care 15; 318 - 355; Reaven, G.M. (1995) Diabetologia 38; 3-13).

Since current oral, anti-diabetic therapies fail to bring FPG levels to within the normal, non-diabetic range and since raised FPG (and glycHbAlc) levels are risk factors for both macro- (Charles, M.A. et al (1996) Lancet 348, 1657-1658; Coutinho, M. et al (1999) Diabetes Care 22; 233-240; Shaw, J.E. et al (2000) Diabetes Care 23, 34-39) and micro-vascular disease (DCCT Research Group (1993) New. Eng. J. Med. 329; 977-986); the reduction and normalisation of elevated FPG levels remains a treatment goal in type 2 DM.

It has been estimated that, after an overnight fast, 74% of HGO was derived from glycogenolysis with the remainder derived from gluconeogenic precursors (Hellerstein et al (1997) Am J Physiol, 272: E163). Glycogen phosphorylase is a key enzyme in the generation by glycogenolysis of glucose-1-phosphate, and hence glucose in liver and also in other tissues such as muscle and neuronal tissue.

Liver glycogen phosphorylase a activity is elevated in diabetic animal models including the db/db mouse and the fa/fa rat (Aiston S et al (2000). Diabetalogia 43, 589-597).

Inhibition of hepatic glycogen phosphorylase with chloroindole inhibitors (CP91149 and CP320626) has been shown to reduce both glucagon stimulated glycogenolysis and glucose output in hepatocytes (Hoover et al (1998) J Med Chem 41, 2934-8; Martin et al (1998) PNAS 95, 1776-81). Additionally, plasma glucose concentration is reduced, in a dose related manner, db/db and ob/ob mice following treatment with these compounds.

Studies in conscious dogs with glucagon challenge in the absence and presence of another glycogen phosphorylase inhibitor, Bay K 3401, also show the potential utility of such agents where there is elevated circulating levels of glucagon, as in both Type 1 and Type 2 diabetes. In the presence of Bay R 3401, hepatic glucose output and arterial plasma glucose following a glucagon challenge were reduced significantly (Shiota et al, (1997), Am J Physiol, 273: E868).

The heterocyclic amides of the present invention possess glycogen phosphorylase inhibitory activity and accordingly are expected to be of use in the treatment of type 2 diabetes, insulin resistance, syndrome X, hyperinsulinaemia, hyperglucagonaemia, cardiac ischaemia and obesity, particularly type 2 diabetes.

According to one aspect of the present invention there is provided a compound of formula (1):

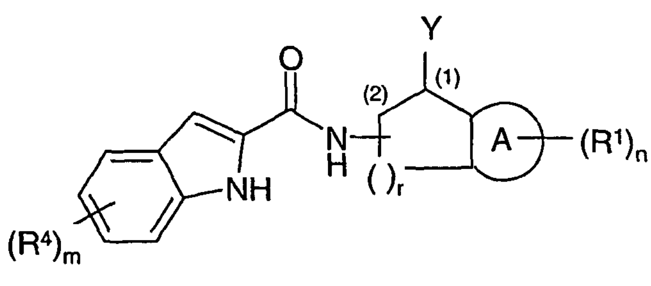

**(1)**

wherein:

A is phenylene or heteroarylene;
n is 0, 1 or 2;
m is 0, 1 or 2;
$R^1$ is independently selected from halo, nitro, cyano, hydroxy, carboxy, carbamoyl, *N*-(1-4C)alkylcarbamoyl, *N,*

*N*-((1-4C)alkyl)$_2$carbamoyl, sulphamoyl, *N*-(1-4C)alkylsulphamoyl, *N,N*-((1-4C)alkyl)$_2$sulphamoyl, -S(O)$_b$(1-4C)alkyl (wherein b is 0,1,or 2), -OS(O)$_2$(1-4C)alkyl, (1-4C)alkyl, (2-4C)alkenyl, (2-4C)alkynyl, (1-4C)alkoxy, (1-4C)alkanoyl, (1-4C)alkanoyloxy, hydroxy(1-4C)alkyl, fluoromethyl, difluoromethyl, trifluoromethyl, trifluoromethoxy and-NHSO$_2$ (1-4C)alkyl;

or, when n is 2, the two $R^1$ groups, together with the carbon atoms of A to which they are attached, may form a 4 to 7 membered saturated ring, optionally containing 1 or 2 heteroatoms independently selected from O, S and N, and optionally being substituted by one or two methyl groups;

$R^4$ is independently selected from halo, nitro, cyano, hydroxy, fluoromethyl, difluoromethyl, trifluoromethyl, trifluoromethoxy, carboxy, carbamoyl, (1-4C)alkyl, (2-4C)alkenyl, (2-4C)alkynyl, (1-4C)alkoxy and (1-4C)alkanoyl;

r is 1 or 2; and

when r is 1 the group

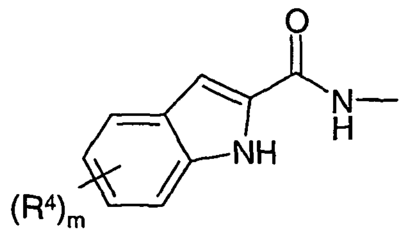

is a substituent on carbon (2) and

when r is 2 (thereby forming a six membered ring) the same group is a substituent on carbon (2) or on carbon (3);

Y is selected from -C(O)$R^2$, -C(O)O$R^2$, -C(O)N$R^2R^3$, -(1-4C)alkyl [optionally substituted by 1 or 2 substituents independently selected from hydroxy, -C=N$R^2$, (1-4C)alkoxy, aryloxy, heterocyclyloxy, -S(O)$_b$$R^2$ (wherein b is 0, 1 or 2), -O-S(O)$_b$$R^2$ (wherein b is 0,1 or 2), -N$R^2R^3$, -N(OH)$R^2$, -N$R^2$C(=O)$R^2$, -NHOHC(=O)$R^2$, -$SO_2$N$R^2R^3$, -N($R^2$)$SO_2R^2$, aryl and heterocyclyl], -C(O)NOH, -C(O)NSH, -C(N)OH, -C(N)SH, -$SO_2$H, -$SO_3$H, -$SO_2$N(OH)$R^2$, -(2-4C)alkenyl, -$SO_2$N$R^2R^3$, -(1-4C)alkylC(O)$R^2$, -(1-4C)alkylC(O)O$R^2$, -(1-4C)alkylSC(O)$R^2$, -(1-4C)alkylOC(O)$R^2$, -(1-4C)alkylC(O)N$R^2R^3$, -(1-4C)alkylOC(O)O$R^2$, -(1-4C)alkylN($R^2$)C(O)O$R^2$, -(1-4C)alkylN($R^2$)C(O)N$R^2R^3$, -(1-4C)alkylOC(O)N$R^2R^3$, (3-6C)cycloalkyl (optionally substituted by 1 or 2 $R^8$), aryl, heterocyclyl (wherein the heterocyclic ring is linked by a ring carbon atom), -(1-4C)alkyl$SO_2$(2-4C)alkenyl and -S(O)$_c$$R^2$ (wherein c is 0, 1 or 2);

$R^2$ and $R^3$ are independently selected from hydrogen, -O(1-4C)alkyl, -S(1-4C)alkyl, -N(1-4C)alkyl, heterocyclyl, aryl and (1-4C)alkyl [optionally substituted by 1 or 2 $R^8$ groups]; or

wherein N$R^2R^3$ may form a 4 to 7 membered saturated, partially saturated or unsaturated ring, optionally containing 1, 2 or 3 additional heteroatoms independently selected from N, O and S (provided there are no O-O, O-S or S-S bonds), wherein any -$CH_2$- may optionally be replaced by -C(=O)-, and any N or S atom may optionally be oxidised to form an N-oxide or SO or $SO_2$ group respectively, and wherein the ring is optionally substituted by 1 or 2 substituents independently selected from halo, cyano, (1-4C)alkyl, hydroxy, (1-4C)alkoxy and (1-4C)alkylS(O)$_b$- (wherein b is 0, 1 or 2);

$R^8$ is independently selected from hydrogen, hydroxy, (1-4C)alkyl, (2-4C)alkenyl, (1-4C)alkoxy, cyano(1-4C)alkyl, amino(1-4C)alkyl [optionally substituted on nitrogen by 1 or 2 groups selected from (1-4C)alkyl, hydroxy, hydroxy(1-4C)alkyl, dihydroxy(1-4C)alkyl, -$CO_2$(1-4C)alkyl, aryl and aryl(1-4C)alkyl], halo(1-4C)alkyl, dihalo(1-4C)alkyl, trihalo(1-4C)alkyl, hydroxy(1-4C)alkyl, dihydroxy(1-4C)alkyl, (1-4C)alkoxy(1-4C)alkoxy, (1-4C)alkoxy(1-4C)alkyl, hydroxy(1-4C)alkoxy, 5- and 6-membered cyclic acetals and mono- and di-methyl derivatives thereof, aryl, heterocyclyl, heterocyclyl(1-4C)alkyl, (3-7C)cycloalkyl (optionally substituted with 1 or 2 hydroxy groups, (1-4C)alkyl or -$CO_2$(1-4C)alkyl), (1-4C)alkanoyl, (1-4C)alkylS(O)$_b$- (wherein b is 0, 1 or 2), (3-6C)cycloalkylS(O)$_b$- (wherein b is 0, 1 or 2), arylS(O)$_b$- (wherein b is 0, 1 or 2), heterocyclylS(O)$_b$- (wherein b is 0, 1 or 2), benzylS(O)$_b$- (wherein b is 0, 1 or 2), (1-4C)alkylS(O)$_c$((1-4C))alkyl (wherein c is 0, 1 or 2), -N(OH)CHO, -C(=N-OH)$NH_2$, -C(=N-OH)NH(1-4C)alkyl, -C(=N-OH)N((1-4C)alkyl)$_2$, -C(=N-OH)NH(3-6C)cycloalkyl, -C(=N-OH)N((3-6C)cycloalkyl)$_2$, -COOO$R^9$, -C(O)N($R^9$)($R^{10}$), -NHC(O)$R^9$, -C(O)NH$SO_2$(1-4C)alkyl, -NH$SO_2R^9$, ($R^9$)($R^{10}$)N$SO_2$-, -COC$H_2$O$R^{11}$, -COC$H_2$OH, ($R^9$)($R^{10}$)N-, -COO$R^9$, -C$H_2$O$R^9$, -C$H_2$COO$R^9$, -C$H_2$OCO$R^9$, -C$H_2$CH($CO_2R^9$)OH, - C$H_2$C(O)N$R^9R^{10}$, -(C$H_2$)$_w$CH(N$R^9R^{10}$)$CO_2R^{9'}$ (wherein w is 1, 2 or 3), and -(C$H_2$)$_w$CH(N$R^9R^{10}$)CO(N$R^{9'}R^{10'}$) (wherein w is 1, 2 or 3) ;

$R^9$, $R^{9'}$, $R^{10}$ and $R^{10'}$ are independently selected from hydrogen, hydroxy, (1-4C)alkyl (optionally substituted by 1 or 2 $R^{11}$), (2-4C)alkenyl, (3-7C)cycloalkyl (optionally substituted by 1 or 2 hydroxy groups), cyano((1-4C))alkyl, trihalo(1-4C)alkyl, aryl, heterocyclyl, heterocyclyl(1-4C)alkyl, -$CO_2$(1-4C)alkyl; or

$R^9$ and $R^{10}$ together with the nitrogen to which they are attached, and/or $R^{9'}$ and $R^{10'}$ together with the nitrogen to which they are attached, form a 4- to 6-membered ring where the ring is optionally substituted on carbon by 1 or 2

substituents independently selected from oxo, hydroxy, carboxy, halo, nitro, cyano, carbonyl, (1-4C)alkoxy and heterocyclyl; or the ring may be optionally substituted on two adjacent carbons by $-O-CH_2-O-$ to form a cyclic acetal wherein one or both of the hydrogens of the $-O-CH_2-O-$ group may be replaced by a methyl;
$R^{11}$ is independently selected from (1-4C)alkyl, and hydroxy(1-4C)alkyl;

or a pharmaceutically acceptable salt thereof.

In a further aspect of the invention, there is provided as compound of the formula (1) as hereinbefore defined, or a pharmaceutically-acceptable salt, or in-vivo hydrolysable ester thereof, wherein Y is selected from $-C(O)R^2$, $-C(O)OR^2$, $-C(O)NR^2R^3$, -(1-4C)alkyl [optionally substituted by 1 or 2 substituents independently selected from hydroxy, $-C=NR^2$, (1-4C)alkoxy, aryloxy, heterocyclyloxy, $-S(O)_bR^2$ (wherein b is 0, 1 or 2), $-O-S(O)_bR^2$ (wherein b is 0, 1 or 2), $-NR^2R^3$, $-N(OH)R^2$, $-NR^2C(=O)R^2$, $-NHOHC(=O)R^2$, $-SO_2NR^2R^3$, $-N(R^2)SO_2R^2$ , aryl and heterocyclyl], -C(O)NOH, -C(O)NSH, -C(N)OH, -C(N)SH, $-SO_2H$, $-SO_3H$, $-SO_2N(OH)R^2$, -(2-4C)alkenyl, $-SO_2NR^2R^3$, -(1-4C)alkylC(O)$R^2$, -(1-4C)alkylC(O)O$R^2$, -(1-4C)alkylOC(O)$R^2$, - (1-4C)alkylC(O)N$R^2R^3$, -(1-4C)alkylOC(O)O$R^2$, -(1-4-C)alkylN($R^2$)C(O)O$R^2$, -(1-4C)alkylN($R^2$)C(O)N$R^2R^3$, -(1-4C)alkylOC(O)N$R^2R^3$, (3-6C)cycloalkyl (optionally substituted by 1 or 2 $R^8$), aryl, heterocyclyl (wherein the heterocyclic ring is linked by a ring carbon atom), and $-S(O)_cR^2$ (wherein c is 0, 1 or 2).

It is to be understood that when A is heteroarylene, the bridgehead atoms joining ring A to the ring may be heteroatoms. Therefore, for example, the definition of

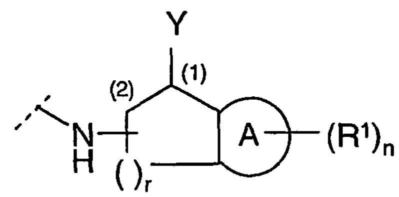

when A is heteroarylene encompasses the structures:

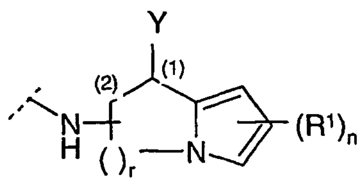
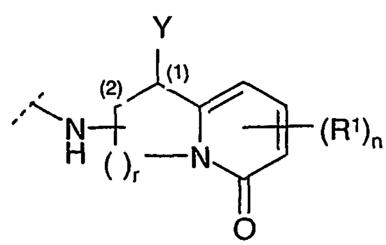
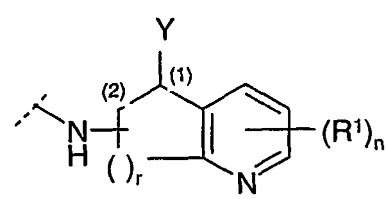

It is to be understood that, where optional substitution on alkyl or cycloalkyl groups in Y, $R^3$, $R^9$ and $R^{10}$ (as defined hereinbefore or hereinafter) allows two hydroxy substituents on the alkyl or cycloalkyl group, or one hydroxy substituent and a second substituent linked by a heteroatom (for example alkoxy), then these two substituents are not substituents on the same carbon atom of the alkyl or cycloalkyl group.

In another aspect, the invention relates to compounds of formula (1) as hereinabove defined or to a pharmaceutically acceptable salt.

Suitable examples of pro-drugs of compounds of formula (1) are in-vivo hydrolysable esters of compounds of formula (1). Therefore in another aspect, are described compounds of formula (1) as hereinabove defined or to an in-vivo hydrolysable ester thereof.

It is to be understood that, insofar as certain of the compounds of formula (1) defined above may exist in optically active or racemic forms by virtue of one or more asymmetric carbon atoms, the invention includes in its definition any such optically active or racemic form which possesses glycogen phosphorylase inhibition activity. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to hereinafter.

Within the present invention it is to be understood that a compound of the formula (1) or a salt thereof may exhibit the phenomenon of tautomerism and that the formulae drawings within this specification can represent only one of the possible tautomeric forms. It is to be understood that the invention encompasses any tautomeric form which has glycogen phosphorylase inhibition activity and is not to be limited merely to any one tautomeric form utilised within the formulae drawings. The formulae drawings within this specification can represent only one of the possible tautomeric

forms and it is to be understood that the specification encompasses all possible tautomeric forms of the compounds drawn not just those forms which it has been possible to show graphically herein.

It is also to be understood that certain compounds of the formula (1) and salts thereof can exist in solvated as well as unsolvated forms such as, for example, hydrated forms. It is to be understood that the invention encompasses all such solvated forms which have glycogen phosphorylase inhibition activity.

It is also to be understood that certain compounds of the formula (1) may exhibit polymorphism, and that the invention encompasses all such forms which possess glycogen phosphorylase inhibition activity.

The present invention relates to the compounds of formula (1) as hereinbefore defined as well as to the salts thereof. Salts for use in pharmaceutical compositions will be pharmaceutically acceptable salts, but other salts may be useful in the production of the compounds of formula (1) and their pharmaceutically acceptable salts. Pharmaceutically acceptable salts of the invention may, for example, include acid addition salts of the compounds of formula (1) as hereinbefore defined which are sufficiently basic to form such salts. Such acid addition salts include for example salts with inorganic or organic acids affording pharmaceutically acceptable anions such as with hydrogen halides (especially hydrochloric or hydrobromic acid of which hydrochloric acid is particularly preferred) or with sulphuric or phosphoric acid, or with trifluoroacetic, citric or maleic acid. Suitable salts include hydrochlorides, hydrobromides, phosphates, sulphates, hydrogen sulphates, alkylsulphonates, arylsulphonates, acetates, benzoates, citrates, maleates, fumarates, succinates, lactates and tartrates. In addition where the compounds of formula (1) are sufficiently acidic, pharmaceutically acceptable salts may be formed with an inorganic or organic base which affords a pharmaceutically acceptable cation. Such salts with inorganic or organic bases include for example an alkali metal salt, such as a sodium or potassium salt, an alkaline earth metal salt such as a calcium or magnesium salt, an ammonium salt or for example a salt with methylamine, dimethylamine, trimethylamine, piperidine, morpholine or tris-(2-hydroxyethyl)amine.

The compounds of formula I may be administered in the form of a pro-drug which is broken down in the human or animal body to give a compound of the invention. A prodrug may be used to alter or improve the physical and/or pharmacokinetic profile of the parent compound and can be formed when the parent compound contains a suitable group or substituent which can be derivatised to form a prodrug. Examples of pro-drugs include in-vivo hydrolysable esters of a compound of the invention or a pharmaceutically-acceptable salt thereof.

Various forms of prodrugs are known in the art, for examples see:

a) Design of Prodrugs, edited by H. Bundgaard, (Elsevier, 1985) and Methods in Enzymology, Vol. 42, p. 309-396, edited by K. Widder, et al. (Academic Press, 1985);
b) A Textbook of Drug Design and Development, edited by Krogsgaard-Larsen and H. Bundgaard, Chapter 5 "Design and Application of Prodrugs", by H. Bundgaard p. 113-191 (1991);
c) H. Bundgaard, Advanced Drug Delivery Reviews, 8, 1-38 (1992);
d) H. Bundgaard, et al., Journal of Pharmaceutical Sciences, 77, 285 (1988); and
e) N. Kakeya, et al., Chem Pharm Bull, 32, 692 (1984).

An in-vivo hydrolysable ester of a compound of formula (1) containing carboxy or hydroxy group is, for example. A pharmaceutically acceptable ester which is cleaved in the human or animal body to produce the parent acid or alcohol.

Suitable pharmaceutically acceptable esters for carboxy include (1-6C)alkoxymethyl esters for example methoxymethyl, (1-6C)alkanoyloxymethyl esters for example pivaloyloxymethyl, phthalidyl esters, (3-8C)cycloalkoxycarbonyloxy(1-6C)alkyl esters for example 1-cyclohexylcarbonyloxyethyl; 1,3-dioxolen-2-onylmethyl esters for example 5-methyl-1,3-dioxolen-2-onylmethyl; and (1-6C)alkoxycarbonyloxyethyl esters for example 1-methoxycarbonyloxyethyl and may be formed at any carboxy group in the compounds of this invention.

Suitable pharmaceutically-acceptable esters for hydroxy include inorganic esters such as phosphate esters (including phosphoramidic cyclic esters) and $\alpha$-acyloxyalkyl ethers and related compounds which as a result of the *in-vivo* hydrolysis of the ester breakdown to give the parent hydroxy group/s. Examples of $\alpha$-acyloxyalkyl ethers include acetoxymethoxy and 2,2-dimethylpropionyloxymethoxy. A selection of *in-vivo* hydrolysable ester forming groups for hydroxy include (1-10C)alkanoyl, for example acetyl; benzoyl; phenylacetyl; substituted benzoyl and phenylacetyl, (1-10C)alkoxycarbonyl (to give alkyl carbonate esters), for example ethoxycarbonyl; di-((1-4C))alkylcarbamoyl and *N*-(di-((1-4C))alkylaminoethyl)-*N*-((1-4C))alkylcarbamoyl (to give carbamates); di-((1-4C))alkylaminoacetyl and carboxyacetyl. Examples of ring substituents on phenylacetyl and benzoyl include aminomethyl, ((1-4C))alkylaminomethyl and di-(((1-4C))alkyl)aminomethyl, and morpholino or piperazino linked from a ring nitrogen atom via a methylene linking group to the 3- or 4- position of the benzoyl ring. Other interesting in-vivo hyrolysable esters include, for example, $R^AC$(O)O(1-6C)alkyl-CO-, wherein $R^A$ is for example, benzyloxy-((1-4C))alkyl, or phenyl). Suitable substituents on a phenyl group in such esters include, for example, 4-((1-4C)alkyl)piperazino-(1-4C)alkyl, piperazino-(1-4C)alkyl and morpholino-($C_1$-$C_4$)alkyl.

In this specification the generic term "alkyl" includes both straight-chain and branched-chain alkyl groups. However references to individual alkyl groups such as "propyl" are specific for the straight chain version only and

references to individual branched-chain alkyl groups such as *t*-butyl are specific for the branched chain version only. For example, "(1-4C)alkyl" includes methyl, ethyl, propyl, isopropyl and *t*-butyl and examples of "(1-6C)alkyl" include the examples of "(1-4C)alkyl" and additionally pentyl, 2,3-dimethylpropyl, 3-methylbutyl and hexyl. An analogous convention applies to other generic terms, for example "(2-4C)alkenyl" includes vinyl, allyl and 1-propenyl and examples of "(2-6C)alkenyl" include the examples of "(2-4C)alkenyl" and additionally 1-butenyl, 2-butenyl, 3-butenyl, 2-methylbut-2-enyl, 3-methylbut-1-enyl, 1-pentenyl, 3-pentenyl and 4-hexenyl. Examples of "(2-4C)alkynyl" includes ethynyl, 1-propynyl and 2-propynyl and examples of "$C_{2-6}$alkynyl"include the examples of "(2-4C)alkynyl" and additionally 3-butynyl, 2-pentynyl and 1-methylpent-2-ynyl.

The term "hydroxy(1-4C)alkyl" includes hydroxymethyl, hydroxyethyl, hydroxypropyl, hydroxyisopropyl and hydroxybutyl. The term "hydroxyethyl" includes 1-hydroxyethyl and 2-hydroxyethyl. The term "hydroxypropyl" includes 1-hydroxypropyl, 2-hydroxypropyl and 3-hydroxypropyl and an analogous convention applies to terms such as hydroxybutyl. The term "dihydroxy(1-4C)alkyl" includes dihydroxyethyl, dihydroxypropyl, dihydroxyisopropyl and dihydroxybutyl. The term "dihydroxypropyl" includes 1,2-dihydroxypropyl and 1,3-dihydroxypropyl. An analogous convention applies to terms such as dihydroxyisopropyl and dihydroxybutyl.

The term "halo" refers to fluoro, chloro, bromo and iodo. The term "dihalo(1-4C)alkyl" includes difluoromethyl and dichloromethyl. The term "trihalo(1-4C)alkyl" includes trifluoromethyl.

Examples of "5- and 6-membered cyclic acetals and mono- and di-methyl derivatives thereof" are:

1,3-dioxolan-4-yl, 2-methyl-1,3-dioxolan-4-yl, 2,2-dimethyl-1,3-dioxolan-4-yl; 2,2-dimethyl-1,3-dioxan-4-yl; 2,2-dimethyl-1,3-dioxan-5-yl; 1,3-dioxan-2-yl.

Examples of "(1-4C)alkoxy" and "-O(1-4C)alkyl" include methoxy, ethoxy, propoxy and isopropoxy. Examples of "(1-6C)alkoxy" include the examples of "(1-4C)alkoxy" and additionally butyloxy, *t*-butyloxy, pentoxy and 1,2-(methyl)$_2$propoxy. Examples of "(1-4C)alkanoyl" include formyl, acetyl and propionyl. Examples of "(1-6C)alkanoyl" include the example of "(1-4C)alkanoyl" and additionally butanoyl, pentanoyl, hexanoyl and 1,2-(methyl)$_2$propionyl. Examples of "(1-4C)alkanoyloxy" and -$CO_2$(1-4C)alkyl include formyloxy, acetoxy and propionoxy. Examples of "(1-6C)alkanoyloxy" include the examples of "(1-4C)alkanoyloxy" and additionally butanoyloxy, pentanoyloxy, hexanoyloxy and 1,2-(methyl)$_2$propionyloxy. Examples of "*N*-((1-4C)alkyl)amino" include methylamino and ethylamino. Examples of "*N*-((1-6C) alkyl)amino" include the examples of "*N*-((1-4C)alkyl)amino" and additionally pentylamino, hexylamino and 3-methylbutylamino. Examples of "*N*,*N*-((1-4C)alkyl)$_2$amino" include *N*-*N*-(methyl)$_2$amino, *N*-*N*-(ethyl)$_2$amino and *N*-ethyl-*N*-methylamino. Examples of "*N*,*N*-((1-6C)alkyl)$_2$amino" include the example of "*N*,*N*-((1-4C)alkyl)$_2$amino" and additionally *N*-methyl-*N*-pentylamino and *N,N*-(pentyl)$_2$amino. Examples of "*N*-((1-4C)alkyl)carbamoyl" are methylcarbamoyl and ethylcarbamoyl. Examples of "*N*-((1-6C)alkyl)carbamoyl" are the examples of "*N*-((1-4C)alkyl)carbamoyl"and additionally pentylcarbamoyl, hexylcarbamoyl and 1,2-(methyl)$_2$propylcarbamoyl. Examples of "*N*,*N*-((1-4C)alkyl)$_2$carbamoyl" are *N,N*-(methyl)$_2$carbamoyl, *N*,*N*-(ethyl)$_2$carbamoyl and *N*-methyl-*N*-ethylcarbamoyl. Examples of "*N,N*-((1-6C) alkyl)$_2$carbamoyl" are the examples of "*N*,*N*-((1-4C)alkyl)$_2$carbamoyl" and additionally *N*,*N*-(pentyl)$_2$carbamoyl, *N*-methyl-*N*-pentylcarbamoyl and *N*-ethyl-*N*-hexylcarbamoyl. Examples of "*N*-((1-4C)alkyl)sulphamoyl" are *N*-(methyl)sulphamoyl and *N*-(ethyl)sulphamoyl. Examples of "*N*-((1-6C)alkyl)sulphamoyl" are the examples of "*N*-((1-4C)alkyl)sulphamoyl" and additionally *N*-pentylsulphamoyl, *N*-hexylsulphamoyl and 1,2-(methyl)$_2$propylsulphamoyl. Examples of "*N*,*N*-((1-4C)alkyl)$_2$sulphamoyl" are *N,N*-(methyl)$_2$sulphamoyl, *N,N*-(ethyl)$_2$sulphamoyl and *N*-(methyl)-*N*-(ethyl)sulphamoyl. Examples of "*N*,*N*-((1-6C)alkyl)$_2$sulphamoyl" are the examples of *"N,N*-((1-4C)alkyl)$_2$sulphamoyl" and additionally *N*,*N*-(pentyl)$_2$sulphamoyl, *N*-methyl-*N* pentylsulphamoyl and *N*-ethyl-*N*-hexylsulphamoyl. Examples of -$NHSO_2$ (1-4C)alkyl are methylsulfonylamino, ethylsulfonylamino, propylsulfonylamino, isopropylsulfonylamino and tert-butylsulfonylamino.

Examples of "cyano(1-4C)alkyl" are cyanomethyl, cyanoethyl and cyanopropyl. Examples of "(5-7C)cycloalkyl" are cyclopentyl, cyclohexyl and cycloheptyl. Examples of "(3-8C)cycloalkyl" and "(3-7C)cycloalkyl" include "(5-7C)cycloalkyl", cyclopropyl, cyclobutyl and cyclooctyl. Examples of "(3-6C)cycloalkyl" include cyclopropyl, cyclobutyl, cyclopentyl and cyclohexyl.

The term "amino(1-4C)alkyl" includes aminomethyl, aminoethyl, aminopropyl, aminoisopropyl and aminobutyl. The term "aminoethyl" includes 1-aminoethyl and 2-aminoethyl. The term "aminopropyl" includes 1-aminopropyl, 2-aminopropyl and 3-aminopropyl and an analogous convention applies to terms such as aminoethyl and aminobutyl.

Examples of "(1-4C)alkoxy(1-4C)alkoxy" are methoxymethoxy, ethoxymethoxy, ethoxyethoxy and methoxyethoxy. Examples of "hydroxy(1-4C)alkoxy" are hydroxyethoxy and hydroxypropoxy. Examples of "hydroxypropoxy" are 1-hydroxypropoxy, 2-hydroxypropoxy and 3-hydroxypropoxy. Examples of "(1-4C)alkoxy(1-4C)alkyl" include methoxymethyl, ethoxymethyl, methoxyethyl, isopropoxymethyl, and tert-butoxymethyl.

Examples of "(1-4C)alkylS(O)$_b$ (wherein b is 0,1 or 2)", "(1-4C)alkylS(O)$_c$ (wherein c is 0 to 2)" and "(1-4C) allcylS(O)$_d$ (wherein d is 0 to 2)", independently include methylthio, ethylthio, propylthio, methanesulphinyl, ethanesulphinyl, propanesulphinyl, mesyl, ethanesulphonyl, propanesulphonyl and isopropanesulphonyl. Examples of "(1-4C)alkylS(O)$_b$(1-4C)alkyl-" (wherein b is 0,1 or 2)" include methylsulfonylmethyl, methylsulfinylmehtyl, methylthiomethyl, ethylsulfonylmethyl, ethylsulfinylmethyl and ethylthiomethyl.

Examples of "-(1-4C)alkylSO$_2$(2-4C)alkenyl" include vinylsulfonylmethyl, vinylsulfonylethyl, and allylsulfonylmethyl.

Examples of "(3-6C)cycloalkylS(O)$_b$ (wherein b is 0,1 or 2)" include cyclopropylthio, cyclopropylsulphinyl, cyclopropylsulphonyl, cyclobutylthio, cyclobutylsulphinyl, cyclobutylsulphonyl, cyclopentylthio, cyclopentylsulphinyl and cyclopentylsulphonyl.

Examples of "arylS(O)$_b$ (wherein b is 0,1 or 2)" include phenylthio, phenylsulphinyl and phenylsulfonyl. Examples of "benzylS(O)$_b$ (wherein b is 0,1 or 2)" inculde benzylthio, benzylsulfinyl and benzylsulfonyl. Examples of "heterocyclylS (O)$_b$ (wherein b is 0,1 or 2)" include pyridylthio, pyridylsulfinyl, pyridylsulfonyl, imidazolylthio, imidazolylsulfinyl, imidazolylsulfonyl, pyrimidinylthio, pyrimidinylsufinyl, pyrimidinylsulfonyl, piperidylthio, piperidylsulfinyl and piperidylsulfonyl.

Examples of "(1-6C)alkoxycarbonyl" include methoxycarbonyl, ethoxycarbonyl, n-and *t*-butoxycarbonyl. Examples of "(1-6C)alkoxycarbonylamino" include methoxycarbonylamino, ethoxycarbonylamino, *n*- and *t*-butoxycarbonylamino. Examples of "(1-6C)alkylsulphonyl-*N*-((1-6C)alkyl)amino" include methylsulphonyl-*N*-methylamino, ethylsulphonyl-*N*-methylamino and propylsulphonyl-*N*-ethylamino. Examples of "(1-6C)alkylsulphonylamino" include methylsulphonylamino, ethylsulphonylamino and propylsulphonylamino. Examples of "(1-6C)alkanoylamino" include formamido, acetamido and propionylamino.

For the avoidance of doubt it is to be understood that where in this specification a group is qualified by 'hereinbefore defined' or 'defined hereinbefore' the said group encompasses the first occurring and broadest definition as well as each and all of the particular definitions for that group.

It is to be understood that where substituents contain two substituents on an alkyl chain, in which both are linked by a heteroatom (for example two alkoxy substituents), then these two substituents are not substituents on the same carbon atom of the alkyl chain.

It is to be understood that optional substituents on any group may be attached to any available atom as appropriate unless otherwise specified, including heteroatoms provided that they are not thereby quaternised.

Within this specification composite terms are used to describe groups comprising more that one functionality such as -(1-6C)alkylNHSO$_2$(1-6C)alkyl. Such terms are to be interpreted in accordance with the meaning which is understood by a person skilled in the art for each component part. For example -(1-6)alkylNHSO$_2$(1-6C)alkyl includes -methylaminosulphonylmethyl, -methylaminosulphonylethyl, -ethylaminosulphonylmethyl, and -propylaminosulphonylbutyl.

Where optional substituents are chosen from "0, 1, 2 or 3" groups it is to be understood that this definition includes all substituents being chosen from one of the specified groups or the substituents being chosen from two or more of the specified groups. An analogous convention applies to substituents chose from "0, 1 or 2" groups and "1 or 2" groups.

Substituents may be present at any suitable position on, for example, an alkyl group. Therefore, hydroxy substituted (1-6C)alkyl includes hydroxymethyl, 1-hydroxyethyl, 2-hydroxyethyl and 3-hydroxypropyl.

"Heterocyclyl" is a saturated, partially saturated or unsaturated, optionally substituted monocyclic ring containing 5 to 7 atoms of which 1, 2, 3 or 4 ring atoms are chosen from nitrogen, sulphur or oxygen, which may, unless otherwise specified, be carbon or nitrogen linked, wherein a -CH$_2$- group can optionally be replaced by a -C(O)-and a ring sulphur atom may be optionally oxidised to form the S-oxide(s). Examples and suitable values of the term "heterocyclyl" are morpholino, morpholinyl, piperidino, piperidyl, pyridyl, pyranyl, pyrrolyl, imidazolyl, thiazolyl, thienyl, dioxolanyl, thiadiazolyl, piperazinyl, isothiazolidinyl, triazolyl, tetrazolyl, pyrrolidinyl, 2-oxazolidinonyl, 5-isoxazolonyl, thiomorpholino, pyrrolinyl, homopiperazinyl, 3,5-dioxapiperidinyl, 3-oxopyrazolin-5-yl, tetrahydropyranyl, tetrahydrothiopyranyl, 1-oxotetrahydrothiopyranyl, 1,1-dioxotetrahydrothiopyranyl, pyrimidyl, pyrazinyl, pyridazinyl, pyrazolyl, pyrazolinyl, isoxazolyl, 4-oxopydridyl, 2-oxopyrrolidyl, 4-oxothiazolidyl, furyl, thienyl, oxazolyl, and oxadiazolyl.

Suitably a "heterocyclyl" is morpholino, morpholinyl, piperidino, piperidyl, pyridyl, pyranyl, pyrrolyl, imidazolyl, thiazolyl, thienyl, thiadiazolyl, piperazinyl, isothiazolidinyl, 1,3,4-triazolyl, tetrazolyl, pyrrolidinyl, thiomorpholino, pyrrolinyl, homopiperazinyl, 3,5-dioxapiperidinyl, pyrimidyl, pyrazinyl, pyridazinyl, pyrazolyl, pyrazolinyl, isoxazolyl, 4-oxopydridyl, 2-oxopyrrolidyl, 4-oxothiazolidyl, furyl, thienyl, oxazolyl, 1,3,4-oxadiazolyl, and 1,2,4-oxadiazolyl.

Conveniently "heterocyclyl" is oxazolyl, 1,3,4-oxadiazolyl, 1,2,4-oxadiazolyl, tetrazolyl, thizoyl, thiadiazolyl, pyridyl, imidazolyl, furyl, thienyl, morpholine, pyrimidyl, pyrazinyl, pyridazinyl, pyrazolyl, pyrazolinyl, and piperazinyl.

Suitable optional substituents for "heterocyclyl" as a saturated or partially saturated ring are 1, 2 or 3 substituents independently selected from halo, cyano, hydroxy, (1-4C)alkyl, (1-4C)alkoxy and (1-4C)alkylS(O)$_b$ (wherein b is 0, 1 or 2). Further suitable substituents for "heterocyclyl" as a saturated or partially saturated ring are 1, 2 or 3 substituents independently selected from fluoro, chloro, cyano, hydroxy, methyl, ethyl, methoxy, methylthio, methylsulfinyl and methylsulfonyl.

Suitable optional susbtituents for "heterocyclyl" as an unsaturated ring are 1, 2 or 3 substituents independently selected from halo, cyano, nitro, amino, hydroxy, (1-4C)alkyl, (1-4C)alkoxy, (1-4C)alkylS(O)b- (wherein b is 0, 1 or 2),

*N*-((1-4C)alkyl)amino and *N,N*-((1-4C)alkyl)$_2$amino. Further suitable optional susbtituents for "heterocyclyl" as an unsaturated ring are 1, 2 or 3 substituents independently selected from fluoro, chloro, cyano, nitro, amino, methylamino, dimethylamino, hydroxy, methyl, ethyl, methoxy, methylthio, methylsulfinyl and methylsulfonyl.

Examples of "heterocyclyl(1-4C)alkyl" are morpholinomethyl, morpholinethyl, morpholinylmethyl, morpholinylethyl, piperidinomethyl, piperidinoethyl, piperidylmethyl, piperidylethyl, imidazolylmethyl, imidazolylethyl, oxazolylmethyl, oxazolylethyl, 1,3,4-oxadiazolylmethyl, 1,2,4-oxadiazolylmethyl, 1,2,4-oxadiazolylethyl, pyridylmethyl, pyridylethyl, furylmethyl, furylethyl, (thienyl)methyl, (thienyl)ethyl, pyrazinylmethyl, pyrazinylethyl, piperazinylmethyl and piperazinylethyl.

Examples of "aryl" are optionally substituted phenyl and naphthyl.

Examples of "aryl(1-4C)alkyl" are benzyl, phenethyl, naphthylmethyl and naphthylethyl.

Suitable optional substituents for "aryl" groups are 1, 2 or 3 substituents independently selected from halo, cyano, nitro, amino, hydroxy, (1-4C)alkyl, (1-4C)alkoxy, (1-4C)alkylS(O)$_b$ (wherein b is 0, 1 or 2), *N*-((1-4C)alkyl)amino and *N,N*-((1-4C)alkyl)$_2$amino. Further suitable optional susbtituents for "aryl" groups are 1, 2 or 3 substituents independently selected from fluoro, chloro, cyano, nitro, amino, methylamino, dimethylamino, hydroxy, methyl, ethyl, methoxy, methylthio, methylsulfinyl and methylsulfonyl.

"Heteroarylene" is a diradical of a heteroaryl group. A heteroaryl group is an aryl, monocyclic ring containing 5 to 7 atoms of which 1, 2, 3 or 4 ring atoms are chosen from nitrogen, sulphur or oxygen. Examples of heteroarylene are oxazolylene, oxadiazolylene, pyridylene, pyrimidinylene, imidazolylene, triazolylene, tetrazolylene, pyrazinylene, pyridazinylene, pyrrolylene, thienylene and furylene.

Suitable optional substituents for heteroaryl groups, unless otherwise defined, are 1, 2 or 3 substituents independently selected from halo, cyano, nitro, amino, hydroxy, (1-4C)alkyl, (1-4C)alkoxy, (1-4C)alkylS(O)$_b$ (wherein b is 0, 1 or 2), *N*-((1-4C)alkyl)amino and *N,N*-((1-4C)alkyl)$_2$amino. Further suitable optional susbtituents for "heteroaryl" groups are 1, 2 or 3 substituents independently selected from fluoro, chloro, cyano, nitro, amino, methylamino, dimethylamino, hydroxy, methyl, ethyl, methoxy, methylthio, methylsulfinyl and methylsulfonyl.

Preferred values of A, Y, $R^1$ to $R^{11}$, r, m and n are as follows. Such values may be used where appropriate with any of the definitions, claims, aspects or embodiments defined hereinbefore or hereinafter.

In one embodiment of the invention are provided compounds of formula (1), in an alternative embodiment are provided pharmaceutically-acceptable salts of compounds of formula (1), in a further alternative embodiment are provided in-vivo hydrolysable esters of compounds of formula (1), and in a further alternative embodiment are provided pharmaceutically-acceptable salts of in-vivo hydrolysable esters of compounds of formula (1). Also are disclosed pro-drugs of compounds of formula (1) and pharmaceutically-acceptable salts of pro-drugs of compounds of formula (1).

Particular examples of in-vivo hydrolysable esters of compounds of the formula (1) are such esters of compounds of the formula (1) wherein Y comprises a group containing a carboxy group. Suitable esters are those hereinbefore described for carboxy groups.

In one aspect of the present invention there is provided a compound of formula (1) as depicted above wherein A is phenylene.

In another aspect of the invention A is heteroarylene.

Preferably A is selected from phenylene, pyridylene, pyrimidinylene, pyrrolylene, imidazolylene, triazolylene, tetrazolylene, oxazolylene, oxadiazolylene, thienylene and furylene.

In one embodiment, when A is heteroarylene, there is a nitrogen in a bridgehead position. In another embodiment, when A is heteroarylene, the heteroatoms are not in bridgehead positions. It will be appreciated that the preferred (more stable) bridgehead position is as shown below

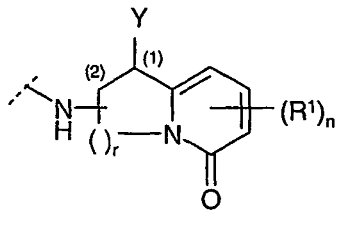

In one aspect of the present invention m is 1 or 2.

In another aspect of the invention m is 1.

In another aspect of the invention m is 0.

In one aspect of the present invention $R^4$ is selected from halo, cyano, hydroxy, fluoromethyl, difluoromethyl and trifluoromethyl.

In another aspect of the invention $R^4$ is halo.

Preferably $R^4$ is selected from chloro and bromo.

In a further aspect $R^4$ is selected from chloro, fluoro and methyl.

In a further aspect $R^4$ is selected from chloro and fluoro

More preferably $R^4$ is chloro.

In one aspect of the invention n is 0 or 1.

In one aspect preferably n is 1.

In another aspect, preferably n is 0.

When n is 2, and the two $R^1$ groups, together with the carbon atoms of A to which they are attached, form a 4 to 7 membered saturated ring, optionally containing 1 or 2 heteroatoms independently selected from O, S and N, conveniently such a ring is a 5 or 6 membered ring.

In one embodiment such a 5 or 6 membered ring contains two O atoms (ie a cyclic acetal). When the two $R^1$ groups together form such a cyclic acetal, preferably it is not substituted. Most preferably the two $R^1$ groups together are the group $-O-CH_2-O-$.

In another aspect of the present invention $R^1$ is selected from halo, nitro, cyano, hydroxy, fluoromethyl, difluoromethyl, trifluoromethyl and (1-4C)alkoxy.

In a further aspect $R^1$ is selected from halo, nitro, cyano, hydroxy, fluoromethyl, difluoromethyl, trifluoromethyl, $-S(O)_b$(1-4C)alkyl (wherein b is 0, 1 or 2), $-OS(O)_2$(1-4C)alkyl, (1-4C)alkyl and (1-4C)alkoxy.

In a further aspect $R^1$ is selected from halo, nitro, cyano, hydroxy, fluoromethyl, difluoromethyl, trifluoromethyl, $-S(O)_bMe$ (wherein b is 0, 1 or 2), $-OS(O)_2Me$, methyl and methoxy.

In a further aspect, $R^1$ is (1-4C)alkyl.

Preferably $R^1$ is selected from halo and (1-4C)alkoxy.

In another embodiment preferably $R^1$ is selected from fluoro, chloro, methyl, ethyl, methoxy and $-O-CH_2-O-$. In one aspect of the invention r is 1 and when r is 1 the group

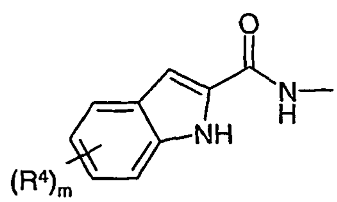

is a substituent on carbon (2) such that an example of when r is 1 is:

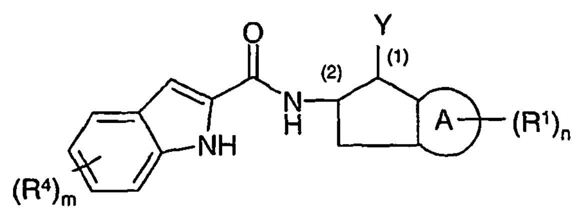

In another aspect of the invention r is 2 and when r is 2 the group

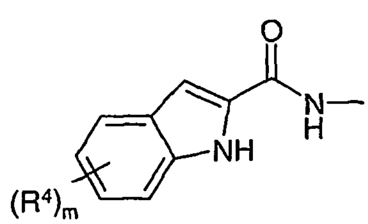

is a substituent on carbon (2) such that an example of when r is 2 is:

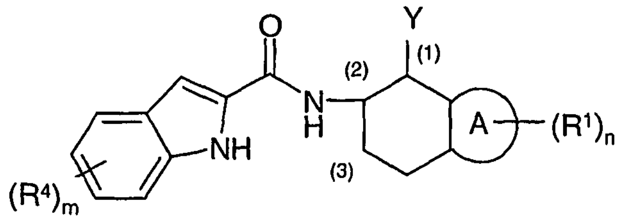

In another aspect of the invention r is 2 and when r is 2 the group

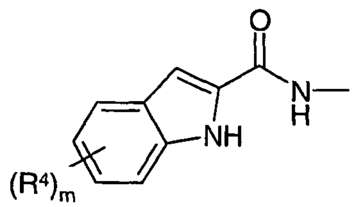

is a substituent on carbon (3) such that an example of when r is 2 is:

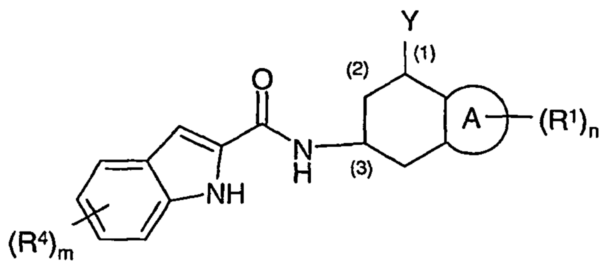

In one aspect, Y is selected from -C(O)$R^2$, -C(O)O$R^2$, -C(O)N$R^2R^3$, -C(O)NOH, -C(O)NSH, -C(N)OH, and-C (N)SH.

In another aspect, Y is selected from -(1-4C)alkyl [optionally substituted by 1 or 2 substituents independently selected from hydroxy, -C=N$R^2$, (1-4C)alkoxy, aryloxy, heterocyclyloxy, -S(O)$_b$$R^2$ (wherein b is 0, 1 or 2), -O-S(O)$_b$$R^2$ (wherein b is 0,1 or 2), -N$R^2R^3$, -N(OH)$R^2$, -N$R^2$C(=O)$R^2$, -NHOHC(=O)$R^2$, -$SO_2$N$R^2R^3$, -N($R^2$)$SO_2R^2$, aryl and heterocyclyl], -(2-4C)alkenyl, -(1-4C)alkylC(O)$R^2$, -(1-4C)alkylC(O)O$R^2$, -(1-4C)alkylOC(O)$R^2$,- (1-4C)alkylC(O)N$R^2R^3$, -(1-4C)alkylOC(O)O$R^2$, -(1-4C)alkylN($R^2$)C(O)O$R^2$, -(1-4C)alkylN($R^2$)C(O)N$R^2R^3$, -(1-4C)alkylOC(O)N$R^2R^3$, (3-6C)cycloalkyl (optionally substituted by 1 or 2 $R^8$), aryl and heterocyclyl (wherein the heterocyclic ring is linked by a ring carbon atom).

In a further aspect Y is selected from -$SO_2$H, -$SO_3$H, -$SO_2$N(OH)$R^2$, -$SO_2$N$R^2R^3$ and -S(O)$_c$$R^2$ (wherein c is 0, 1 or 2).

In a further aspect, Y is selected from-C(O)$R^2$, -C(O)O$R^2$, -C(O)N$R^2R^3$, -(1-4C)alkyl [optionally substituted by a substituent selected from hydroxy, -C=N$R^2$, (1-4C)alkoxy, aryloxy, heterocyclyloxy, -S(O)$_b$$R^2$ (wherein b is 0, 1 or 2), -O-S(O)$_b$$R^2$ (wherein b is 0, 1 or 2), -N$R^2R^3$, -N(OH)$R^2$, -N$R^2$C(=O)$R^2$, -NHOHC(=O)$R^2$, -$SO_2$N$R^2R^3$, -N($R^2$)$SO_2R^2$ , aryl and heterocyclyl], -C(O)NOH, -C(O)NSH, -C(N)OH, -$SO_2$H, -$SO_3$H, -$SO_2$N$R^2R^3$, -(1-4C)alkylC(O)$R^2$, -(1-4C)alkylC (O)O$R^2$, -(1-4C)alkylSC(O)$R^2$, -(1-4C)alkylOC(O)$R^2$, - (1-4C)alkylC(O)N$R^2R^3$, -(1-4C)alkylOC(O)O$R^2$, -(1-4C)alkylN ($R^2$)C(O)O$R^2$, -(1-4C)alkylN($R^2$)C(O)N$R^2R^3$, -(1-4C)alkylOC(O)N$R^2R^3$, -S (O)$_c$$R^2$ (wherein c is 0, 1 or 2), aryl, heterocyclyl (wherein the heterocyclic ring is linked by a ring carbon atom) and -(1-4C)alkyl$SO_2$(2-4C)alkenyl.

In a further aspect, Y is selected from-C(O)$R^2$, -C(O)O$R^2$, -C(O)N$R^2R^3$, -(1-4C)alkyl [optionally substituted by a substituent selected from hydroxy, -C=N$R^2$, (1-4C)alkoxy, aryloxy, heterocyclyloxy, -S(O)$_b$$R^2$ (wherein b is 0, 1 or 2), -O-S(O)$_b$$R^2$(wherein b is 0,1 or 2), -N$R^2R^3$, -N(OH)$R^2$, -N$R^2$C(=O)$R^2$, -NHOHC(=O)$R^2$, -$SO_2$N$R^2R^3$, -N($R^2$)$SO_2R^2$, aryl and heterocyclyl], -C(O)NOH, -C(O)NSH, -C(N)OH, -$SO_2$H, -$SO_3$H, -$SO_2$N$R^2R^3$, -(1-4C)alkylC(O)$R^2$, -(1-4C)alkylC(O) O$R^2$, -(1-4C)alkylOC(O)$R^2$, -(1-4C)alkylC(O)N$R^2R^3$, -(1-4C)alkylOC(O)O$R^2$, -(1-4C)alkylN($R^2$)C(O)O$R^2$, -(1-4C)alkylN ($R^2$)C(O)N$R^2R^3$, -(1-4C)alkylOC(O)N$R^2R^3$, -S(O)$_c$$R^2$ (wherein c is 0, 1 or 2), aryl and heterocyclyl (wherein the heterocyclic ring is linked by a ring carbon atom).

In a further aspect, Y is selected from-C(O)$R^2$ , -C(O)O$R^2$, -C(O)N$R^2R^3$, -(1-4C)alkyl [optionally substituted by a substituent selected from hydroxy, -C=N$R^2$, (1-4C)alkoxy, aryloxy, heterocyclyloxy, -S(O)$_b$$R^2$ (wherein b is 0, 1 or 2), -O-S(O)$_b$$R^2$ (wherein b is 0,1 or 2), -N$R^2R^3$, -N(OH)$R^2$, -N$R^2$C(=O)$R^2$, -NHOHC(=O)$R^2$, -$SO_2$N$R^2R^3$, -N($R^2$)$SO_2$$R^2$, aryl and heterocyclyl], -C(O)NOH, -C(O)NSH, -C(N)OH, -$SO_2$H, -$SO_3$H, -$SO_2$N$R^2R^3$, -(1-4C)alkylC(O)$R^2$, -(1-4C)alkylC (O)O$R^2$, -(1-4C)alkylSC(O)$R^2$, -(1-4C)alkylOC(O)$R^2$, -(1-4C)alkylC(O)N$R^2R^3$, -(1-4C)alkylOC(O)O$R^2$, -(1-4C)alkylN ($R^2$)C(O)O$R^2$, -(1-4C)alkylN($R^2$)C(O)N$R^2R^3$, -(1-4C)alkylOC(O)N$R^2R^3$, - S(O)$_c$$R^2$ (wherein c is 0, 1 or 2), aryl, heterocyclyl (wherein the heterocyclic ring is linked by a ring carbon atom) and -(1-4C)alkyl$SO_2$(2-4C)alkenyl.

In a further aspect, Y is selected from-C(O)$R^2$, -C(O)O$R^2$, -C(O)N$R^2R^3$, -(1-4C)alkyl [optionally substituted by a substituent selected from hydroxy, -C=N$R^2$, (1-4C)alkoxy, aryloxy, heterocyclyloxy, -S(O)$_b$$R^2$ (wherein b is 0, 1 or 2), -O-S(O)$_b$$R^2$ (wherein b is 0, 1 or 2), -N$R^2R^3$, -N(OH)$R^2$, -N$R^2$C(=O)$R^2$, -NHOHC(=O)$R^2$, -$SO_2$N$R^2R^3$, -N($R^2$)$SO_2$$R^2$ , aryl and heterocyclyl], -C(O)NOH, -(1-4C)alkylC(O)$R^2$, -(1-4C)alkylC(O)OR , -(1-4C)alkylOC(O)$R^2$, - (1-4C)alkylC(O) N$R^2R^3$,- (1-4C)alkylOC(O)O$R^2$, -(1-4C)alkylN($R^2$)C(O)O$R^2$, -(1-4C)alkylN($R^2$)C(O)N$R^2R^3$, -(1-4C)alkylOC(O)N$R^2R^3$, aryl and heterocyclyl (wherein the heterocyclic ring is linked by a ring carbon atom).

In a further aspect, Y is selected from-C(O)$R^2$, -C(O)O$R^2$, -C(O)N$R^2R^3$, -(1-4C)alkyl [optionally substituted by a substituent selected from hydroxy, -C=N$R^2$, (1-4C)alkoxy, aryloxy, heterocyclyloxy, -S(O)$_b$$R^2$ (wherein b is 0, 1 or 2), -O-S(O)$_b$$R^2$ (wherein b is 0,1 or 2), -N$R^2R^3$, -N(OH)$R^2$, -N$R^2$C(=O)$R^2$, -NHOHC(=O)$R^2$, -$SO_2$N$R^2R^3$, -N($R^2$)$SO_2$$R^2$ , aryl and heterocyclyl], -C(O)NOH, -(1-4C)alkylC(O)$R^2$, -(1-4C)alkylC(O)O$R^2$, -(1-4C)alkylSC(O)$R^2$, -(1-4C)alkylOC(O) $R^2$, -(1-4C)alkylC(O)N$R^2R^3$, -(1-4C)alkylOC(O)O$R^2$, -(1-4C)alkylN($R^2$)C(O)O$R^2$, -(1-4C)alkylN($R^2$)C(O)N$R^2R^3$, -(1-4C) alkylOC(O)N$R^2R^3$, aryl and heterocyclyl (wherein the heterocyclic ring is linked by a ring carbon atom) and -(1-4C) alkyl$SO_2$(2-4C)alkenyl.

In a further aspect, Y is selected from-C(O)$R^2$, -C(O)O$R^2$, -C(O)N$R^2R^3$, -(1-4C)alkyl [optionally substituted by a substituent selected from hydroxy, (1-4C)alkoxy, aryloxy, heterocyclyloxy , -S(O)$_b$$R^2$ (wherein b is 0, 1 or 2), -O-S (O)$_b$$R^2$ (wherein b is 0, 1 or 2), -N$R^2R^3$, -N(OH)$R^2$, -N$R^2$C(=O)$R^2$, aryl and heterocyclyl], -C(O)NOH, -(1-4C)alkylC(O) $R^2$, -(1-4C)alkylC(O)O$R^2$, -(1-4C)alkylOC(O)$R^2$, -(1-4C)alkylC(O)N$R^2R^3$, -(1-4C)alkylOC(O)O$R^2$, -C1-4C)alkylN($R^2$)C (O)O$R^2$, -(1-4C)alkylN($R^2$)C(O)N$R^2R^3$, -(1-4C)alkylOC(O)N$R^2R^3$, aryl and heterocyclyl (wherein the heterocyclic ring is linked by a ring carbon atom).

In a further aspect, Y is selected from-C(O)$R^2$ , -C(O)O$R^2$, -C(O)N$R^2R^3$, -(1-4C)alkyl [optionally substituted by a substituent selected from hydroxy, (1-4C)alkoxy, aryloxy, heterocyclyloxy , -S(O)$_b$$R^2$ (wherein b is 0, 1 or 2), -O-S (O)$_b$$R^2$ (wherein b is 0, 1 or 2), -N$R^2R^3$, -N(OH)$R^2$, -N$R^2$C(=O)$R^2$, aryl and heterocyclyl], -C(O)NOH, 5-(1-4C)alkylC(O) $R^2$, -(1-4C)alkylC(O)O$R^2$, -(1-4C)alkylSC(O)$R^2$, -(1-4C)alkylOC(O)$R^2$, -(1-4C)alkylC(O)N$R^2R^3$, -(1-4C)alkylOC(O)O$R^2$, -(1-4C)alkylN($R^2$)C(O)O$R^2$, -(1-4C)alkylN($R^2$)C(O)N$R^2R^3$, -(1-4C)alkylOC(O)N$R^2R^3$, aryl and heterocyclyl (wherein the heterocyclic ring is linked by a ring carbon atom) and -(1-4C)alkyl$SO_2$(2-4C)alkenyl.

In a further aspect, Y is selected from-C(O)$R^2$ , -C(O)O$R^2$, -C(O)N$R^2R^3$, -C(O)NOH, and -(1-4C)alkyl [optionally substituted by a substituent selected from hydroxy, -S(O)$_b$$R^2$ (wherein b is 0, 1 or 2), -O-S(O)$_b$$R^2$ (wherein b is 0, 1 or 2), aryloxy, heterocyclyloxy, -N$R^2R^3$, -N(OH)$R^2$, -N$R^2$C(=O)$R^2$, aryl and heterocyclyl].

In a further aspect, Y is selected from-C(O)$R^2$ , -C(O)O$R^2$, -C(O)N$R^2R^3$, -C(O)NOH, and -(1-4C)alkyl [optionally substituted by a substituent selected from hydroxy, -S(O)$_b$$R^2$ (wherein b is 0, 1 or 2), -O-S(O)$_b$$R^2$ (wherein b is 0, 1 or 2), -N$R^2R^3$, -N(OH)$R^2$, and -N$R^2$C(=O)$R^2$].

In a further aspect, Y is selected from-C(O)$R^2$, -C(O)O$R^2$, -C(O)N$R^2R^3$, -C(O)NOH, and -(1-4C)alkyl [optionally substituted by a -N$R^2$C(=O)$R^2$], -(1-4C)alkylC(O)$R^2$, -(1-4C)alkylC(O)O$R^2$, -(1-4C)alkylOC(O)$R^2$, - (1-4C)alkylC(O) N$R^2R^3$, -(1-4C)alkylOC(O)O$R^2$, -(1-4C)alkylN($R^2$)C(O)O$R^2$, -(1-4C)alkylN($R^2$)C(O)N$R^2R^3$, and -(1-4C)alkylOC(O) N$R^2R^3$.

In a further aspect, Y is selected from-C(O)$R^2$, -C(O)O$R^2$, -C(O)N$R^2R^3$, -C(O)NOH, and -(1-4C)alkyl [optionally substituted by a -N$R^2$C(=O)$R^2$], -(1-4C)alkylC(O)$R^2$, -(1-4C)alkylC(O)O$R^2$, -(1-4C)alkylSC(O)$R^2$, -(1-4C)alkylOC(O)$R^2$, -(1-4C)alkylC(O)N$R^2R^3$, -(1-4C)alkylOC(O)O$R^2$, -(1-4C)alkylN($R^2$)C(O)O$R^2$, -(1-4C)alkylN($R^2$)C(O)N$R^2R^3$, -(1-4C) alkyl$SO_2$(2-4C)alkenyl and -(1-4C)alkylOC(O)N$R^2R^3$.

In one aspect $R^2$ and $R^3$ are independently selected from hydrogen, -O(1-4C)alkyl, -S(1-4C)alkyl, and-N(1-4C) alkyl.

In one aspect $R^2$ and $R^3$ are independently selected from hydrogen, -O(1-4C)alkyl and -N(1-4C)alkyl.

In another aspect $R^2$ and $R^3$ are independently selected from hydrogen, heterocyclyl, aryl and (1-4C)alkyl [optionally substituted by 1 or 2 $R^8$ groups].

In a further aspect $R^2$ and $R^3$ are independently selected from hydrogen and (1-4C)alkyl [optionally substituted by 1 or 2 $R^8$ groups].

In another aspect an N$R^2R^3$ group forms a 4 to 7 membered saturated, partially saturated or unsaturated ring, optionally containing 1, 2 or 3 additional heteroatoms independently selected from N, O and S, wherein any -$CH_2$- may optionally be replaced by - C(=O)-, and any N or S atom may optionally be oxidised to form an N-oxide or SO or $SO_2$ group respectively, and wherein the ring is optionally substituted by 1 or 2 substituents independently selected from halo, cyano, (1-4C)alkyl, hydroxy, (1-4C)alkoxy and (1-4C)alkylS(O)$_b$- (wherein b is 0, 1 or 2).

In another aspect an $NR^2R^3$ group forms a 5 or 6 membered saturated, partially saturated or unsaturated ring, optionally containing 1, 2 or 3 additional heteroatoms independently selected from N, O and S, wherein any $-CH_2-$ may optionally be replaced by - C(=O)-, and any N or S atom may optionally be oxidised to form an N-oxide or SO or $SO_2$ group respectively, and wherein the ring is optionally substituted by 1 or 2 substituents independently selected from halo, cyano, (1-4C)alkyl, hydroxy, (1-4C)alkoxy and (1-4C)alkyl$S(O)_b$- (wherein b is 0, 1 or 2). Suitably, such rings are selected from morpholine, thiomorpholine (and oxidised versions thereof), pyrrolidine, dihydropyridine, tetrahydropyridine, piperazine, piperidine.

Suitable optional substituents for a ring comprising $NR^2R^3$ are 1 or 2 substituents independently selected from halo, hydroxy and (1-4C)alkoxy. In one aspect the ring has 2 substituents and they are both hydroxy. In one aspect the ring has 2 substituents and they are both halo, particularly both are fluoro.

In another aspect, a ring comprising $NR^2R^3$ contains an additional ring atom selected from O, N and S. In another aspect, an additional sulphur atom is oxidised to form an S=O or $SO_2$ group.

In a further aspect $R^2$ and $R^3$ are independently selected from hydrogen, -O(1-4C)alkyl, -N(1-4C)alkyl, (1-4C) alkyl [optionally substituted by 1 or 2 $R^8$ groups];or an $NR^2R^3$ group forms a 5 or 6 membered saturated, partially saturated or unsaturated ring, optionally containing 1, 2 or 3 additional heteroatoms independently selected from N, O and S, wherein any $-CH_2-$ may optionally be replaced by -C(=O)-, and any N or S atom may optionally be oxidised to form an N-oxide or SO or $SO_2$ group respectively, and wherein the ring is optionally substituted by 1 or 2 substituents independently selected from halo, cyano, (1-4C)alkyl, hydroxy, (1-4C)alkoxy and (1-4C)alkyl$S(O)_b$- (wherein b is 0, 1 or 2).

In a further aspect $R^2$ and $R^3$ are independently selected from hydrogen, -O(1-4C)alkyl, -N(1-4C)alkyl, (1-4C) alkyl [optionally substituted by 1 or 2 $R^8$ groups];or an $NR^2R^3$ group forms a 5 or 6 membered saturated, partially saturated or unsaturated ring, optionally containing 1 or 2 additional heteroatoms independently selected from N, O and S, wherein any $-CH_2-$ may optionally be replaced by -C(=O)-, and any N or S atom may optionally be oxidised to form an N-oxide or SO or $SO_2$ group respectively, and wherein the ring is optionally substituted by 1 or 2 substituents independently selected from halo, hydroxy and (1-4C)alkoxy.

In a further aspect $R^2$ and $R^3$ are independently selected from hydrogen, -O(1-4C)alkyl, -N(1-4C)alkyl, (1-4C) alkyl [optionally substituted by 1 or 2 $R^8$ groups];or an $NR^2R^3$ group forms morpholine, thiomorpholine (and oxidised versions thereof), pyrrolidine, dihydropyridine, tetrahydropyridine, piperazine, piperidine ring and wherein the ring is optionally substituted by 1 or 2 substituents independently selected from halo, hydroxy and (1-4C)alkoxy.

In a further aspect $R^2$ and $R^3$ are independently selected from hydrogen, -O(1-4C)alkyl, -N(1-4C)alkyl, (1-4C) alkyl [optionally substituted by 1 or 2 $R^8$ groups];or an $NR^2R^3$ group forms a morpholine, thiomorpholine (and oxidised versions thereof), pyrrolidine, or piperidine ring and wherein the ring is optionally substituted by 1 or 2 substituents independently selected from chloro, fluoro, hydroxy and methoxy;

In one aspect $R^8$ is independently selected from hydrogen, hydroxy, (1-4C)alkyl, (1-4C)alkoxy, cyano(1-4C) alkyl, amino(1-4C)alkyl [optionally substituted on nitrogen by 1 or 2 groups selected from (1-4C)alkyl, hydroxy, hydroxy (1-4C)alkyl, dihydroxy(1-4C)alkyl, $-CO_2$(1-4C)alkyl, aryl and aryl(1-4C)alkyl], halo(1-4C)alkyl, dihalo(1-4C)alkyl, trihalo (1-4C)alkyl, hydroxy(1-4C)alkyl, dihydroxy(1-4C) alkyl, (1-4C)alkoxy(1-4C)alkoxy, (1-4C)alkoxy(1-4C)alkyl, hydroxy (1-4C)alkoxy, 5- and 6-membered cyclic acetals and mono- and di-methyl derivatives thereof, aryl, heterocyclyl, heterocyclyl(1-4C)alkyl, (1-4C)alkanoyl, (1-4C)alkyl$S(O)_b$- (wherein b is 0, 1 or 2, (1-4C)alkyl$S(O)_c$(1-4C)alkyl- (wherein c is 0, 1 or 2), -N(OH)CHO, $-COCOOR^9$, $-C(O)N(R^9)(R^{10})$, $-NHC(O)R^9$ , $-C(O)NHSO_2$(1-4C)alkyl, $-NHSO_2R^9$, $(R^9)(R^{10})NSO_2-$, $-COCH_2OR^{11}$, $-COCH_2OH$, $(R^9)(R^{10})N-$, $-COOR^9$, $-CH_2OR^9$, $-CH_2COOR^9$ , $-CH_2OCOR^9$, $-CH_2CH(CO_2R^9)OH$, $-CH_2C(O)NR^9R^{10}$, $-(CH_2)_wCH(NR^9R^{10})CO_2R^{9'}$ (wherein w is 1, 2 or 3), and $-(CH_2)_wCH(NR^9R^{10})CO(NR^{9'}R^{10'})$ (wherein w is 1, 2 or 3).

In another aspect, $R^8$ is independently selected from hydrogen, hydroxy, (2-4C)alkyl, (1-4C)alkoxy, amino (1-4C)alkyl, halo(1-4C)alkyl, dihalo(1-4C)alkyl, trihalo(1-4C)alkyl, hydroxy(1-4C)alkyl, dihydroxy(1-4C)alkyl, (1-4C) alkoxy(1-4C)alkoxy, (1-4C)alkoxy(1-4C)alkyl, hydroxy(1-4C)alkoxy, (1-4C)alkanoyl, (1-4C)alkyl$S(O)_b$- (wherein b is 0, 1 or 2, (1-4C)alkyl$S(O)_c$(1-4C)alkyl- (wherein c is 0, 1 or 2), ), -N(OH)CHO, $-COCOOR^9$, $-C(O)N(R^9)(R^{10})$, $-NHC(O)R^9$ , $-C(O)NHSO_2$(1-4C)alkyl, $-NHSO_2R^9$, $(R^9)(R^{10})NSO_2-$, $-COCH_2OR^{11}$, $-COCH_2OH$, $(R^9)(R^{10})N-$, $-COOR^9$, $-CH_2OR^9$, $-CH_2COOR^9$ and $-CH_2OCOR^9$.

In another aspect, $R^8$ is independently selected from hydrogen, hydroxy, (1-4C)alkyl, (1-4C)alkoxy, (1-4C) alkanoyl, $-COCOOR^9$, $-C(O)N(R^9)(R^{10})$, $-NHC(O)R^9$, -N(OH)CHO, $-C(O)NHSO_2$(1-4C)alkyl, $-NHSO_2R^9$, $(R^9)(R^{10})NSO_2-$, $-COCH_2OR^{11}$, $-COCH_2OH$, $(R^9)(R^{10})N-$, $-COOR^9$, $-CH_2OR^9$, $-CH_2COOR^9$ and $-CH_2OCOR^9$.

In another aspect, $R^8$ is independently selected from hydrogen, hydroxy, (1-4C)alkyl, (1-4C)alkoxy, (1-4C) alkanoyl, $-COCOOR^9$, $-C(O)N(R^9)(R^{10})$, $-NHC(O)R^9$, -N(OH)CHO, $-COOR^9$, $-CH_2OR^9$, $-CH_2COOR^9$ and $-CH_2OCOR^9$.

In one aspect $R^9$ and $R^{10}$ are independently selected from hydrogen, hydroxy and (1-4C)alkyl) optionally substituted by 1 or 2 $R^{11}$).

Suitably $R^{11}$ is (1-4C)alkyl.

In one aspect of the invention is provided a compound of the formula (I), or a pharmaceutically-acceptable salt thereof, wherein

A is phenylene;
n is 0, 1 or 2;
m is 1 or 2;
$R^1$ is selected from fluoro, chloro, methyl, ethyl, methoxy and -O-$CH_2$-O-;
$R^4$ is halo;
Y is selected from-C(O)$R^2$ , -C(O)O$R^2$, -C(O)N$R^2R^3$, -(1-4C)alkyl [optionally substituted by 1 or 2 substituents independently selected from hydroxy, -C=N$R^2$, (1-4C)alkoxy, aryloxy, heterocyclyloxy, -S$(O)_b R^2$ (wherein b is 0, 1 or 2), -O-S$(O)_b R^2$ (wherein b is 0, 1 or 2), -N$R^2R^3$, -N(OH)$R^2$, -N$R^2$C(=O)$R^2$, -NHOHC(=O)$R^2$, -$SO_2$N$R^2R^3$, -N($R^2$)$SO_2R^2$, aryl and heterocyclyl], -C(O)NOH, -C(O)NSH, -C(N)OH, -$SO_2$H, -$SO_3$H, -$SO_2$N$R^2R^3$, -(1-4C)alkylC(O)O$R^2$, -(1-4C)alkylOC(O)$R^2$, -(1-4C)alkylC(O)N$R^2R^3$, -(1-4C)alkylOC(O)$R^2$, -(1-4C)alkylN($R^2$)C(O)$R^2$, -(1-4C)alkylN($R^2$)C(O)N$R^2R^3$, -(1-4C)alkylOC(O)N$R^2R^3$, -S$(O)_c R^2$ (wherein c is 0, 1 or 2), aryl and heterocyclyl (wherein the heterocyclic ring is linked by a ring carbon atom);
$R^2$ and $R^3$ are independently selected from hydrogen, -O(1-4C)alkyl, -N(1-4C)alkyl, (1-4C)alkyl [optionally substituted by 1 or 2 $R^8$ groups];or an N$R^2R^3$ group forms a 5 or 6 membered saturated, partially saturated or unsaturated ring, optionally containing 1, 2 or 3 additional heteroatoms independently selected from N, O and S, wherein any -$CH_2$- may optionally be replaced by -C(=O)-, and any N or S atom may optionally be oxidised to form an N-oxide or SO or $SO_2$ group respectively, and wherein the ring is optionally substituted by 1 or 2 substituents independently selected from halo, cyano, (1-4C)alkyl, hydroxy, (1-4C)alkoxy and (1-4C)alkylS$(O)_b$- (wherein b is 0, 1 or 2);
$R^8$ is independently selected from hydrogen, hydroxy, (1-4C)alkyl, (1-4C)alkoxy, amino(1-4C)alkyl, halo(1-4C)alkyl, dihalo(1-4C)alkyl, trihalo(1-4C)alkyl, hydroxy(1-4C)alkyl, dihydroxy(1-4C)alkyl, (1-4C)alkoxy(1-4C)alkoxy, (1-4C)alkoxy(1-4C)alkyl, hydroxy(1-4C)alkoxy, (1-4C)alkanoyl, (1-4C)alkylS$(O)_b$- (wherein b is 0, 1 or 2, (1-4C)alkylS$(O)_c$(1-4C)alkyl- (wherein c is 0, 1 or 2), -N(OH)CHO, -COCOO$R^9$, -C(O)N($R^9$)($R^{10}$), -NHC(O)$R^9$ , -C(O)NH$SO_2$(1-4C)alkyl, -NH$SO_2R^9$, ($R^9$)($R^{10}$)N$SO_2$-, -CO$CH_2$O$R^{11}$, -CO$CH_2$OH, ($R^9$)($R^{10}$)N- , -COO$R^9$, -$CH_2$O$R^9$, -$CH_2$COO$R^9$ and -$CH_2$OCO$R^9$;
$R^9$ and $R^{10}$ are independently selected from hydrogen, hydroxy and (1-4C)alkyl) optionally substituted by 1 or 2 $R^{11}$);
$R^{11}$ is (1-4C)alkyl.

In a further aspect of the invention, is provided a compound of the formula (I) or a pharmaceutically-acceptable salt thereof, wherein
A is phenylene;
n is 0, 1 or 2;
m is 1 or 2;
$R^1$ is selected from fluoro, chloro, methyl, ethyl, methoxy and -O-$CH_2$-O-;
$R^4$ is halo;
Y is selected from-C(O)$R^2$ , -C(O)O$R^2$, -C(O)N$R^2R^3$, -(1-4C)alkyl [optionally substituted by a substituent selected from hydroxy, (1-4C)alkoxy, aryloxy, heterocyclyloxy, -S$(O)_b R^2$ (wherein b is 0, 1 or 2), -O-S$(O)_b R^2$ (wherein b is 0, 1 or 2), -N$R^2R^3$,-N(OH)$R^2$, -N$R^2$C(=O)$R^2$, aryl and heterocyclyl], -C(O)NOH, -(1-4C)alkylC(O)O$R^2$, -(1-4C)alkylOC(O)$R^2$, -(1-4C)alkylC(O)N$R^2R^3$ , -(1-4C)alkylOC(O)O$R^2$, -(1-4C)alkylN($R^2$)C(O)O$R^2$, -(1-4C)alkylN($R^2$)C(O)N$R^2R^3$, -(1-4C)alkylOC(O)N$R^2R^3$, aryl and heterocyclyl (wherein the heterocyclic ring is linked by a ring carbon atom);
$R^2$ and $R^3$ are independently selected from hydrogen, -O(1-4C)alkyl, -N(1-4C)alkyl, (1-4C)alkyl [optionally substituted by 1 or 2 $R^8$ groups];or an N$R^2R^3$ group forms a morpholine, thiomorpholine (and oxidised versions thereof), pyrrolidine, dihydropyridine, tetrahydropyridine, piperazine or piperidine ring and wherein the ring is optionally substituted by 1 or 2 substituents independently selected from halo, hydroxy and (1-4C)alkoxy;
$R^8$ is independently selected from hydrogen, hydroxy, (1-4C)alkyl, (1-4C)alkoxy, (1-4C)alkanoyl, -COCOO$R^9$, -C(O)N($R^9$)($R^{10}$), -NHC(O)$R^9$ , -N(OH)CHO, -C(O)NH$SO_2$(1-4C)alkyl, -NH$SO_2R^9$, ($R^9$)($R^{10}$)N$SO_2$-, -CO$CH_2$O$R^{11}$, -CO$CH_2$OH, ($R^9$)($R^{10}$)N-, -COO$R^9$, -$CH_2$O$R^9$, -$CH_2$COO$R^9$ and -$CH_2$OCO$R^9$;
$R^9$ and $R^{10}$ are independently selected from hydrogen, hydroxy and (1-4C)alkyl) optionally substituted by 1 or 2 $R^{11}$);
$R^{11}$ is (1-4C)alkyl.

In a further aspect of the invention, is provided a compound of the formula (I) or a pharmaceutically-acceptable salt thereof, wherein
A is phenylene;
n is 0;
m is 1;
$R^4$ is chloro;
Y is selected from-C(O)$R^2$ , -C(O)O$R^2$, -C(O)N$R^2R^3$, -(1-4C)alkyl [optionally substituted by a substituent selected from hydroxy, (1-4C)alkoxy, aryloxy, heterocyclyloxy, -S$(O)_b R^2$ (wherein b is 0, 1 or 2), -O-S$(O)_b R^2$ (wherein b is 0, 1 or 2), -N$R^2R^3$, -N(OH)$R^2$, -N$R^2$C(=O)$R^2$, aryl and heterocyclyl, -C(O)NOH, -(1-4C)alkylC(O)O$R^2$, -(1-4C)alkylOC(O)$R^2$ -(1-4C)allcylC(O)N$R^2R^3$ , -(1-4C)alkylOC(O)O$R^2$, -(1-4C)alkylN($R^2$)C(O)O$R^2$, -(1-4C)alkylN($R^2$)C(O)N$R^2R^3$, -(1-4C)alkylOC(O)N$R^2R^3$, aryl and heterocyclyl (wherein the heterocyclic ring is linked by a ring carbon atom);
$R^2$ and $R^3$ are independently selected from hydrogen, -O(1-4C)alkyl, -N(1-4C)alkyl, (1-4C)alkyl [optionally substituted

by 1 or 2 $R^8$ groups] ;or an $NR^2R^3$ group forms a morpholine, thiomorpholine (and oxidised versions thereof), pyrrolidine, or piperidine ring and wherein the ring is optionally substituted by 1 or 2 substituents independently selected from chloro, fluoro, hydroxy and methoxy;

$R^8$ is independently selected from hydrogen, hydroxy, (1-4C)alkyl, (1-4C)alkoxy, (1-4C)alkanoyl, $-COCOOR^9$, -C(O)N$(R^9)(R^{10})$, $-NHC(O)R^9$, -N(OH)CHO, $-C(O)NHSO_2$(1-4C)alkyl, $-NHSO_2R^9$, $(R^9)(R^{10})NSO_2$-, $-COCH_2OR^{11}$, $-COCH_2OH$, $(R^9)(R^{10})N$- , $-COOR^9$, $-CH_2OR^9$, $-CH_2COOR^9$ and $-CH_2OCOR^9$;

$R^9$ and $R^{10}$ are independently selected from hydrogen, hydroxy and (1-4C)alkyl) optionally substituted by 1 or 2 $R^{11}$);

$R^{11}$ is (1-4C)alkyl.

In a further aspect of the invention, is provided a compound of the formula (I) or a pharmaceutically-acceptable salt thereof, wherein

A is phenylene;

n is 0;

m is 1;

$R^4$ is chloro;

Y is selected from-$C(O)R^2$ , $-C(O)OR^2$, $-C(O)NR^2R^3$, -(1-4C)alkyl [optionally substituted by 1 or 2 substituents independently selected from hydroxy, $-C{=}NR^2$, (1-4C)alkoxy, aryloxy, heterocyclyloxy, $-S(O)_bR^2$ (wherein b is 0, 1 or 2), -O-$S(O)_bR^2$ (wherein b is 0,1 1 or 2), $-NR^2R^3$, $-N(OH)R^2$, $-NR^2C(=O)R^2$, $-NHOHC(=O)R^2$, $-SO_2NR^2R^3$ , $-N(R^2)SO_2R^2$ , aryl and heterocyclyl], -C(O)NOH, -C(O)NSH, -C(N)OH, $-SO_2H$, $-SO_3H$, $-SO_2NR^2R^3$, -(1-4C)alkyl$C(O)R^2$, -(1-4C)alkylC(O)$OR^2$, -(1-4C)alkyl$OC(O)R^2$, - (1-4C)alkyl$C(O)NR^2R^3$, -(1-4C)alkyl$OC(O)OR^2$, -(1-4C)alkyl$N(R^2)C(O)OR^2$, -(1-4C)alkylN$(R^2)C(O)NR^2R^3$, -(1-4C)alkyl$OC(O)NR^2R^3$-$S(O)_cR^2$ (wherein c is 0, 1 or 2), -(1-4C)alkyl$SC(O)R^2$, -(1-4C)alkyl$SO_2$(2-4C) alkenyl, aryl and heterocyclyl (wherein the heterocyclic ring is linked by a ring carbon atom);

$R^2$ and $R^3$ are independently selected from hydrogen, -O(1-4C)alkyl, -N(1-4C)alkyl, (1-4C)alkyl [optionally substituted by 1 or 2 $R^8$ groups];or an $NR^2R^3$ group forms a 5 or 6 membered saturated, partially saturated or unsaturated ring, optionally containing 1, 2 or 3 additional heteroatoms independently selected from N, O and S, wherein any $-CH_2$- may optionally be replaced by -C(=O)-, and any N or S atom may optionally be oxidised to form an N-oxide or SO or $SO_2$ group respectively, and wherein the ring is optionally substituted by 1 or 2 substituents independently selected from halo, cyano, (1-4C)alkyl, hydroxy, (1-4C)alkoxy and (1-4C)alkyl$S(O)_b$- (wherein b is 0, 1 or 2);

$R^8$ is independently selected from hydrogen, hydroxy, (1-4C)alkyl, (1-4C)alkoxy, amino(1-4C)alkyl, halo(I-4C)alkyl, dihalo (1-4C)alkyl, trihalo(1-4C)alkyl, hydroxy(1-4C)alkyl, dihydroxy(1-4C)alkyl, (1-4C)alkoxy(1-4C)alkoxy, (1-4C)alkoxy(1-4C) alkyl, hydroxy(1-4C)alkoxy, (1-4C)alkanoyl, (1-4C)alkyl$S(O)_b$- (wherein b is 0, 1 or 2, (1-4C)alkyl$S(O)_c$(1-4C)alkyl- (wherein c is 0, 1 or 2), -N(OH)CHO, $-COCOOR^9$, $-C(O)N(R^9)(R^{10})$, $-NHC(O)R^9$, $-C(O)NHSO_2$((1-4C)alkyl $-NHSO_2R^9$, $(R^9)(R^{10}NSO_2$-, $-COCH_2OR^{11}$ , $-COCH_2OH$, $(R^9)(R^{10})N$-, $-COOR^9$, $-CH_2OR^9$, $-CH_2COOR^9$ and-$CH_2OCOR^9$;

$R^9$ and $R^{10}$ are independently selected from hydrogen, hydroxy and (1-4C)alkyl) optionally substituted by 1 or 2 $R^{11}$);

$R^{11}$ is (1-4C)alkyl.

In a further aspect of the invention, is provided a compound of the formula (I) or a pharmaceutically-acceptable salt thereof, wherein

A is phenylene;

n is 0;

m is 1;

$R^4$ is chloro;

Y is selected from-$C(O)R^2$ , $-C(O)OR^2$, $-C(O)NR^2R^3$, -(1-4C)alkyl [optionally substituted by a substituent selected from hydroxy, (1-4C)alkoxy, aryloxy, heterocyclyloxy, $-S(O)_bR^2$ (wherein b is 0, 1 or 2), -O-$S(O)_bR^2$ (wherein b is 0, 1 or 2), $-NR^2R^3$, $-N(OH)R^2$ , $-NR^2C(=O)R^2$, aryl and heterocyclyl], -C(O)NOH, -(1-4C)alkyl$C(O)R^2$, -(1-4C)alkyl$C(O)OR^2$, -(1-4C) alkyl$OC(O)R^2$, - (1-4C)alkyl$C(O)NR^2R^3$ , -(1-4C)alkyl$OC(O)OR^2$, -(1-4C)alkyl$N(R^2)C(O)OR^2$, -(1-4C)alkyl$N(R^2)C(O)NR^2R^3$, -(1-4C)alkyl$OC(O)NR^2R^3$, -(1-4C)alkyl$SC(O)R^2$, -(1-4C)alkyl$SO_2$(2-4C)alkenyl, aryl and heterocyclyl (wherein the heterocyclic ring is linked by a ring carbon atom);

$R^2$ and $R^3$ are independently selected from hydrogen, -O(1-4C)alkyl, -N(1-4C)alkyl, (1-4C)alkyl [optionally substituted by 1 or 2 $R^8$ groups];or an $NR^2R^3$ group forms a morpholine, thiomorpholine (and oxidised versions thereof), pyrrolidine, dihydropyridine, tetrahydropyridine, piperazine or piperidine ring and wherein the ring is optionally substituted by 1 or 2 substituents independently selected from halo, hydroxy and (1-4C)alkoxy;

$R^8$ is independently selected from hydrogen, hydroxy, (1-4C)alkyl, (1-4C)alkoxy, (1-4C)alkanoyl, $-COCOOR^9$, -C(O)N$(R^9)(R^{10})$, $-NHC(O)R^9$, -N(OH)CHO, $-C(O)NHSO_2$(1-4C)alkyl, $-NHSO_2R^9$, $(R^9)(R^{10})NSO_2$-, $-COCH_2OR^{11}$, $-COCH_2OH$, $(R^9)(R^{10})N$-, $-COOR^9$, $-CH_2OR^9$, $-CH_2COOR^9$ and $-CH_2OCOR^9$;

$R^9$ and $R^{10}$ are independently selected from hydrogen, hydroxy and (1-4C)alkyl) optionally substituted by 1 or 2 $R^{11}$);

$R^{11}$ (1-4C)alkyl.

In a further aspect of the invention, is provided a compound of the formula (I) or a pharmaceutically-acceptable salt thereof, wherein

A is phenylene;

n is 0;
m is 1;
$R^4$ is chloro;
Y is selected from-$C(O)R^2$ , -$C(O)OR^2$, -$C(O)NR^2R^3$, -(1-4C)alkyl [optionally substituted by a substituent selected from hydroxy, (1-4C)alkoxy, aryloxy, heterocyclyloxy, -$S(O)_bR^2$ (wherein b is 0, 1 or 2), -O-$S(O)_bR^2$ (wherein b is 0, 1 or 2), -$NR^2R^3$, -$N(OH)R^2$, -$NR^2C(=O)R^2$, aryl and heterocyclyl], -C(O)NOH, -(1-4C)alkylC(O)$R^2$, -(1-4C)alkylC(O)O$R^2$, -(1-4C) alkylOC(O)$R^2$, -(1-4C)alkylC(O)N$R^2R^3$ , -(1-4C)alkylOC(O)O$R^2$, -(1-4C)alkylN($R^2$)C(O)O$R^2$, -(1-4C)alkylN($R^2$)C(O) N$R^2R^3$, -(1-4C)alkylSC(O)$R^2$, -(1-4C)alkylOC(O)N$R^2R^3$, -(1-4C)alkyl$SO_2$(2-4C)alkenyl, aryl and heterocyclyl (wherein the heterocyclic ring is linked by a ring carbon atom);
$R^2$ and $R^3$ are independently selected from hydrogen, -O(1-4C)alkyl, -N(1-4C)alkyl, (1-4C)alkyl [optionally substituted by 1 or 2 $R^8$ groups];or an $NR^2R^3$ group forms a morpholine, thiomorpholine (and oxidised versions thereof), pyrrolidine, or piperidine ring and wherein the ring is optionally substituted by 1 or 2 substituents independently selected from chloro, fluoro, hydroxy and methoxy;
$R^8$ is independently selected from hydrogen, hydroxy, (1-4C)alkyl, (1-4C)alkoxy, (1-4C)alkanoyl, -COCOO$R^9$, -C(O)N ($R^9$)($R^{10}$), -NHC(O)$R^9$, -N(OH)CHO, -C(O)NH$SO_2$(1-4C)alkyl, -NH$SO_2R^9$, ($R^9$)($R^{10}$)N$SO_2$-, -$COCH_2OR^{11}$, -$COCH_2OH$, ($R^9$)($R^{10}$)N- , -COO$R^9$, -$CH_2OR^9$, -$CH_2COOR^9$ and -$CH_2OCOR^9$;
$R^9$ and $R^{10}$ are independently selected from hydrogen, hydroxy and (1-4C)alkyl) optionally substituted by 1 or 2 $R^{11}$);
$R^{11}$ is (1-4C)alkyl.

In a further aspect of the invention, is provided a compound of the formula (I) or a pharmaceutically-acceptable salt thereof, wherein
A is phenylene;
n is 0;
m is 1;
$R^4$ is chloro;
Y is selected from-$C(O)R^2$ , -$C(O)OR^2$, -$C(O)NR^2R^3$, -(1-4C)alkyl [optionally substituted by a substituent selected from hydroxy, (1-4C)alkoxy, aryloxy, heterocyclyloxy, -$S(O)_bR^2$ (wherein b is 0, 1 or 2), -O-$S(O)_bR^2$ (wherein b is 0, 1 or 2), -$NR^2R^3$, -$N(OH)R^2$, -$NR^2C(=O)R^2$, -$SO_2NR^2R^3$, aryl and heterocyclyl], -C(O)NOH, -(1-4C)alkylC(O)$R^2$, -(1-4C)alkylC (O)O$R^2$, -(1-4C)alkylOC(O)$R^2$, -(1-4C)alkylC(O)N$R^2R^3$,- (1-4C)alkylOC(O)O$R^2$, -(1-4C)alkylN($R^2$)C(O)O$R^2$, -(1-4C) alkylN($R^2$)C(O)N$R^2R^3$, -(1-4C)alkylSC(O)$R^2$, -(1-4C)alkylOC(O)N$R^2R^3$, -(1-4C)alkyl$SO_2$(2-4C)alkenyl, -$SO_cR^2$ (wherein c is 0, 1 or 2), aryl and heterocyclyl (wherein the heterocyclic ring is linked by a ring carbon atom);
$R^2$ and $R^3$ are independently selected from hydrogen, heterocyclyl, -O(1-4C)alkyl, -N(1-4C)alkyl, (1-4C)alkyl [optionally substituted by 1 or 2 $R^8$ groups];or an $NR^2R^3$ group forms a morpholine, thiomorpholine (and oxidised versions thereof), pyrrolidine, or piperidine ring and wherein the ring is optionally substituted by 1 or 2 substituents independently selected from chloro, fluoro, hydroxy and methoxy;
$R^8$ is independently selected from hydrogen, hydroxy, (1-4C)alkyl, (1-4C)alkoxy, (1-4C)alkanoyl, -COCOO$R^9$, -C(O)N ($R^9$)($R^{10}$), -NHC(O)$R^9$, -N(OH)CHO, -C(O)NH$SO_2$(1-4C)alkyl, -NH$SO_2R^9$, ($R^9$)($R^{10}$)N$SO_2$-, -$COCH_2OR^{11}$, -$COCH_2OH$, ($R^9$)($R^{10}$)N-, -COO$R^9$, -$CH_2OR^9$, -$CH_2COOR^9$, -$CH_2OCOR^9$, aryl, heterocyclyl, and 5- and 6-membered cyclic acetals and mono- and di-methyl derivatives thereof;
$R^9$ and $R^{10}$ are independently selected from hydrogen, hydroxy and (1-4C)alkyl) optionally substituted by 1 or 2 $R^{11}$);
$R^9$ and $R^{10}$ together with the nitrogen to which they are attached form a 4- to 6-membered ring as hereinbefore defined;
R11 is (1-4C)alkyl.

In a further aspect of the invention, is provided a compound of the formula (I) or a pharmaceutically-acceptable salt thereof, wherein
A is phenylene;
n is 0;
m is 1;
$R^4$ is chloro;
Y is selected from -$C(O)OR^2$, -$C(O)NR^2R^3$, -(1-4C)alkyl [optionally substituted by a substituent selected from hydroxy, (1-4C)alkoxy, -$S(O)_bR^2$ (wherein b is 0, 1 or 2), -O-$S(O)_bR^2$ (wherein b is 0, 1 or 2), -$NR^2R^3$, -$NR^2C(=O)R^2$ and -$SO_2NR^2R^3$], -(1-4C)alkylC(O)$R^2$, -(1-4C)alkylC(O)O$R^2$, -(1-4C)alkylOC(O)$R^2$, -(1-4C)alkylC(O)N$R^2R^3$, -(1-4C)alkylOC (O)O$R^2$, -(1-4C)alkylN($R^2$)C(O)O$R^2$, -(1-4C)alkylN($R^2$)C(O)N$R^2R^3$, -(1-4C)alkylSC(O)$R^2$, -(1-4C)alkylOC(O)N$R^2R^3$, -(1-4C)alkyl$SO_2$(2-4C)alkenyl, -$SO_cR^2$ (wherein c is 0, 1 or 2);
$R^2$ and $R^3$ are independently selected from hydrogen, heterocyclyl, -O(1-4C)alkyl, -N(1-4C)alkyl, (1-4C)alkyl [optionally substituted by 1 or 2 $R^8$ groups]; or an $NR^2R^3$ group forms a morpholine, thiomorpholine (and oxidised versions thereof), pyrrolidine, or piperidine ring and wherein the ring is optionally substituted by 1 or 2 substituents independently selected from chloro, fluoro, hydroxy and methoxy; $R^8$ is independently selected from hydrogen, hydroxy, -C(O)N($R^9$)($R^{10}$), -NHC (O)$R^9$, -COO$R^9$, -$CH_2OR^9$ -$CH_2COOR^9$, -$CH_2OCOR^9$, aryl, heterocyclyl, and 5- and 6-membered cyclic acetals and mono- and di-methyl derivatives thereof;

$R^9$ and $R^{10}$ are independently selected from hydrogen, hydroxy and (1-4C)alkyl) or $R^9$ and $R^{10}$ together with the nitrogen to which they are attached form a 4- to 6-membered ring as hereinbefore defined.

In a further aspect of the invention, is provided a compound of the formula (I) or a pharmaceutically-acceptable salt thereof, wherein
A is phenylene;
n is 0;
m is 1;
$R^4$ is chloro;
Y is selected from -C(O)O$R^2$, -C(O)N$R^2R^3$, -(1-4C)alkyl [optionally substituted by a substituent selected from hydroxy, (1-4C)alkoxy, $-S(O)_bR^2$ (wherein b is 0, 1 or 2), $-O-S(O)_bR^2$ (wherein b is 0, 1 or 2), $-NR^2R^3$, $-NR^2C(=O)R^2$ and $-SO_2NR^2R^3$], -(1-4C)alkylC(O)$R^2$, -(1-4C)alkylC(O)O$R^2$, -(1-4C)alkylOC(O)$R^2$, -(1-4C)alkylC(O)N$R^2R^3$, -(1-4C)alkylOC(O)O$R^2$, -(1-4C)alkylN($R^2$)C(O)O$R^2$, -(1-4C)alkylN($R^2$)C(O)N$R^2R^3$, -(1-4C)alkylSC(O)$R^2$, -(1-4C)alkylOC(O)N$R^2R^3$, -(1-4C)alkyl$SO_2$(2-4C)alkenyl, $-SO_cR^2$ (wherein c is 0, 1 or 2);
$R^2$ and $R^3$ are independently selected from hydrogen, heterocyclyl, -O(1-4C)alkyl, -N(1-4C)alkyl, (1-4C)alkyl [optionally substituted by 1 or 2 $R^8$ groups]; or an $NR^2R^3$ group forms a morpholine, thiomorpholine (and oxidised versions thereof), pyrrolidine, or piperidine ring and wherein the ring is optionally substituted by 1 or 2 substituents independently selected from chloro, fluoro, hydroxy and methoxy;
$R^8$ is independently selected from hydrogen, hydroxy, -C(O)N($R^9$)($R^{10}$), -NHC(O)$R^9$, -COO$R^9$, $-CH_2OR^9$, $-CH_2COOR^9$, $-CH_2OCOR^9$, aryl, heterocyclyl, and 5- and 6-membered cyclic acetals and mono- and di-methyl derivatives thereof;
$R^9$ and $R^{10}$ are independently selected from hydrogen, hydroxy and (1-4C)alkyl) or $R^9$ and $R^{10}$ together with the nitrogen to which they are attached form a morpholine, thiomorpholine (and oxidised versions thereof), pyrrolidine, or piperidine ring.

In a further aspect of the invention, is provided a compound of the formula (I) wherein
A is phenylene;
n is 0;
m is 1;
$R^4$ is chloro;
Y is selected from -C(O)O$R^2$, -C(O)N$R^2R^3$, -(1-4C)alkyl [optionally substituted by a substituent selected from $-S(O)_bR^2$ (wherein b is 0, 1 or 2), $-O-S(O)_bR^2$ (wherein b is 0, 1 or 2), $-NR^2R^3$, $-NR^2C(=O)R^2$ and $-SO_2NR^2R^3$], -(1-4C)alkylC(O)O$R^2$, -(1-4C)alkylOC(O)$R^2$, -(1-4C)alkylC(O)N$R^2R^3$, -(1-4C)alkylSC(O)$R^2$, -(1-4C)alkyl$SO_2$(2-4C)alkenyl and $-SO_cR^2$ (wherein c is 0, 1 or 2);
$R^2$ and $R^3$ are independently selected from hydrogen, heterocyclyl, and (1-4C)alkyl [optionally substituted by 1 or 2$R^8$ groups]; or an $NR^2R^3$ group forms a morpholine, thiomorpholine (and oxidised versions thereof), pyrrolidine, or piperidine ring and wherein the ring is optionally substituted by 1 or 2 substituents independently selected from chloro, fluoro, hydroxy and methoxy;
$R^8$ is independently selected from hydrogen, hydroxy, -C(O)N($R^9$)($R^{10}$), -NHC(O)$R^9$ , -COO$R^9$, aryl, heterocyclyl, and 5- and 6-membered cyclic acetals and mono- and di-methyl derivatives thereof;
$R^9$ and $R^{10}$ are independently selected from hydrogen, hydroxy and (1-4C)alkyl) or $R^9$ and $R^{10}$ together with the nitrogen to which they are attached form a morpholine ring.

In one aspect of the invention is provided a compound of the formula (I) or a pharmaceutically-acceptable thereof, wherein
A is heteroarylene;
n is 0, 1 or 2;
m is 1 or 2;
$R^1$ is selected from fluoro, chloro, methyl, ethyl, methoxy and $-O-CH_2-O-$;
$R^4$ is halo;
Y is selected from-C(O)$R^2$ , -C(O)O$R^2$, -C(O)N$R^2R^3$, -(1-4C)alkyl [optionally substituted by a substituent selected from hydroxy, (1-4C)alkoxy, aryloxy, heterocyclyloxy, $-S(O)_bR^2$ (wherein b is 0, 1 or 2), $-O-S(O)_bR^2$ (wherein b is 0, 1 or 2), $-NR^2R^3$, -N(OH)$R^2$, $-NR^2C(=O)R^2$, aryl and heterocyclyl], -C(O)NOH, -(1-4C)alkylC(O)$R^2$, -(1-4C)alkylC(O)O$R^2$, -(1-4C)alkylOC(O)$R^2$, -(1-4C)alkylC(O)N$R^2R^3$ , -(1-4C)alkylOC(O)O$R^2$, -(1-4C)alkylN($R^2$)C(O)O$R^2$, -(1-4C)alkylN($R^2$)C(O)N$R^2R^3$, -(1-4C)alkylOC(O)N$R^2R^3$, -(1-4C)alkylSC(O)$R^2$, -(1-4C)alkyl$SO_2$(2-4C)alkenyl, aryl and heterocyclyl (wherein the heterocyclic ring is linked by a ring carbon atom);
$R^2$ and $R^3$ are independently selected from hydrogen, -O(1-4C)alkyl, -N(1-4C)alkyl, (1-4C)alkyl [optionally substituted by 1 or 2 $R^8$ groups];or an $NR^2R^3$ group forms a morpholine, thiomorpholine (and oxidised versions thereof), pyrrolidine, dihydropyridine, tetrahydropyridine, piperazine or piperidine ring and wherein the ring is optionally substituted by 1 or 2 substituents independently selected from halo, hydroxy and (1-4C)alkoxy;
$R^8$ is independently selected from hydrogen, hydroxy, (1-4C)alkyl, (1-4C)alkoxy, (1-4C)alkanoyl, -COCOO$R^9$, -C(O)N($R^9$)($R^{10}$), -NHC(O)$R^9$, -N(OH)CHO, -C(O)NH$SO_2$(1-4C)alkyl, $-NHSO_2R^9$, ($R^9$)($R^{10}$)N$SO_2$-, $-COCH_2OR^{11}$, $-COCH_2OH$,

$(R^9)(R^{10})N$- , $-COOR^9$, $-CH_2OR^9$, $-CH_2COOR^9$ and $-CH_2OCOR^9$;
$R^9$ and $R^{10}$ are independently selected from hydrogen, hydroxy and (1-4C)alkyl);
$R^{11}$ is (1-4C)alkyl.

In one aspect of the invention is provided a compound of the formula (I) or a pharmaceutically-acceptable salt thereof, wherein
A is heteroarylene;
n is 0, 1 or 2;
m is 1 or 2;
$R^1$ is selected from fluoro, chloro, methyl, ethyl, methoxy and $-O-CH_2-O-$;
$R^4$ is halo;
Y is selected from-$C(O)R^2$ , $-C(O)OR^2$, $-C(O)NR^2R^3$, -(1-4C)alkyl [optionally substituted by 1 or 2 substituents independently selected from hydroxy, $-C=NR^2$, (1-4C)alkoxy, aryloxy, heterocyclyloxy, $-S(O)_bR^2$ (wherein b is 0, 1 or 2), $-O-S(O)_bR^2$ (wherein b is 0, 1 or 2), $-NR^2R^3$, $-N(OH)R^2$, $-NR^2C(=O)R^2$, $-NHOHC(=O)R^2$, $-SO_2NR^2R^3$, $-N(R^2)SO_2R^2$, aryl and heterocyclyl], -C(O)NOH, -C(O)NSH, -C(N)OH, $-SO_2H$, $-SO_3H$, $-SO_2NR^2R^3$, -(1-4C)alkylC(O)$OR^2$, -(1-4C)alkylOC(O)$R^2$, - (1-4C)alkylC(O)$NR^2R^3$ ,- (1-4C)alkylOC(O)$OR^2$, -(1-4C)alkylN($R^2$)C(O)$OR^2$, -(1-4C)alkylN($R^2$)C(O)$NR^2R^3$, -(1-4C)alkylOC(O)$NR^2R^3$, $-S(O)_cR^2$ (wherein c is 0, 1 or 2), aryl and heterocyclyl (wherein the heterocyclic ring is linked by a ring carbon atom);
$R^2$ and $R^3$ are independently selected from hydrogen, -0(1-4C)alkyl, -N(1-4C)alkyl, (1-4C)alkyl [optionally substituted by 1 or 2 $R^8$ groups];or an $NR^2R^3$ group forms a 5 or 6 membered saturated, partially saturated or unsaturated ring, optionally containing 1, 2 or 3 additional heteroatoms independently selected from N, O and S, wherein any $-CH_2$- may optionally be replaced by -C(=O)-, and any N or S atom may optionally be oxidised to form an N-oxide or SO or $SO_2$ group respectively, and wherein the ring is optionally substituted by 1 or 2 substituents independently selected from halo, cyano, (1-4C)alkyl, hydroxy, (1-4C)alkoxy and (1-4C)alkyl$S(O)_b$- (wherein b is 0, 1 or 2);
$R^8$ is independently selected from hydrogen, hydroxy, (1-4C)alkyl, (1-4C)alkoxy, amino(1-4C)alkyl, halo(1-4C)alkyl, dihalo(1-4C)alkyl, trihalo(1-4C)alkyl, hydroxy(1-4C)alkyl, dihydroxy(1-4C)alkyl, (1-4C)alkoxy(1-4C)alkoxy, (1-4C)alkoxy (1-4C)alkyl, hydroxy(1-4C)alkoxy, (1-4C)alkanoyl, (1-4C)alkyl$S(O)_b$- (wherein b is 0, 1 or 2, (1-4C)alkyl$S(O)_c$((1-4C)) alkyl (wherein c is 0, 1 or 2), -N(OH)CHO, $-COCOOR^9$, $-C(O)N(R^9)(R^{10})$, $-NHC(O)R^9$, $-C(O)NHSO_2$((1-4C)alkyl), $-NHSO_2R^9$, $(R^9)(R^{10})NSO_2$-, $-COCH_2OR^{11}$, $-COCH_2OH$, $(R^9)(R^{10})N$-, $-COOR^9$, $-CH_2OR^9$, $-CH_2COOR^9$ and $-CH_2OCOR^9$;
$R^9$ and $R^{10}$ are independently selected from hydrogen, hydroxy and (1-4C)alkyl);
$R^{11}$ is (1-4C)alkyl.

In a further aspect of the invention, is provided a compound of the formula (I) or a pharmaceutically-acceptable salt thereof, wherein
A is heteroarylene;
n is 0, 1 or 2;
m is 1 or 2;
$R^1$ is selected from fluoro, chloro, methyl, ethyl, methoxy and $-O-CH_2-O-$;
$R^4$ is halo;
Y is selected from-$C(O)R^2$ , $-C(O)OR^2$, $-C(O)NR^2R^3$, -(1-4C)alkyl [optionally substituted by a substituent selected from hydroxy, (1-4C)alkoxy, aryloxy, heterocyclyloxy, $-S(O)_bR^2$ (wherein b is 0, 1 or 2), $-O-S(O)_bR^2$ (wherein b is 0, 1 or 2), $-NR^2R^3$, $-N(OH)R^2$, $-NR^2C(=O)R^2$, aryl and heterocyclyl], -C(O)NOH, -(1-4C)alkylC(O)$OR^2$,- (1-4C)alkylOC(O)$R^2$, -(1-4C)alkylC(O)$NR^2R^3$ , -(1-4C)alkylOC(O)$OR^2$, -(1-4C)alkylN($R^2$)C(O)$OR^2$, -(1-4C)alkylN($R^2$)C(O)$NR^2R^3$, -(1-4C) alkylOC(O)$NR^2R^3$, aryl and heterocyclyl (wherein the heterocyclic ring is linked by a ring carbon atom);
$R^2$ and $R^3$ are independently selected from hydrogen, -O(1-4C)alkyl, -N(1-4C)alkyl, (1-4C)alkyl [optionally substituted by 1 or 2 $R^8$ groups];or an $NR^2R^3$ group forms a morpholine, thiomorpholine (and oxidised versions thereof), pyrrolidine, dihydropyridine, tetrahydropyridine, piperazine or piperidine ring and wherein the ring is optionally substituted by 1 or 2 substituents independently selected from halo, hydroxy and (1-4C)alkoxy;
$R^8$ is independently selected from hydrogen, hydroxy, (1-4C)alkyl, (1-4C)alkoxy, (1-4C)alkanoyl, $-COCOOR^9$, $-C(O)N(R^9)(R^{10})$, $-NHC(O)R^9$, -N(OH)CHO, $-C(O)NHSO_2$(1-4C)alkyl, $-NHSO_2R^9$, $(R^9)(R^{10})NSO_2$-, $-COCH_2OR^{11}$ , $-COCH_2OH$, $(R^9)(R^{10})N$-, $-COOR^9$,$-CH_2OR^9$, $-CH_2COOR^9$ and $-CH_2OCOR^9$;
$R^9$ and $R^{10}$ are independently selected from hydrogen, hydroxy and (1-4C)alkyl);
$R^{11}$ is (1-4C)alkyl.

In a further aspect of the invention, is provided a compound of the formula (I) or a pharmaceutically-acceptable salt thereof, wherein
A is heteroarylene;
n is 0;
m is 1;
$R^4$ is chloro;

Y is selected from-$C(O)R^2$ , $-C(O)OR^2$, $-C(O)NR^2R^3$, -(1-4C)alkyl [optionally substituted by a substituent selected from hydroxy, (1-4C)alkoxy, aryloxy, heterocyclyloxy, $-S(O)_bR^2$ (wherein b is 0, 1 or 2), $-O-S(O)_bR^2$ (wherein b is 0, 1 or 2), $-NR^2R^3$, $-N(OH)R^2$, $-NR^2C(=O)R^2$, aryl and heterocyclyl], -C(O)NOH, -(1-4C)alkylC(O)$OR^2$, -(1-4C)alkylOC(O)$R^2$, -(1-4C)alkylC(O)$NR^2R^3$, -(1-4C)alkylOC(O)$OR^2$, -(1-4C)alkylN($R^2$)C(O)$OR^2$, -(1-4C)alkylN($R^2$)C(O)$NR^2R^3$, -(1-4C) alkylOC(O)$NR^2R^3$, aryl and heterocyclyl (wherein the heterocyclic ring is linked by a ring carbon atom);
$R^2$ and $R^3$ are independently selected from hydrogen, -O(1-4C)alkyl, -N(1-4C)alkyl, (1-4C)alkyl [optionally substituted by 1 or 2 $R^8$ groups]; or an $NR^2R^3$ group forms a morpholine, thiomorpholine (and oxidised versions thereof), pyrrolidine, or piperidine ring and wherein the ring is optionally substituted by 1 or 2 substituents independently selected from chloro, fluoro, hydroxy and methoxy;
$R^8$ is independently selected from hydrogen, hydroxy, (1-4C)alkyl, (1-4C)alkoxy, (1-4C)alkanoyl, $-COCOOR^9$, -C(O)N($R^9$)($R^{10}$), -NHC(O)$R^9$, -N(OH)CHO, $-C(O)NHSO_2$(1-4C)alkyl, $-NHSO_2R^9$, ($R^9$)($R^{10}$)$NSO_2$-, $-COCH_2OR^{11}$, $-COCH_2OH$, ($R^9$)($R^{10}$)N- , $-COOR^9$, $-CH_2OR^9$, $-CH_2COOR^9$ and $-CH_2OCOR^9$;
$R^9$ and $R^{10}$ are independently selected from hydrogen, hydroxy and (1-4C)alkyl) optionally substituted by 1 or 2 $R^{11}$);
$R^{11}$ is (1-4C)alkyl.

In a further aspect of the invention, is provided a compound of the formula (I) or a pharmaceutically-acceptable salt thereof, wherein
A is heteroarylene;
n is 0;
m is 1;
$R^4$ is chloro;
Y is selected from-$C(O)R^2$ , $-C(O)OR^2$, $-C(O)NR^2R^3$, -(1-4C)alkyl [optionally substituted by a substituent selected from hydroxy, (1-4C)alkoxy, aryloxy, heterocyclyloxy, $-S(O)_bR^2$ (wherein b is 0, 1 or 2), $-O-S(O)_bR^2$ (wherein b is 0, 1 or 2), $-NR^2R^3$, $-N(OH)R^2$, $-NR^2C(=O)R^2$, aryl and heterocyclyl], -C(O)NOH, -(1-4C)alkylC(O)$R^2$, -(1-4C)alkylC(O)$OR^2$, -(1-4C) alkylOC(O)$R^2$, - (1-4C)alkylC(O)$NR^2R^3$ , -(1-4C)alkylOC(O)$OR^2$, -(1-4C)alkylN($R^2$)C(O)$OR^2$, -(1-4C)alkylN($R^2$)C(O) $NR^2R^3$, -(1-4C)alkylOC(O)$NR^2R^3$, -(1-4C)alkylSC(O)$R^2$, -(1-4C)alkyl$SO_2$(2-4C)alkenyl, aryl and heterocyclyl (wherein the heterocyclic ring is linked by a ring carbon atom);
$R^2$ and $R^3$ are independently selected from hydrogen, -O(1-4C)alkyl, -N(1-4C)alkyl, (1-4C)alkyl [optionally substituted by 1 or 2 $R^8$ groups]; or an $NR^2R^3$ group forms a morpholine, thiomorpholine (and oxidised versions thereof), pyrrolidine, or piperidine ring and wherein the ring is optionally substituted by 1 or 2 substituents independently selected from chloro, fluoro, hydroxy and methoxy;
$R^8$ is independently selected from hydrogen, hydroxy, (1-4C)alkyl, (1-4C)alkoxy, (1-4C)alkanoyl, $-COCOOR^9$, -C(O)N ($R^9$)($R^{10}$), -NHC(O)$R^9$ , -N(OH)CHO, $-C(O)NHSO_2$(1-4C)alkyl, $-NHSO_2R^9$, ($R^9$)($R^{10}$)$NSO_2$-, $-COCH_2OR^{11}$ , $-COCH_2OH$, ($R^9$)($R^{10}$)N-, $-COOR^9$, $-CH_2OR^9$, $-CH_2COOR^9$ and $-CH_2OCOR^9$;
$R^9$ and $R^{10}$ are independently selected from hydrogen, hydroxy and (1-4C)alkyl) optionally substituted by 1 or 2$R^{11}$);
$R^{11}$ is (1-4C)alkyl.

Particular compounds of the invention are each of the Examples or a pharmaceutically-acceptable salt thereof, each of which provides a further independent aspect of the invention. In a further aspect of the invention there is provided any two or more of the Examples or a pharmaceutically-acceptable salt thereof.

Preferred compounds of the invention are of the formula (1A), wherein Y, $R^1$ to $R^4$, m and n are as defined in any aspect or embodiment described hereinbefore or hereinafter.

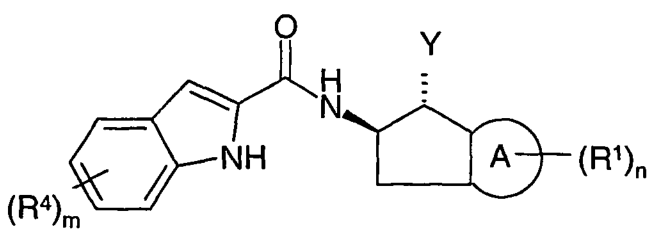

**(1A)**

Another aspect of the present invention provides a process for preparing a compound of formula (1) or a pharmaceutically acceptable salt or an in-vivo hydrolysable ester thereof which process (wherein A, Y, $R^1$, $R^4$, m, r and n are, unless otherwise specified, as defined in formula (1)) comprises of:

a) reacting an acid of the formula (2):

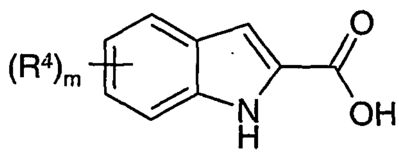

(2)

or an activated derivative thereof; with an amine of formula (3):

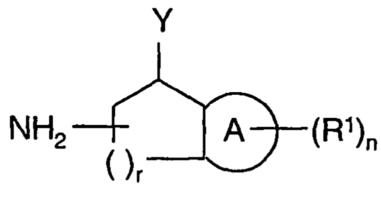

(3)

and thereafter if necessary:

i) converting a compound of the formula (1) into another compound of the formula (1);
ii) removing any protecting groups;
iii) forming a pharmaceutically acceptable salt or in-vivo hydrolysable ester.

Specific reaction conditions for the above reaction are as follows.

*Process a)* Acids of formula (2) and amines of formula (3) may be coupled together in the presence of a suitable coupling reagent. Standard peptide coupling reagents known in the art can be employed as suitable coupling reagents, or for example carbonyldiimidazole, 1-ethyl-3-(3-dimethylaminopropyl)carbodi-imide hydrochloride (EDCI) and dicyclohexyl-carbodiimide (DCCI), optionally in the presence of a catalyst such as 1-hydroxybenzotriazole, dimethylaminopyridine or 4-pyrrolidinopyridine, optionally in the presence of a base for example triethylamine, di-isopropylethylamine, pyridine, or 2,6-di-*alkyl*-pyridines such as 2,6-lutidine or 2,6-di-*tert*-butylpyridine. Suitable solvents include dimethylacetamide, dichloromethane, benzene, tetrahydrofuran and dimethylformamide. The coupling reaction may conveniently be performed at a temperature in the range of -40 to 40˚C.

Suitable activated acid derivatives include acid halides, for example acid chlorides, and active esters, for example pentafluorophenyl esters. The reaction of these types of compounds with amines is well known in the art, for example they may be reacted in the presence of a base, such as those described above, and in a suitable solvent, such as those described above. The reaction may conveniently be performed at a temperature in the range of -40 to 40˚C.

The acids of formula (2) are commercially available or they are known compounds or they are prepared by processes known in the art.

Compounds of formula (3) where Y is $-C(O)R^2$ are known compounds or they are prepared by processes known in the art. When Y is $-C(O)OR^2$, the amines of formula (3) may be prepared according to Scheme 3:

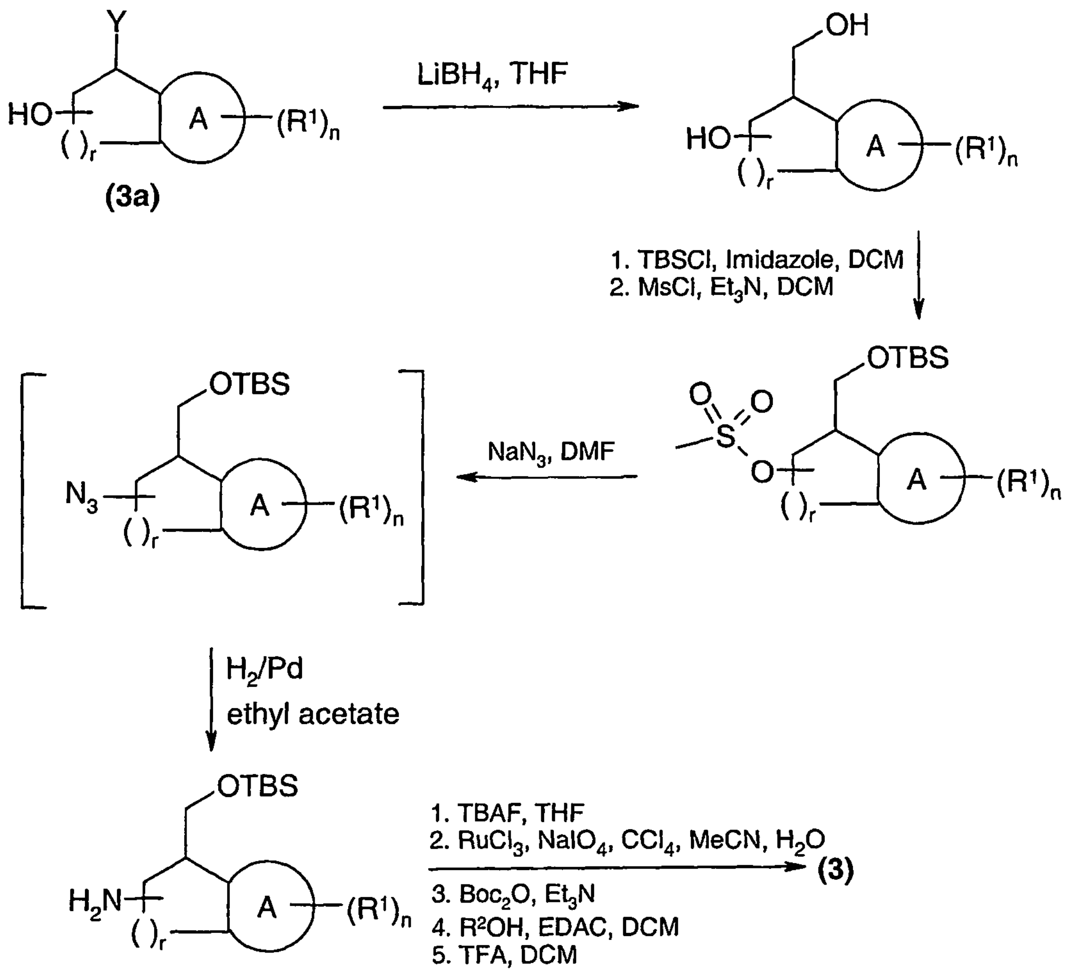


(3a)

(3)

**Scheme 3**

Compounds of formula (3a) are commercially available or they are known compounds or they are prepared by processes known in the art. For example, starting from primary amines of formula (4), in which R is H or a suitable protecting group, one or both of $R^1$ and/or $R^2$ may be introduced by acylation, (for example reacting with acetoxyacetic acid and 1-(3-dimethylaminopropyl)-3-ethyl-carbodiimide hydrochloride (EDAC)), alkylation, reductive alkylation, sulphonation or related processes, followed by O-deprotection when appropriate Alternatively, one or both of $R^1$ and $R^2$ may be obtained by modification of functionality in groups previously thus introduced, by reduction, oxidation, hydrolysis (for example the conversion of an acetoxy group to a hydroxy group), nucleophilic displacement, amidation, or a related process, or a combination of these processes, followed by O-deprotection when appropriate. It will be appreciated that such modifications may include modifications which convert one compound of the formula (1) into another compound of the formula (1).

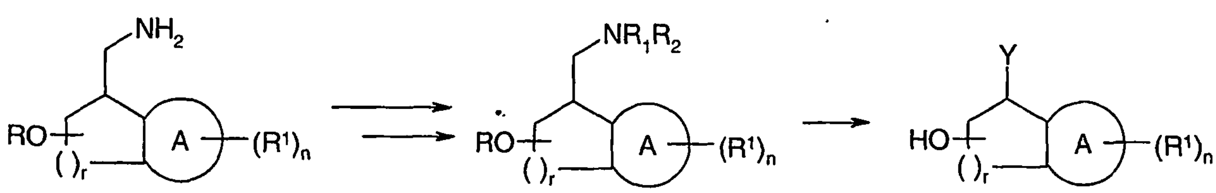

(4)

(3a)

Amines of formula (3) may alternatively be obtained by applying the processes described for the preparation of compounds of formula (3a) to compounds of formula (5) in which W is $NH_2$ or a nitrogen atom with one or two suitable

protecting groups.

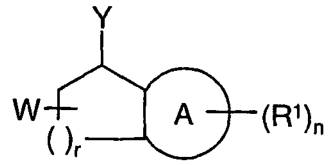

**(5)**

Compounds of the formula (3) may be made by deprotection of compounds of formula (C), which are are commercially available, known compounds, prepared by processes known in the art or may be prepared by the novel route shown in **Scheme 3A,** wherein Y is -SR, or $-CH(CO_2R')_2$, (wherein R represents $R^2$ as defined hereinbefore, or a group which may be converted to a group $R^2$ using standard chemistry; and wherein R' is (1-6C)alkyl).

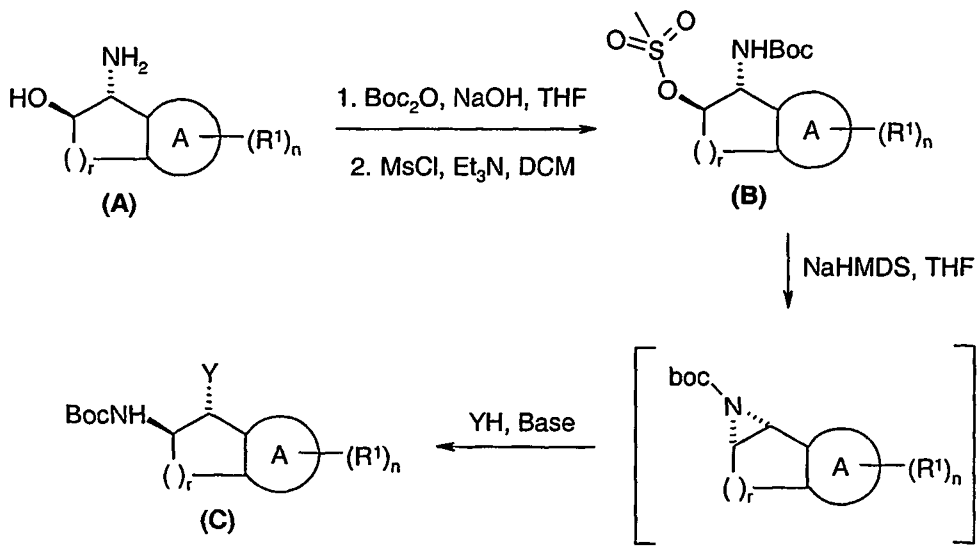


**Scheme 3A**

Compound A (where r = 1; A = phenyl; $R^1$ = H) is commercially available [(1R,2R)-(-)-trans-1-Amino-2-indanol, Cas. Reg. No.:163061-73-2 or [(1S,2S)-(-)-trans-1-Amino-2-indanol Cas. Reg. No.: 13286-59-4]. Compounds of type B can be prepared by methods known in the literature, such as those shown above in Scheme 3A. The conversion of compounds of type B to C is novel and forms a further independent aspect of the invention. It will be appreciated that the process shown in Scheme 3A applies equally to the opposite enantiomers of compounds A, B and C to those shown. In compound (C), where Y = $-CH(CO_2R')_2$, the malonate ester can be transformed to a variety of functional groups by standard methods known in the art. Deprotection of a compound of formula (C) by removal of the Boc group by any suitable process known in the art gives a compound of formula (3).

Suitable conditions for the above processes are exemplified in Intermediates 16 and 22 (compounds of formula (B)) and Intermediates 15, 21, 24 and 25 (compounds of formula (C)).

Compounds of the formula (B) are novel and provide a further independent aspect of the invention. Particular compounds of the formula (B) are those wherein r = 1. Further particular compounds of the formula (B) are those wherein A is phenylene. Still further particular compounds of the formula (B) are those wherein n = 0.

Compounds of the formula (3) where r = 1 and wherein A is heteroarylene can be prepared from suitably functionalised cycloalkyl fused heterocycles. For example, when A is pyridine,

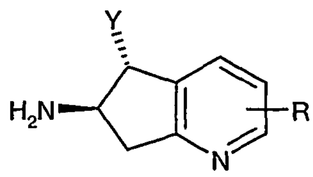

**(3b)**

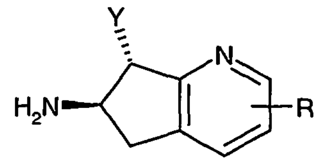

**(3c)**

compounds of formula (3b) and (3c) may be prepared from the corresponding pyrindinone regioisomer according to Scheme 4 :-

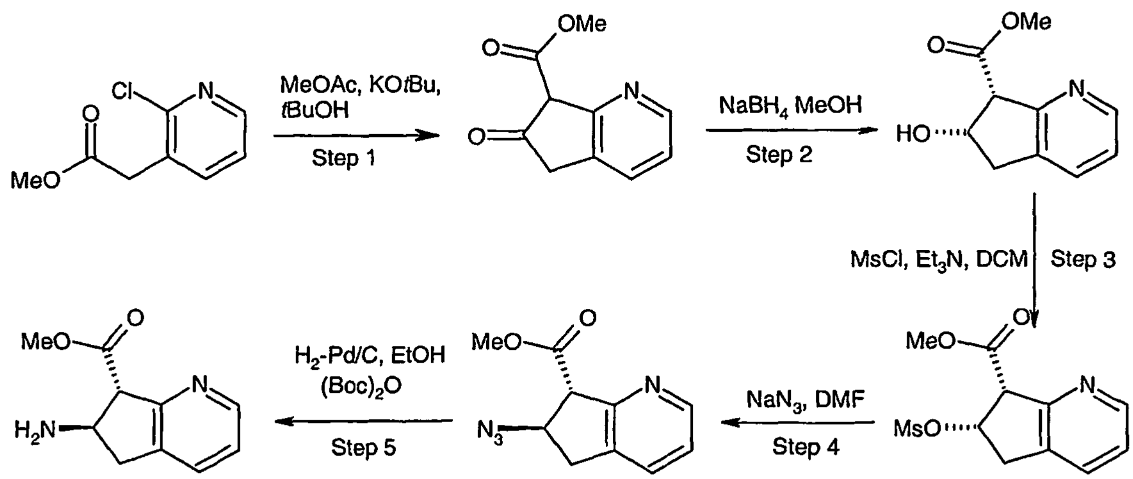


**Scheme 4**

Step 1 is performed on a compound known in the literature (Ger. Offen. **1997**, 78). Steps 2, 3, 4, and 5 are performed using standard techniques known in the art.

It will be appreciated the regiosimers of the beta-ketoesters (Scheme 5) would lead to the preparation of the four possible pyridyl regioisomers of the beta amino ester.

It will be appreciated that, in a similar manner, compounds of the formula (3) wherein A is heteroarylene containing a bridgehead nitrogen can be prepared from the appropriate suitably functionalised cycloalkyl fused heterocycles.

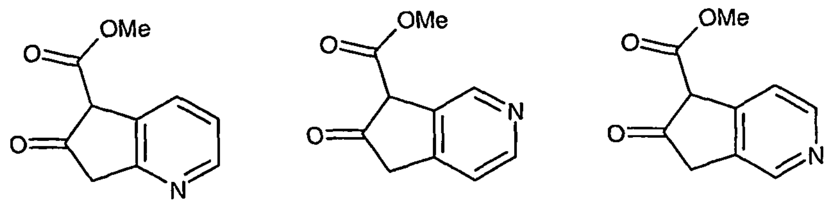

**Scheme 5**

It will be appreciated that the processes described above for formation and modification of Y as $CH_2NR^1R^2$ may be applied similarly whether to make the compound of formula (3) before coupling to the acid of formula (2) or whether to the product of such a coupling.

It will be appreciated that certain of the various ring substituents in the compounds of the present invention, for example $R^1$ and $R^4$, may be introduced by standard aromatic substitution reactions or generated by conventional functional group modifications either prior to or immediately following the processes mentioned above, and as such are included in the process aspect of the invention. Such reactions may convert one compound of the formula (1) into another compound of the formula (1). Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogen group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulphinyl or alkylsulphonyl.

It will also be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or *t*-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *t*-butoxycarbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a *t*-butyl group which may be removed, for example, by treatment with an acid, for example an organic acid such as trifluoroacetic acid, or for example a benzyl group which may be removed, for example, by hydrogenation over a catalyst such as palladium-on-carbon.

The protecting groups may be removed at any convenient stage in the synthesis using conventional techniques well known in the chemical art.

Certain intermediates in the preparation of a compound of the formula (1) are novel and form another aspect of the invention.

As stated hereinbefore the compounds defined in the present invention possesses glycogen phosphorylase inhibitory activity. This property may be assessed, for example, using the procedure set out below.

## Assay

The activity of the compounds is determined by measuring the inhibitory effect of the compounds on glycogen degradation, the production of glucose-1-phosphate from glycogen is monitored by the multienzyme coupled assay, as described in EP 0 846 464 A2, general method of Pesce et al (Pesce, M A, Bodourian, S H, Harris, R C, and Nicholson, J F (1977) Clinical Chemistry 23, 1171 - 1717). The reactions were in 384well microplate format in a volume of 50μl. The change in fluorescence due to the conversion of the co-factor NAD to NADH is measured at 340nM excitation, 465nm emission in a Tecan Ultra Multifunctional Microplate Reader. The reaction is in 50mM HEPES, 3.5mM $KH_2PO_4$, 2.5mM $MgCl_2$, 2.5mM ethylene glycol-bis(b-aminoethyl ether) *N*,*N*,*N'*,*N'*-tetraacetic acid, 100mM KCl, 8mM D-(+)-glucose pH7.2, containing 0.5mM dithiothreitol, the assay buffer solution. Human recombinant liver glycogen phosphorylase α (hrl GPα) 20nM is pre-incubated in assay buffer solution with 6.25mM NAD, 1.25mg type III glycogen at 1.25 mg $ml^{-1}$ the reagent buffer, for 30 minutes. The coupling enzymes, phosphoglucomutase and glucose-6-phosphate dehydrogenase (Sigma) are prepared in reagent buffer, final concentration 0.25Units per well. 20μl of the hrl GPa solution is added to 10μl compound solution and the reaction started with the addition of 20ul coupling enzyme solution. Compounds to be tested are prepared in 10μl 5% DMSO in assay buffer solution, with final concentration of 1 % DMSO in the assay.

The non-inhibited activity of GPa is measured in the presence of 10μl 5% DMSO in assay buffer solution and maximum inhibition measured in the presence of 5mgs $ml^{-1}$ N-ethylmaleimide. After 6 hours at 30°C Relative Fluoresence Units (RFUs) are measured at 340nM excitation, 465nm emission.

The assay is performed at a test concentration of inhibitor of 10μM or 100μM. Compounds demonstrating significant inhibition at one or both of these concentrations may be further evaluated using a range of test concentrations of inhibitor to determine an $IC_{50}$, a concentration predicted to inhibit the enzyme reaction by 50%.

Activity is calculated as follows:-

% inhibition = (1 - (compound RFUs - fully inhibited RFUs)/ (non-inhibited rate RFUs - fully inhibited RFUs)) * 100.

Typical $IC_{50}$ values for compounds of the invention when tested in the above assay are in the range 100μM to 1nM. For example, Example 2 was found to have an $IC_{50}$ of 868nm.

The inhibitory activity of compounds was further tested in rat primary hepatocytes. Rat hepatocytes were isolated by the collagenase perfusion technique, general method of Seglen (P.O. Seglen, Methods Cell Biology (1976) 13 29-83). Cells were cultured on Nunclon six well culture plates in DMEM (Dulbeco's Modified Eagle's Medium) with high level of glucose containing 10% foetal calf serum, NEAA (non essential amino acids), Glutamine, penicillin /streptomycin ((100units/100ug)/ml) for 4 to 6 hours. The hepatocytes were then cultured in the DMEM solution without foetal calf serum and with 10nM insulin and 10nM dexamethasone. Experiments were initiated after 18-20 hours culture by washing the cells and adding Krebs-Henseleit bicarbonate buffer containing 2.5mM $CaCl_2$ and 1% gelatin. The test compound was added and 5 minutes later the cells were challenged with 25nM glucagon. The Krebs-Henseleit solution was removed after 60 min incubation at 37°C, 95%$O_2$/5%$CO_2$ and the glucose concentration of the Krebs-Henseleit solution measured.

According to a further aspect of the invention there is provided a pharmaceutical composition which comprises a compound of the formula (1), or a pharmaceutically acceptable salt or *in vivo* hydrolysable ester thereof, as defined hereinbefore in association with a pharmaceutically-acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents. In one aspect, the compositions of the invention are in a form suitable for oral dosage.

Suitable pharmaceutically acceptable excipients for a tablet formulation include, for example, inert diluents such as lactose, sodium carbonate, calcium phosphate or calcium carbonate, granulating and disintegrating agents such as corn starch or algenic acid; binding agents such as starch; lubricating agents such as magnesium stearate, stearic acid or talc; preservative agents such as ethyl or propyl p-hydroxybenzoate, and anti-oxidants, such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin, or olive oil.

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or

more preservatives (such as ethyl or propyl p-hydroxybenzoate, anti-oxidants (such as ascorbic acid), colouring agents, flavouring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffin or cetyl alcohol. Sweetening agents such as those set out above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavouring and colouring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, an esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in 1,3-butanediol.

Compositions for administration by inhalation may be in the form of a conventional pressurised aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

For further information on formulation the reader is referred to Chapter 25.2 in Volume 5 of Comprehensive Medicinal Chemistry (Corwin Hansch; Chairman of Editorial Board), Pergamon Press 1990.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 2 g of active agent compounded with an appropriate and convenient amount of excipients which may vary from about 5 to about 98 percent by weight of the total composition. Dosage unit forms will generally contain about 1 mg to about 500 mg of an active ingredient. For further information on Routes of Administration and Dosage Regimes the reader is referred to Chapter 25.3 in Volume 5 of Comprehensive Medicinal Chemistry (Corwin Hansch; Chairman of Editorial Board), Pergamon Press 1990.

The compound of formula (1) will normally be administered to a warm-blooded animal at a unit dose within the range 5-5000 mg per square meter body area of the animal, i.e. approximately 0.1-100 mg/kg, and this normally provides a therapeutically-effective dose. A unit dose form such as a tablet or capsule will usually contain, for example 1-250 mg of active ingredient. Preferably a daily dose in the range of 1-50 mg/kg is employed. However the daily dose will necessarily be varied depending upon the host treated, the particular route of administration, and the severity of the illness being treated. Accordingly the optimum dosage may be determined by the practitioner who is treating any particular patient.

The inhibition of glycogen phosphorylase activity described herein may be applied as a sole therapy or may involve, in addition to the subject of the present invention, one or more other substances and/or treatments. Such conjoint treatment may be achieved by way of the simultaneous, sequential or separate administration of the individual components of the treatment. Simultaneous treatment may be in a single tablet or in separate tablets.

For example, in order to prevent, delay or treat type 2 diabetes mellitus, the compounds of the present invention or their pharmaceutically acceptable salts may be administered in combination with one or more of the following agent(s):

1) Insulin and insulin analogues;
2) Insulin secretagogues including sulphonylureas (for example glibenclamide, glipizide), prandial glucose regulators (for example repaglinide, nateglinide) and glucokinase activators
3) Agents that improve incretin action (for example dipeptidyl peptidase IV inhibitors, GLP-1 agonists)
4) Insulin sensitising agents including PPARgamma agonists (for example pioglitazone and rosiglitazone); and agents with combined PPARalpha and gamma activity

5) Agents that modulate hepatic glucose balance (for example metformin, fructose 1, 6 bisphosphatase inhibitors, glycogen synthase kinase inhibitors, glucokinase activators)
6) Agents designed to reduce the absorption of glucose from the intestine (for example acarbose);
7) Agents that prevent the reabsorption of glucose by the kidney (SGLT inhibitors)
8) Agents designed to treat the complications of prolonged hyperglycaemia (for example aldose reductase inhibitors)
9) Anti-obesity agents (for example sibutramine and orlistat);
10) Anti- dyslipidaemia agents such as, HMG-CoA reductase inhibitors (statins, eg pravastatin); PPARα agonists (fibrates, eg gemfibrozil); bile acid sequestrants (cholestyramine); cholesterol absorption inhibitors (plant stanols, synthetic inhibitors); bile acid absorption inhibitors (IBATi) and nicotinic acid and analogues (niacin and slow release formulations);
11) Antihypertensive agents such as, β blockers (eg atenolol, inderal); ACE inhibitors (eg lisinopril); Calcium antagonists (eg. nifedipine); Angiotensin receptor antagonists (eg candesartan), α antagonists and diuretic agents (eg. furosemide, benzthiazide);
12) Haemostasis modulators such as, antithrombotics, activators of fibrinolysis and antiplatelet agents; thrombin antagonists; factor Xa inhibitors; factor VIIa inhibitors); antiplatelet agents (eg. aspirin, clopidogrel); anticoagulants (heparin and Low molecular weight analogues, hirudin) and warfarin;
13) Agents which antagonise the actions of glucagon; and
14) Anti-inflammatory agents, such as non-steroidal anti-inflammatory drugs (eg. aspirin) and steroidal anti-inflammatory agents (eg. cortisone).

According to a further aspect of the present invention there is provided a compound of the formula (1), or a pharmaceutically acceptable salt *or in vivo* hydrolysable ester thereof, as defined hereinbefore, for use in a method of treatment of a warm-blooded animal such as man by therapy.

According to an additional aspect of the invention there is provided a compound of the formula (1), or a pharmaceutically acceptable salt *or in vivo* hydrolysable ester thereof, as defined hereinbefore, for use as a medicament.

According to an additional aspect of the invention there is provided a compound of the formula (1), or a pharmaceutically acceptable salt *or in vivo* hydrolysable ester thereof, as defined hereinbefore, for use as a medicament in the treatment of type 2 diabetes, insulin resistance, syndrome X, hyperinsulinaemia, hyperglucagonaemia, cardiac ischaemia or obesity in a warm-blooded animal such as man.

According to this another aspect of the invention there is provided the use of a compound of the formula (1), or a pharmaceutically acceptable salt or *in vivo* hydrolysable ester thereof, as defined hereinbefore in the manufacture of a medicament for use in the treatment of type 2 diabetes, insulin resistance, syndrome X, hyperinsulinaemia, hyperglucagonaemia, cardiac ischaemia or obesity in a warm-blooded animal such as man.

According to this another aspect of the invention there is provided the use of a compound of the formula (1), or a pharmaceutically acceptable salt or *in vivo* hydrolysable ester thereof, as defined hereinbefore in the manufacture of a medicament for use in the treatment of type 2 diabetes in a warm-blooded animal such as man.

According to a further feature of this aspect of the invention there is provided the use of a compound of formula I in the manufacture of a medicament for use in a method of producing a glycogen phosphorylase inhibitory effect in a warm-blooded animal, such as man, in need of such treatment which comprises administering to said animal an effective amount of a compound of formula (1), According to this further feature of this aspect of the invention there is provided the use of a compound of formula I in the manufacture of a medicament for use in a method of treating type 2 diabetes, insulin resistance, syndrome X, hyperinsulinaemia, hyperglucagonaemia, cardiac ischaemia or obesity in a warm-blooded animal, such as man, in need of such treatment which comprises administering to said animal an effective amount of a compound of formula (1).

According to this further feature of this aspect of the invention there is provide the use of a compound of formula (I) in the manufacture of a medicament for use in a method of treating type 2 diabetes in a warm-blooded animal, such as man, in need of such treatment which comprises administering to said animal an effective amount of a compound of formula (1).

As stated above the size of the dose required for the therapeutic or prophylactic treatment of a particular cell-proliferation disease will necessarily be varied depending on the host treated, the route of administration and the severity of the illness being treated. A unit dose in the range, for example, 1-100 mg/kg, preferably 1-50 mg/kg is envisaged.

In addition to their use in therapeutic medicine, the compounds of formula (1) and their pharmaceutically acceptable salts are also useful as pharmacological tools in the development and standardisation of *in vitro* and *in vivo* test systems for the evaluation of the effects of inhibitors of cell cycle activity in laboratory animals such as cats, dogs, rabbits, monkeys, rats and mice, as part of the search for new therapeutic agents.

In the above other pharmaceutical composition, process, use and medicament manufacture features, the alternative and preferred embodiments of the compounds of the invention described herein also apply.

## Examples

The invention will now be illustrated by the following examples in which, unless stated otherwise:

(i) temperatures are given in degrees Celsius (˚C); operations were carried out at room or ambient temperature, that is, at a temperature in the range of 18-25˚C and under an atmosphere of an inert gas such as argon;
(ii) organic solutions were dried over anhydrous magnesium sulphate; evaporation of solvent was carried out using a rotary evaporator under reduced pressure (600-4000 Pascals; 4.5-30 mmHg) with a bath temperature of up to 60˚C;
(iii) chromatography means flash chromatography on silica gel; thin layer chromatography (TLC) was carried out on silica gel plates; where a Bond Elut column is referred to, this means a column containing 10 g or 20 g or 50 g of silica of 40 micron particle size, the silica being contained in a 60 ml disposable syringe and supported by a porous disc, obtained from Varian, Harbor City, California, USA under the name "Mega Bond Elut SI"; "Mega Bond Elut" is a trademark; where a Biotage cartridge is referred to this means a cartridge containing KP-SIL™ silica, 60μ, particle size 32-63mM, supplied by Biotage, a division of Dyax Corp., 1500 Avon Street Extended, Charlottesville, VA 22902, USA;
(iv) in general, the course of reactions was followed by TLC and reaction times are given for illustration only;
(v) yields are given for illustration only and are not necessarily those which can be obtained by diligent process development; preparations were repeated if more material was required;
(vi) where given, NMR data is in the form of delta values for major diagnostic protons, given in parts per million (ppm) relative to tetramethylsilane (TMS) as an internal standard, determined at 300 MHz using perdeuterio dimethyl sulphoxide (DMSO-$\delta_6$) as solvent unless otherwise indicated, other solvents (where indicated in the text) include deuterated chloroform $CDCl_3$;
(vii) chemical symbols have their usual meanings; SI units and symbols are used;
(viii) reduced pressures are given as absolute pressures in Pascals (Pa); elevated pressures are given as gauge pressures in bars;
(ix) solvent ratios are given in volume: volume (v/v) terms;
(x) The following abbreviations are used:

| | |
|---|---|
| RT | retention time |
| EtOAc | ethyl acetate; |
| MeOH | methanol; |
| EtOH | ethanol; |
| DCM | dichloromethane; |
| HOBT | 1-hydroxybenzotriazole; |
| DIPEA | di-isopropylethylamine; |
| EDCI | 1-ethyl-3-(3-dimethylaminopropyl)carbodi-imide hydrochloride; |
| $Et_2O$ | diethyl ether; |
| THF | tetrahydrofuran; |
| DMF | *N*, *N* dimethylformamide; |
| HATU | *O*-(7-Azabenzotriazol-1-yl)-*N,N,N',N'*-tetramethyluroniumhexafluorophosphate |
| EDAC | 1-(3-dimethylaminopropyl)-3-ethyl-carbodiimide hydrochloride |
| TFA | Trifluoroacetic acid |
| DMTMM | 4-(4,6-Dimethoxy-1,3,5-triazin-2-yl)-4-methylmorpholinium chloride |
| DMA | N, N-dimethylacetamide |
| $NaHCO_3$ | Sodium bicarbonate |
| NaHMDS | Sodium hexamethyldisilazide |
| mCPBA | meta-chloroperbenzoic acid |

Certain intermediates described hereinafter within the scope of the invention may also possess useful activity, and are provided as a further feature of the invention.

### Example 1: Methyl(1*R*,2*R*)-2-{[(5-chloro-1*H*-indole-2-yl)carbonyl]amino}indane-1-carboxylate

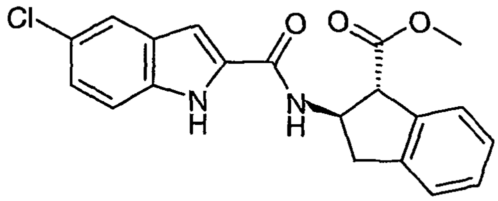

Methyl (1*R*,2*R*)-2-aminoindane-1-carboxylate trifluoroacetic acid salt (**Intermediate 1**; 201 mg, 0.65 mmol), 5-chloro-1*H*-indole-2-carboxylic acid (129 mg, 0.66 mmol) and DIPEA (0.229 ml, 1.3 mmol) were dissolved in DCM (3 mL). HOBT (89mg, 0.66 mmol) and EDCI (134g, 0.69 mmol) were added and the mixture stirred at ambient temperature for 16 h. The volatiles were removed under reduced pressure and the residue dissolved in ethyl acetate (20 ml), washed with water (10 ml), 1N citric acid (5 ml), water (5 ml), saturated aqueous sodium bicarbonate (5 ml), water (5 ml) and brine (5 ml), dried ($MgSO_4$) and the volatiles removed under reduced pressure. The crude residue was purified by silica gel chromatography (3:1, iso-hexane:ethyl acetate) to give the title compound (171 mg, 86%) as a pale yellow solid.
$^1$H NMR (d6-DMSO, 300MHz,) δ: 3.05 (dd, 1H), 3.42 (dd, 1H), 4.1 (d, 1H), 5.0 (m, 1H), 7.1-7.3 (m, 6H), 7.4 (d, 1H), 7.7 (s, 1H), 8.85 (d, 1H), 11.8 (s, 1H);
MS m/z 369, 371 (M+H).

**Example 2: 5-Chloro-*N*-[(1*R*,2*R*)-1-(hydroxymethyl)-2,3-dihydro-1*H*-inden-2-yl]-indole-2-carboxamide**

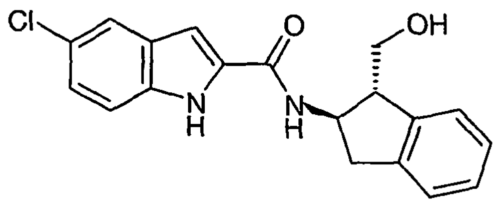

*N*-[(1*R*,2*R*)-1-({[*tert*-Butyl(dimethyl)silyl]oxy}methyl)-2,3-dihydro-1*H*-inden-2-yl]- 5-chloro-1*H*-indole-2-carboxamide (**Intermediate 11**; 611mg, 1.35 mmol) was dissolved in THF (8 ml), tetrabutylammonium fluoride (1.6 ml, 1M in THF, 1.6 mmol) added and the mixture stirred at ambient temperature for 16 h. The volatiles were removed under reduced pressure and the residue dissolved in ethyl acetate (30 ml), washed with water (10 ml), 1N citric acid (10 ml), water (10 ml), saturated aqueous sodium bicarbonate (10 ml), water (10 ml) and brine (10 ml), dried ($MgSO_4$) and the solvent removed under reduced pressure to give the title compound (430 mg, 94%) as a solid.
$^1$H NMR (d6-DMSO, 300MHz) δ: 2.95 (dd, 1H), 3.3 (m, 1H), 3.7 (m, 2H), 4.6 (m, 1H), 4.8 (t, 1H), 7.2 (m, 5H), 7.4 (m, 1H), 7.5 (d, 1H), 7.7 (s, 1H), 8.8 (d, 1H), 11.8 (s, 1H);
MS m/z 341, 343 (M+H).

**Example 3: (1*R*,2*R*)-2-{[(5-chloro-1*H*-indole-2-yl)carbonyl]amino}indane-1-carboxylic acid**

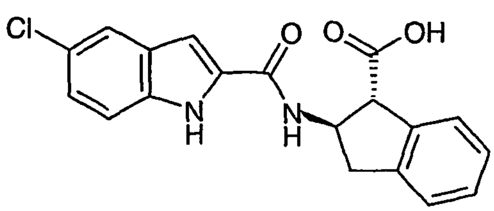

Methyl (1*R*,2*R*)-2-{[(5-chloro-1*H*-indole-2-yl)carbonyl]amino]indane-1-carboxylate ; (**Example 1**; 145 mg, 0.39 mmol) was dissolved in THF (3 mL), hydrochloric acid (0.6 mL, 6M aqueous) added and the mixture stirred at 60 °C for 28 h.

Evaporation of the volatiles under reduced pressure gave a residue which was purified by flash silica gel chromatography (1:1, *iso*-hexane:ethyl acetate) then crystallized( ethyl acetate/toluene) to give the title compound (7 mg, 5%) as an cream solid.
$^1$H NMR(d$^6$DMSO, 400MHz) δ: 2.95 (dd, 1H), 3.4 (dd, 1H), 4.12 (d, 1H), 4.97 (m, 1H), 7.1-7.35 (m, 6H), 7.45 (d, 1H), 7.7 (s, 1H), 8.85 (d, 1H), 11.75 (s, 1H), 12.65 (s, 1H); MS m/z 353, 355 (M-H).

**Example 4: 5-Fluoro-*N*-[(1*R*,2*R*)-1-({[(2-hydroxyethyl)amino]sulfonyl}methyl)-2,3-dihydro-1*H*-inden-2-yl]-1*H*-indole-2-carboxamide**

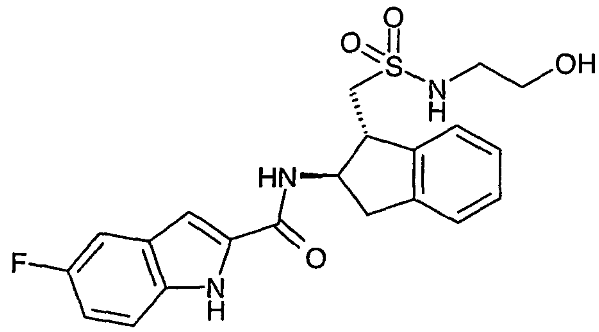

Triethylamine (0.12 mL, 0.87 mmol), HOBT (30 mg, 0.22 mmol), 1-[(1*R*,2*R*)-2-amino-2,3-dihydro-1*H*-inden-1-yl]-*N*-(2-hydroxyethyl)methanesulfonamide hydrochloride (**Intermediate 12**, 61 mg, 0.20 mmol) and EDCI (42 mg, 0.22 mmol) were added to a suspension of 5-fluoroindole-2-carboxylic acid (28 mg, 0.16 mmol) in DMF (5 mL). The reaction was stirred at ambient temperature for approximately 16 h. The reaction mixture was diluted with water (10 mL) and washed with EtOAc (20 mL). The organic layer was washed with water (2x10 mL), 1M aqueous citric acid (10 mL), saturated $NaHCO_3$ (10 mL) and evaporated to give the title compound (54 mg, 78%) as a cream solid.
$^1$H NMR δ: 3.03 (m, 4H), 3.36 (m, 4H), 3.69 (m, 1H), 4.62 (m, 1H), 7.03 (m, 1H), 7.20 (m, 5H), 7.38 (m, 2H), 7.52 (m, 1H), 8.77 (d, 1H), 11.67 (s, 1H); MS m/z 432.2.
The following examples were made by the process of **Example 4** using 1-[(1*R*,2*R*)-2-amino-2,3-dihydro-1*H*-inden-1-yl]-*N*-(2-hydroxyethyl)methanesulfonamide hydrochloride (**Intermediate 12**) and the appropriate commercially available carboxylic acid:

**Example5:*N*-[(1*R*,2*R*)-1-({[(2-Hydroxyethyl)amino]sulfonyl}methyl)-2,3-dihydro-1*H*-inden-2-yl]-5-methyl-1*H*-indole-2-carboxamide**

**Example 6: *N*-[(1*R*,2*R*)-1-({[(2-Hydroxyethyl)amino]sulfonyl}methyl)-2,3-dihydro-1*H*-inden-2-yl]-1*H*-indole-2-carboxamide**

**Example7:5-Chloro-*N*-[(1*R*,2*R*)-1-({[(2-hydroxyethyl)amino]sulfonyl)methyl)-2,3-dihydro-1*H*-inden-2-yl]-1*H*-indole-2-carboxamide**

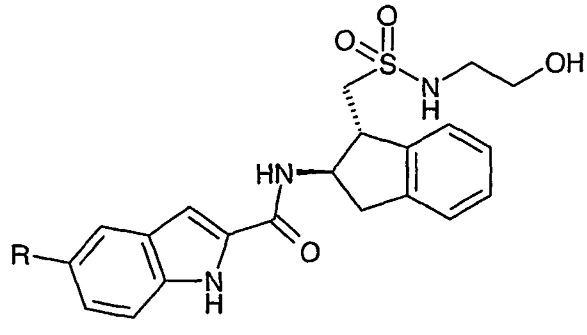

| Example | R | HPLC* RT(mins) | M/z |
|---|---|---|---|
| **5** | Me | 2.17 | 428.2 |
| **6** | H | 2.53 | 414.0 |
| **7** | Cl | 2.25 | 448.2 |
| *HPLC specification: Waters 2790 Separations module, Column: Phenomenex Synergi 4u MAX-RP 8A (50x2 mm), Mobile phase: Acetonitrile:Water:Formic acid, 49.5:49.5:1, Flow rate/operating pressure: 1.1ml/min at 200psi) | | | |

The following examples were made by the process of **Example 4**, using 3-({[(1*R*,2*R*)-2-amino-2,3-dihydro-1*H*-inden-1-yl]methyl}sulfonyl)propan-1-ol hydrochloride (**Intermediate 18**) and the appropriate commercially available carboxylic acid:

**Exampel 8: 5-Fluoro-*N*-((1*R*,2*R*)-1-{[(3-hydroxypropyl)sulfonyl]methyl}-2,3-dihydro-1*H*-inden-2-yl)-1*H*-indole-2-carboxamide**

**Example 9: *N*-((1*R*,2*R*)-1-{[(3-Hydroxypropyl)sulfonyl]methyl]-2,3-dihydro-1*H*-inden-2-yl)-5-methyl-1*H*-indole-2-carboxamide**

**Example 10: *N*-((1*R*,2*R*)-1-{[(3-Hydroxypropyl)sulfonylmethyl}-2,3-dihydro-1*H*-inden-2-yl)-1*H*-indole-2-carboxamide**

**Example 11: 5-Chloro-*N*-((1*R*,2*R*)-1-{[(3-hydroxypropyl)sulfonyl]methyl}-2,3-dihydro-1*H*-inden-2-yl)-1*H*-indole-2-carboxamide**

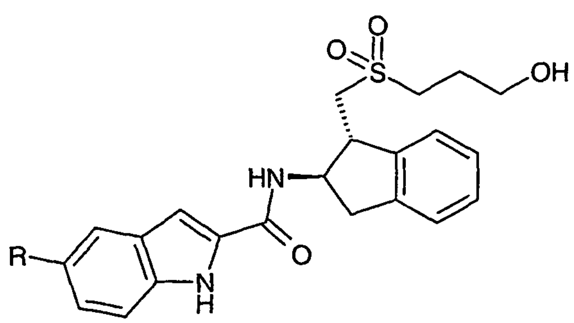

| Example | R | NMR | HPLC* RT(mins) | M/z |
|---|---|---|---|---|
| **8** | F | 1.83 (m, 2M, 2.97 (m, 1H), 3.23 (m, 3H), 3.50 (m, 4H), 3.80 (m, 1H), 4.63 (m, 2H), 7.03 (m, 1H), 7.13 (s, 1H), 7.23 (m, 3H), 7.40 (m, 2H), 7.49 (m, 1H), 8.75 (d, 1H), 11.66 (s, 1H) | - | 431.2 |
| **9** | Me | - | 2.15 | 427.2 |
| **10** | H | - | 2.01 | 413.2 |

(continued)

| Example | R | NMR | HPLC* RT(mins) | M/z |
|---|---|---|---|---|
| **11** | Cl | - | 2.21 | 447.2 |
| *HPLC specification: Waters 2790 Separations module, Column: Phenomenex Synergi 4u MAX-RP 8A (50x2 mm), Mobile phase: Acetonitrile:Water:Formic acid, 49.5:49.5:1, Flow rate/operating pressure: 1.1ml/min at 200psi). | | | | |

**Example 12: [((1*R*,2*R*)-2-{[(5-Chloro-1*H*-indol-2-yl)carbonyl]amino}-2,3-dihydro-1*H*-inden-1-yl)thio]acetic acid**

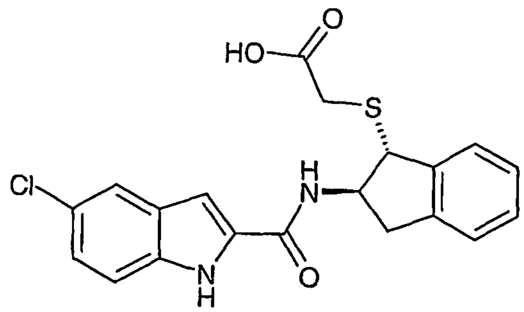

Methyl [((1*R*,2*R*)-2-{[(5-chloro-1*H*-indol-2-yl)carbonyl]amino}-2,3-dihydro-1*H*-inden-1-yl)thio]acetate (**Example 13**; 1.15 g, 2.8 mmol) was dissolved in MeOH/ THF (40 mL) next was added 2M aqueous sodium hydroxide (2.78 mL, 5.5 mmol) before stirring for 18 h. The solution was stripped to dryness before dissolving in EtOAc (90 mL) and water (50 mL) and acidifying with 2M aqueous hydrochloric acid (3 mL), the organic layer was separated then washed with brine (50 mL) and evaporated to give the title compound (1.11 g, 100%) as a cream foam.

$^1$H NMR δ: 2.97 (m, 1H), 3.54 (m, 3H), 4.51 (m, 1H), 4.67 (m, 1H), 7.16 (m, 2H), 7.29 (m, 4H), 7.41 (d, 1H), 7.65 (s, 1H), 8.84 (d, 1H), 11.79 (s, 1H), 12.47 (bs, 1H); MS m/z 401.3.

**Example 13: Methyl [((1*R*,2*R*)-2-{[(5-chloro-1*H*-indol-2-yl)carbonyl]amino}-2,3-dihydro-1*H*-inden-1-yl)thio]acetate**

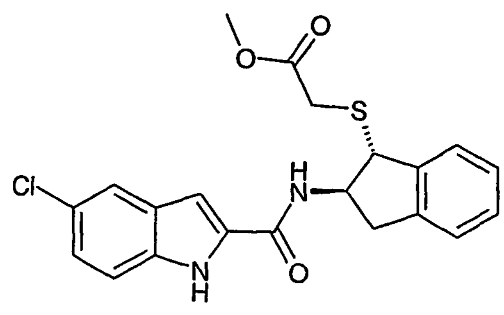

Methyl ({(1*R*,2*R*)-2-[(*tert*-butoxycarbonyl)amino]-2,3-dihydro-1*H*-inden-1-yl}thio)acetate (**Intermediate 21**; 2.43 g, 7.2 mmol) was dissolved in DCM (25 mL) before adding HCl (4M in dioxane, 25 mL) and stirring at ambient temperature for 1 h. The solvent was evaporated before slurrying in ether (80 mL) and filtration to give a white solid. This solid (1.00 g, 3.6 mmol), triethylamine (2.23 mL, 16 mmol), HOBT (542 mg, 4.0 mmol), methyl {[(1*R*,2*R*)-2-amino-2,3-dihydro-1*H*-inden-1-yl]thio}acetate and EDAC (771 mg, 4.0 mmol) were added to a suspension of 5-Chloroindole-2-carboxylic acid (715 mg, 3.6 mmol) in DMF (30 mL). The reaction was stirred at ambient temperature for approximately 16 h. The reaction mixture was diluted with water (50 mL) and washed with EtOAc (2 x 50 mL). The organic layers were combined and washed with water (2 x 50 mL), 1M aqueous citric acid (50 mL), saturated $NaHCO_3$ (50 mL) and evaporated to give

the title compound (1.38g, 91%) as a cream foam.
$^1$H NMR δ: 2.98 (m, 1H), 3.41 (m, 1H), 3.65 (m, 5H), 4.50 (d, 1H), 4.69 (m, 1H), 7.16 (m, 2H), 7.26 (m, 4H), 7.41 (d, 1H), 7.67 (s, 1H), 8.83 (d, 1H), 11.79 (s, 1H); MS m/z 415.2.

**Example 14: 5-Fluoro-*N*-((1*R*,2*R*)-1-{[(2-hydroxyethyl)sulfonyl]methyl}-2,3-dihydro-1*H*-inden-2-yl)-1*H*-indole-2-carboxamide**

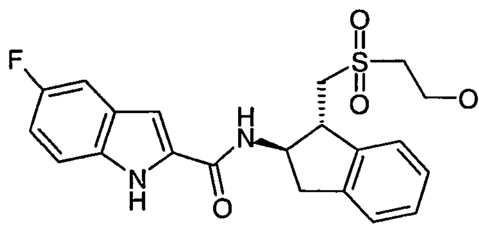

To a solution of 5-fluoro-1*H*-indole-2-carboxylic acid (89.5 mg, 0.5 mmol), 2-({[(1*R*,2*R*)-2-amino-2,3-dihydro-1*H*-inden-1-yl]methyl}sulfonyl)ethanol hydrochloride (**Intermediate 17**, 145 mg , 0.5 mmol), HOBT (81 mg,0.6 mmol) and DIPEA (129 mg, 1.0 mmol) in DMF (2 mL) was added EDCI (143 mg, 0.75 mmol) and the mixture stirred at ambient temperature for 2h. The product was isolated from the reaction mixture by reverse phase HPLC (5-95% acetonitrile / water gradient containing 0.2% TFA) to give the title compound (103 mg, 49.5%) as a white solid.
$^1$H NMR δ: 2.95 (dd, 1H), 3.3 (m, 3H), 3.55 (d, 2H), 3.85 (m, 3H), 4.65 (m, 1H), 5.15 (t, 1H), 7.0 (t, 1H), 7.15 (s, 1H), 7.25 (m, 3H), 7.4 (m, 2H), 7.5 (m, 1H), 8.7 (d, 1H), 11.65 (s,1H); MS m/z 417.
The following examples were made by the process of **Example 14**, using 2-({[(1*R*,2*R*)-2-amino-2,3-dihydro-1*H*-inden-1-yl]methyl}sulfonyl)ethanol hydrochloride (**Intermediate 17**) as the amine and the appropriate commercially available carboxylic acid:

**Example 15: 5-Chloro-*N*-((1*R*,2*R*)-1-{[(2-hydroxyethyl)sulfonyl]methyl}-2,3-dihydro-1*H*-inden-2-yl)-1*H*-indole-2-carboxamide**

**Example 16: *N*-((1*R*,2*R*)-1-{[(2-Hydroxyethyl)sulfonyl]methyl}-2,3-dihydro-1*H*-inden-2-yl)-5-methyl-1*H*-indole-2-carboxamide**

**Example 17: *N*-((1*R*,2*R*)-1-{[(2-Hydroxyethyl)sulfonyl]methyl)-2,3-dihydro-1*H*-inden-2-yl)-1*H*-indole-2-carboxamide**

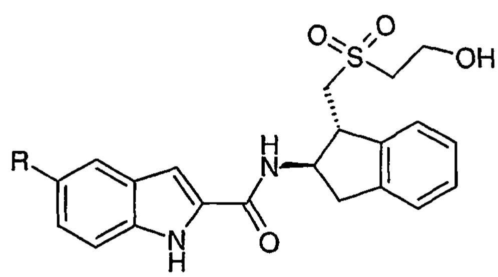

| Example | R | $^1$H NMR | MS m/z |
|---|---|---|---|
| **15** | Cl | 2.95 (dd, 1H), 3.3 (m, 3H), 3.55 (d, 2H), 3.85 (m, 3H), 4.65 (m, 1H), 5.1 (t, 1H), 7.2 (m, 5H), 7.4 (d, 1H), 7.5 (m, 1H), 7.7 (s, 1H), 8.75 (d, 1H), 11.76 (s, 1H); | 433 |

(continued)

| Example | R | $^1H$ NMR | MS m/z |
|---|---|---|---|
| **16** | Me | 2.35 (s,3H), 2.95 (dd, 1H), 3.3 (m, 3H), 3.55 (m, 2H), 3.8 (m, 3H), 4.6 (m, 1H), 5.1 (t, 1H), 7.0 (d, 1H), 7.05 (s, 1H), 7.2 (m, 3H), 7.3 (d, 1H), 7.37 (s, 1H), 7.5 (m, 1H), 8.6 (d, 1H), 11.4 (s, 1H); | 413 |
| **17** | H | 2.95 (dd, 1H), 3.3 (m, 3H), 3.6 (d, 2H), 3.85 (m, 3H), 4.6 (m, 1H), 7.0 (t, 1H), 7.2 (m, 5H), 7.4 (d, 1H), 7.5 (m, 1H), 7.6 (d, 1H), 8.7 (d, 1H), 11.54 (s, 1H); | 399 |

**Example 18: *N*-{(1*R*,2*R*)-1-[(2-Amino-2-oxoethyl)thio]-2,3-dihydro-1*H*-inden-2-yl}-5-chloro-1*H*-indole-2-carboxamide**

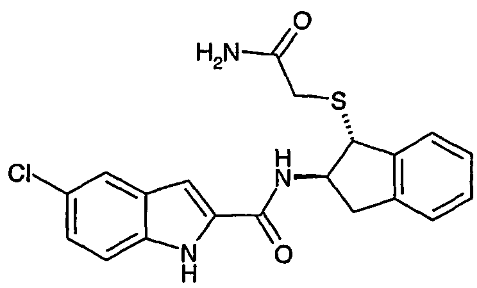

Methyl [((1*R*,2*R*)-2-([(5-chloro-1*H*-indol-2-yl)carbonyl]amino}-2,3,dihydro-1*H*-inden-1-yl)thio]acetate (**Example 13**, 213 mg, 0.52 mmol) was dissolved in 7M ammonia in MeOH (5 mL) before heating at 130 ˚C for 30 mins in a microwave. The solution was evaporated to give the title compound (179 mg, 91%) as a brown foam.
$^1$HNMR δ: 2.96 (dd, 1H), 3.33 (m, 3H), 4.52 (d, 1H), 4.66 (m, 1H), 7.06 (s, 1H), 7.16 (m, 2H), 7.26 (m, 3H), 7.40 (m, 3H) 7.66 (s, 1H), 8.87 (d, 1H), 11.78 (s, 1H); MS m/z 400.3.

**Intermediate 1: Methyl (1*R*,2*R*)-2-aminoindane-1-carboxylate trifluoroacetic acid salt**

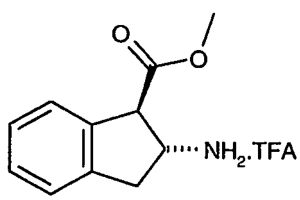

Methyl (1*R*, 2*R*)-2-[(*tert*-butoxycarbonyl)amino]indane-1-carboxylate (**Intermediate 2**; 1.3g, 4.43 mmol) was dissolved in DCM (5 mL), TFA (5 mL) added and the mixture stirred at ambient temperature for 4 h. The volatiles were removed under reduced pressure to give the title compound (1.35 g, 100%) as a foam. $^1$H NMR ($CDCl_3$) δ: 3.1(dd, 1H), 3.45(dd, 1H), 3.74(s, 3H), 4.27(d, 1H), 4.4(m, 1H), 7.3(m, 1H).

**Intermediate 2: Methyl (1*R*,2*R*)-2-[(*tert*-butoxycarbonyl)amino]indane-1-carboxylate**

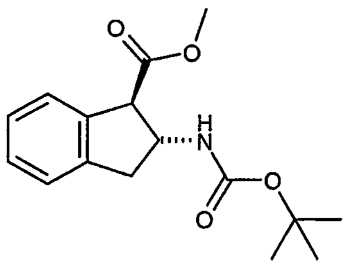

(1*R*,2*R*)-2-[(*tert*-Butoxycarbonyl)amino]indane-1-carboxylic acid (**Intermediate 3**; 1.85 g, 6.65 mmol) was dissolved in methanol (25 mL) and isohexane (25 mL), trimethylsilyldiazomethane (7.0 mL, 2.0M in hexane, 1.4 mmol) was added and the mixture stirred at ambient temperature for 24 h. The volatiles were removed by evaporation and the crude residue purified by silica gel chromatography (8:1, iso-hexane:ethyl acetate) to give the title compound (1.3 g, 68%) as a foam.

1H NMR ($CDCl_3$) δ**:** 1.42(s, 9H), 2.81(dd, 1H), 3.48(dd, 1H), 3.75(s, 3H), 3.97(d, 1H), 4.75(m, 2H), 7.25(m, 4H).

**Intermediate 3: (1*R*,2*R*)-2-[(*tert*-Butoxycarbonyl)amino]indane-1-carboxylic acid**

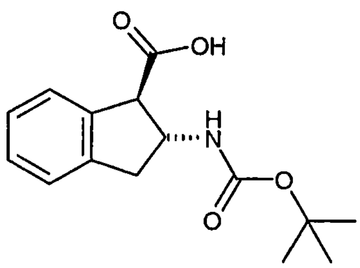

To a solution of *tert*-butyl [(1*R*,2*R*)-1-(hydroxymethyl)-2,3-dihydro-1*H*-inden-2-yl]carbamate (**Intermediate 4**; 1.75 g, 6.65 mmol) in $CCl_4$ (20 mL) and $CH_3CN$ (20 mL) was added sodium metaperiodate (5.7 g, 26.6 mmol) in water (30 mL) and ruthenium trichloride hydrate (138 mg, 0.665 mmol) and the mixture stirred at ambient temperature for 3 h. Ethyl acetate (100 mL) was added, the organic phase separated, washed with water (2 x 10 mL), brine (25 mL), dried ($MgSO_4$), filtered and the volatiles removed under reduced pressure to give the title compound (1.9 g, 100%) as a brown foam.

$^1$H NMR δ: 1.39 (s, 9H), 2.78 (dd, 1H), 3.02 (dd, 1H), 3.85 (d, 1H), 4.5 (m, 1H), 7.2 (m, 4H).

**Intermediate 4: *tert*-Butyl [(1*R*,2*R*)-1-(hydroxymethyl)-2,3-dihydro-1*H*-inden-2-yl]carbamate**

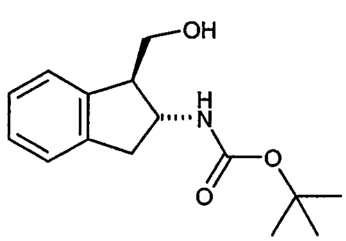

Tetrabutylammonium fluoride (10.0 mL, 2.0M in THF, 20.0 mmol) was added to a solution of *tert*-butyl [(1*R*,2*R*)-1-({[*tert*-butyl(dimethyl)silyl]oxy}methyl)-2,3-dihydro-1*H*-inden-2-yl]carbamate (**Intermediate 5**; 4.1 g, 10.9 mmol) in THF (50 mL) and stirred at ambient temperature for 4 h. The volatiles were removed under reduced pressure and the residue dissolved in ethyl acetate (100 mL), washed with water (2 x 50 mL), brine (50 mL), dried ($MgSO_4$) and the volatiles

removed under reduced pressure. The crude residue was triturated (4:1, iso-hexane:ethyl acetate), filtered and dried to give the title compound (1.5 g, 54%) as white solid.
$^1$H NMR δ: 1.44 (s, 9H), 2.78 (dd, 1H), 3.15 (m, 2H), 3.61 (m, 1H), 3.75 (m, 1H), 4.07 (m, 1H), 4.7 (m, 1H), 7.19 (m, 4H), 7.37 (m, 1H).

**Intermediate 5: *tert*-Butyl [(1*R*,2*R*)-1-({[*tert*-butyl(dimethyl)silyl]oxy}methyl)-2,3-dihydro-1*H*-inden-2-yl]carbamate**

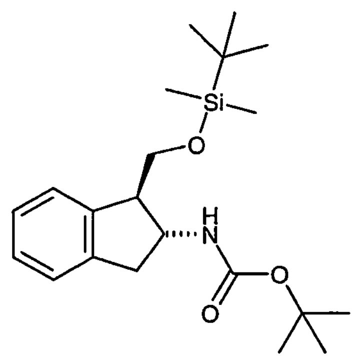

(1*R*,2*R*)-1-({([*tert*-Butyl(dimethyl)silyl]oxy}methyl)-2,3-dihydro-1*H*-inden-2-yl]amine (**Intermediate 6**; 3.1 g, 11.2 mmol) and triethylamine (3.1 mL, 22.4 mmol) were dissolved in DCM (40 mL). Di-*tert*-butyl dicarbonate (2.9 g, 13.4 mmol) in DCM (10 mL) was added and the mixture stirred at ambient temperature for 24 h. The volatiles were removed under reduced pressure and the residue dissolved in ethyl acetate (75 mL), washed with water (2 x 50 mL), brine (50 mL), dried ($MgSO_4$) and the volatiles removed under reduced pressure. The crude residue was purified by silica gel chromatography (16:1, iso-hexane:ethyl acetate) to give the title compound (4.2 g, 100%) as a colourless oil.
1H NMR δ: 0.3 (d, 6H), 0.85 (s, 9H), 1.42 (s, 9H), 2.75 (dd, 1H), 3.15 (m, 2H), 3.79 (m, 1H), 3.95 (m, 1H), 4.05 (m, 1H), 7.15 (m, 4H), 7.3 (m, 1H).

**Intermediate 6: [(1*R*,2*R*)-1-({[*tert*-Butyl(dimethyl)silyl]oxy}methyl)-2,3-dihydro-1*H*-inden-2-yl]amine**

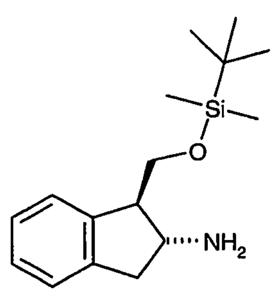

(1*S*,2*S*)-1-({[*tert*-Butyl(dimethyl)silyl]oxy}methyl)-2,3-dihydro-1*H*-inden-2-yl methanesulfonate (**Intermediate 7**; 7.2g, 20.2 mmol) was dissolved in DMA (50 mL), sodium azide (3.94 g, 60.6 mmol) added and the mixture stirred at 60 ˚C for 7 h. The mixture was poured into ethyl acetate (250 mL), washed with water (6 x 75 mL), brine (100 mL) and dried ($MgSO_4$). Palladium on carbon (500 mg, 10% w/w) was added, the mixture stirred under a hydrogen atmosphere for 6h, filtered through Celite and the volatiles removed under reduced pressure to give the title compound (5.2 g, 93%) as a pale brown oil.
1H NMR δ: 0.07 (d, 6H), 0.9 (s, 9H), 2.58 (dd, 1H), 2.89 (m, 1H), 3.1 (dd, 1H), 3.3 (broad s, 2H), 3.41 (m, 1H), 3.85 (m, 2H), 7.2 (m, 4H).

**Intermediate 7: (1*S*,2*S*)-1-({[*tert*-Butyl(dimethyl)silyl]oxy}methyl)-2,3-dihydro-1*H*-inden-2-yl methanesulfonate**

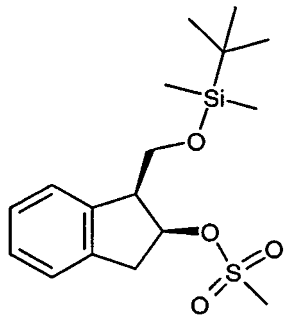

(1*S*,2*S*)-1-({[*tert*-Butyl(dimethyl)silyl]oxy}methyl)indan-2-ol (**Intermediate 8**; 6.3 g, 22.65 mmol) and triethylamine (4.7 mL, 34.0 mmol) were dissolved in DCM (90 mL) at 5˚C. Methanesulfonyl chloride (2.86 g, 24.9 mmol) in DCM (10 mL) was added and the mixture stirred at ambient temperature for 2h. The volatiles were removed under reduced pressure and the residue dissolved in ethyl acetate (150 mL), washed with water (2x50 mL), brine (50 mL), dried ($MgSO_4$) and the volatiles removed under reduced pressure. The crude residue was purified by silica gel chromatography (6:1, iso-hexane:ethyl acetate) to give the title compound (7.2 g, 89%) as a colourless oil.
1H NMR δ: 0.03 (d, 6H), 0.85 (s,9H), 3.19 (s, 3H), 3.21 (m, 2H), 3.45 (m, 1H), 3.95 (m, 2H), 5.45 (m, 1H), 7.22 (m, 4H).

**Intermediate 8: (1*S*,2*S*)-1-({[*tert*-Butyl(dimethyl)silyl]oxy}methyl)indan-2-ol**

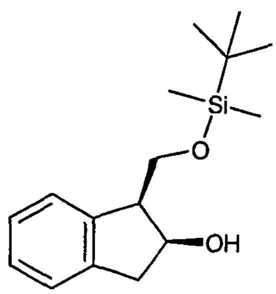

(1*R*,2S)-1-(Hydroxymethyl)-1,3-dihydro-2*H*-inden-2-one (**Intermediate 9**; 9.0 g, 54.8 mmol) and imidazole (4.5 g, 65.8 mmol) were dissolved in DCM (75 mL) at 10 ˚C. *tert*- Butyldimethylchlorosilane (9.1 g, 60.3 mmol) in DCM (25 mL) was added, the mixture allowed to warm to ambient temperature and stirred for 2 h. The volatiles were removed under reduced pressure and the residue dissolved in ethyl acetate (150 mL), washed with water (2 x 50 mL), brine (50 mL), dried ($MgSO_4$) and the volatiles removed under reduced pressure. The crude residue was purified by silica gel chromatography (16:1, iso-hexane:ethyl acetate) to give the title compound (9.5 62%) as a colourless oil. 1H NMR δ: 0.03 (d, 6H), 0.9(s, 9H), 2.78(dd, 1H), 3.0(dd, 1H), 3.1(m, 1H), 3.9(m, 2H), 4.54(m, 1H), 4.68(d, 1H), 7.2(m, 4H).

**Intermediate 9: (1*R*,2*S*)-1-(Hydroxymethyl)-1,3-dihydro-2*H*-inden-2-one**

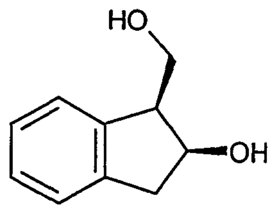

Methyl (1*R*,2*S*)-2-hydroxyindane-1-carboxylate (**Intermediate 10**; 10.56 g, 55.0 mmol) was dissolved in dry THF (100 mL) under a nitrogen atmosphere at 0 °C. $LiBH_4$ (55.0 mL, 2.0M in THF, 110.0 mmol) was added and the reaction stirred between 0 to 5 °C for 0.5 h, allowed to warm to ambient temperature and stirred for a further 2h. The mixture was poured into saturated $NaHCO_3$, extracted with ethyl acetate (200 mL) and the organic phase washed with water (2 x 50 mL), brine (50 mL) and dried ($MgSO_4$). The volatiles were removed by evaporation under reduced pressure to give the title compound (9.1g, 93%) as a colourless oil.
$^1$H NMR δ: 2.7 (m, 1H), 2.95 (m, 1H), 3.05 (m, 1H), 3.55 (m, 1H), 3.8 (m, 1H), 4.55 (m, 3H), 7.2 (m,4H).

**Intermediate 10: Methyl (1*R*,2*S*)-2-hydroxyindane-1-carboxylate**

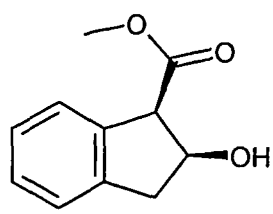

(Reference:Didier, E et al. Tetrahedron 47(27), 4941-4958, 1991) De-ionised water (20 L) was warmed to 34°C, bakers yeast (3 Kg) added and the mixture stirred for 0.5hr. Methyl 2-oxoindane-1-carboxylate (40g, 0.21 mmol) was added, the suspension stirred for 3 days and filtered through Celite. The aqueous filtrate was extracted with ethyl acetate (4 x 2.5L) and the organic extracts dried ($MgSO_4$), filtered and the volatiles removed by evaporation under reduced pressure. The crude residues were purified by flash silica gel chromatography (4:1 iso-hexane:ethyl acetate) the solvent evaporated and the resultant solid recrystallised from iso-hexane/ethyl acetate to give the title compound (10.8 g, 27%) as colourless needles.
Mp = 72.5-73.5°C (lit = 73.2°C)
$[\propto]_D$ = +48.7° (C=1.0, $CHCl_3$) (lit=+48.3°)
1H NMR δ: 2.85(dd, 1H), 3.04(dd, 1H), 3.61(s, 3H), 4.1(d, 1H), 4.76(m, 1H), 5.2(d, 1H), 7.2(m, 4H).

**Intermediate 11: *N*-[(1*R*,2*R*)-1-({[*tert*-Butyl(dimethyl)silyl]oxy}methyl)-2,3-dihydro-1*H*-inden-2-yl]-5-chloro-1*H*-indole-2-carboxamide**

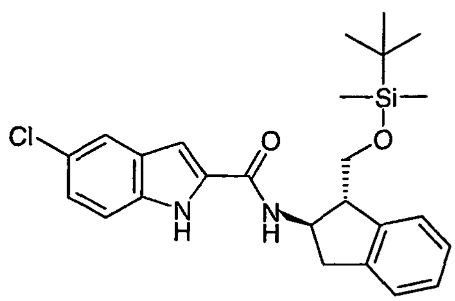

[(1*R*,2*R*)-1-({[*tert*-Butyl(dimethyl)silyl]oxy}methyl)-2,3-dihydro-1*H*-inden-2-yl]amine (**Intermediate 6**; 277.0 mg, 1.0

mmol), 5-chloro-1*H*-indole-2-carboxylic acid (293 mg, 1.5 mmol) and DIPEA (260 μl, 1.5 mmol) were dissolved in DCM (10 ml). HOBT (200 mg, 1.5 mmol) and EDCI (360 mg, 1.87 mmol) were added and the mixture stirred at ambient temperature for 2 h. The volatiles were removed under reduced pressure and the residue dissolved in ethyl acetate (30 ml), washed with water (10 ml), 1N citric acid (10 ml), water (10 ml), saturated aqueous sodium bicarbonate (10 ml), water (10 ml) and brine (10 ml), dried ($MgSO_4$) and the solvent removed under reduced pressure. The crude residue was purified by flash silica gel chromatography (9:1, iso-hexane:ethyl acetate) to give the title compound (682 mg, 89%) as a pale orange foam.
$^1$H NMR($CDCl_3$, 300MHz) δ: 0.02 (d, 6H), 0.85 (s, 9H), 2.95 (dd, 1H), 3.35 (m, 1H), 3.6 (dd, 1H), 3.85 (m, 1H), 4.05 (dd, 1H), 4.73 (m, 1H), 6.55 (d, 1H), 6.75 (s, 1H), 7.25 (m, 5H), 7.4 (d, 1H), 7.6 (s, 1H), 9.6 (s, 1H).

**Intermediate 12: 1-[(1*R*,2*R*)-2-amino-2,3-dihydro-1*H*-inden-1-yl]-*N*-(2-hydroxyethyl)methanesulfonamide hydrochloride**

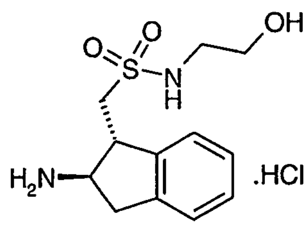

*tert*-Butyl [(1*R*,2*R*)-1-({[(2-hydroxyethyl)amino]sulfonyl}methyl)-2,3-dihydro-1*H*-inden-2-yl]carbamate (**Intermediate 13**, 1.25 g, 4.1 mmol) was dissolved in DCM (20 mL) next 4M HCl in dioxane (20 mL) was added and the solution stirred for 1 hr. The slurry was then stripped to dryness before slurrying in $Et_2O$ (40 mL) and filtering to afford the title compound (314mg, 89%) as a white solid.
$^1$H NMR δ: 2.94 (d, 1H), 3.11 (m, 2H), 3.47 (m, 5H), 3.75 (m, 1H), 4.07 (m, 1H), 4.88 (m, 1H), 7.26 (m, 3H), 7.45 (m, 2H), 8.30 (bs, 3H); MS m/z 271.3.

**Intermediate 13: *tert*-Butyl[(1*R*,2*R*)-1-({[(2-hydroxyethyl)amino]sulfonyl}methyl)-2,3-dihydro-1*H*-inden-2-yl] carbamate**

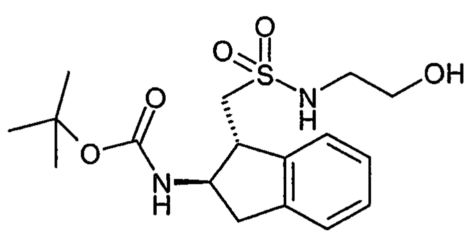

*tert*-Butyl [(1*R*,2*R*)-1-({[(2-hydroxyethyl)amino]sulfinyl}methyl)-2,3-dihydro-1*H*-inden-2-yl]carbamate (**Intermediate 14**, 729 mg, 2.1mmol) was dissolved in DCM (30 mL) to this was added *m*-CPBA (558 mg, 2.3 mmol) and the solution stirred for 16 h. The solution was washed with saturated $NaHCO_3$ (4 x15 mL) and evaporated to give a white solid. The crude material was purified by flash column chromatography (silica gel eluting with 0-100% EtOAc/isohexane) to give the title compound (436 mg, 57%) as a white solid.
$^1$H NMR δ: 1.40 (s, 9H), 2.78 (m, 1H), 3.05 (m, 3H), 3.35 (m, 5H), 4.05 (m, 1H), 4.72 (t, 1H), 7.16 (m, 5H), 7.48 (m, 1H); MS m/z 369.1.

**Intermediate 14: *tert*-butyl [(1*R*,2*R*)-1-({[(2-hydroxyethyl)amino]sulfinyl}methyl)-2,3-dihydro-1*H*-inden-2-yl]carbamate**

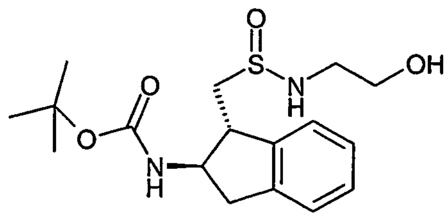

*S*-({(1*R*,2*R*)-2-[(*tert*-Butoxycarbonyl)amino]-2,3-dihydro-1*H*-inden-1-yl}methyl) ethanethioate (**Intermediate 15**, 1.19g, 3.7 mmol) was dissolved in DCM (20 mL) and cooled to -10 ˚C before adding acetic anhydride (0.35 mL, 3.7 mmol) then thionyl chloride ($SO_2Cl_2$) (0.60 mL, 7.4 mmol) the solution was then stirred for 1 hour. After evaporation the solid was redissolved in DCM (20 mL) before adding ethanolamine (0.45 mL, 7.4 mmol). The reaction was stirred for 16 h before the supernatant was purified by flash column chromatography (silica gel eluting with 0-10% MeOH/ DCM) to give the title compound (739 mg, 56%) as a white solid.
$^1$H NMR δ: 1.40 (s, 9H), 2.75 (m, 1H), 3.05 (m, 5H), 3.48 (m, 2H), 4.06 (m, 1H), 4.64 (m, 1H), 6.07 (m, 1H), 7.22 (m, 6H); MS m/z 355.1.

**Intermediate 15: *S*-({(1*R*,2*R*)-2-[(*tert*-Butoxycarbonyl)amino]-2,3-dihydro-1*H*-inden-1-yl}methyl)ethanethioate**

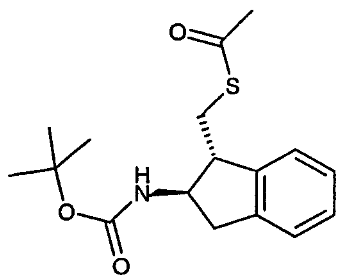

{(1*R*,2*R*)-2-[(*tert*-Butoxycarbonyl)amino]-2,3-dihydro-1*H*-inden-1-yl}methyl methanesulfonate (**Intermediate 16**; 1.314 g, 3.85 mmol) in DMF (10 mL) was added to a solution of thioacetic acid (351 mg, 4.62 mmol) and caesium carbonate (813 mg, 2.5 mmol) in DMF (30 mL). The solution was stirred at ambient temperature for 16 h then filtered. Water (20 mL) and EtOAc (100 mL) were added, the organic layer was separated, washed with water (20 mL) and brine (20 mL), dried ($MgSO_4$) filtered and evaporated. The residue was purified by column chromatography (eluent: EtOAc:toluene, 1:19) to afford the title compound (919 mg, 75%) as an oil which crystallised on standing.
$^1$H NMR ($CDCl_3$) δ: 1.4 (s, 9H), 2.4 (s, 3H), 2.8 (dd, 1H), 3.2 (m, 3H), 3.3 (dd, 1H), 4.1 (m, 1H), 4.8 (m, 1H), 7.2 (m, 3H), 7.3 (m, 1H).

**Intermediate 16: {(1*R*,2*R*)-2-[(*tert*-butoxycarbonyl)amino]cyclopentyl}methyl methanesulfonate**

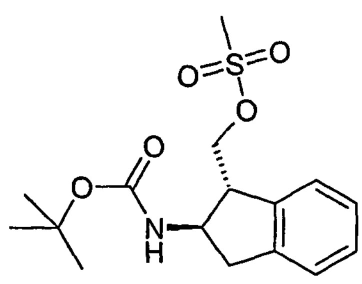

To *tert*-butyl [(1*R*,2*R*)-1-(hydroxymethyl)-2,3-dihydro-1*H*-inden-2-yl]carbamate (**Intermediate 4**; 4.85 g, 18 mmol) dissolved in DCM (100 mL) at 0 ˚C was added triethylamine (3.08 mL, 22 mmol) and methanesulfonyl chloride (1.58 mL, 20 mmol) and the mixture was stirred for 2 h. The mixture was washed with water (2 x 50 mL), dried ($MgSO_4$) and evaporated under reduced pressure to give the title compound (6.64g) as a white solid.
$^1H$ NMR δ: 1.40 (s, 9H), 2.76 (m, 1H), 3.16 (m, 4H), 3.37 (m, 1H), 4.11 (m, 1H), 4.46 (m, 2H), 7.24 (m, 5H); MS m/z 342.1.

**Intermediate 17: 2-({[(1*R*,2*R*)-2-amino-2,3-dihydro-1*H*-inden-1-yl]methyl}sulfonyl)ethanol hydrochloride**

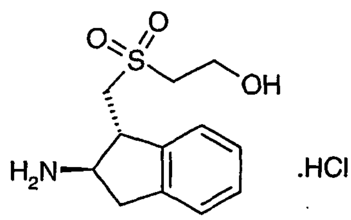

*tert*-Butyl ((1*R*,2*R*)-1-{[(2-hydroxyethyl)sulfonyl]methyl}-2,3-dihydro-1*H*-inden-2-yl)carbamate (**Intermediate 19**, 1.78 g, 5 mmol) was dissolved in DCM (20 mL) and treated with HCl (4M in dioxan, 50 mL) and stirred at ambient temperature for 2 h. Volatiles were then removed by evaporation under reduced pressure. The resulting white solid was stirred with ether (50 mL) and recovered by filtration to give the title compound (1.38g, 94%) as a white solid.
$^1H$ NMR δ: 2.95 (dd, 1H), 3.5 (m, 5H), 3.85 (m, 3H), 4.05 (m, 1H), 7.3 (m, 3H), 7.4 (m, 1H), 8.4 (bs, 3H).
The following intermediate was prepared by the method of **Intermediate 17**, using *tert*-butyl ((1*R*,2*R*)-1{[(3-hydroxypropyl)sulfonyl]methyl}-2,3-dihydro-1*H*-inden-2-yl)carbamate (**Intermediate 20**) as the carbamate.

**Intermediate 18: 3-({[(1*R*,2*R*)-2-amino-2,3-dihydro-1*H*-inden-1-yl]methyl}sulfonyl)propan-1-ol hydrochloride**

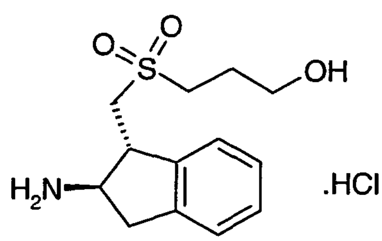

$^1H$ NMR δ: 1.88 (m, 2H), 2.97 (dd, 1H), 3.40 (m, 7H), 3.76 (m, 2H), 3.99 (m, 1H), 7.26 (m, 3H), 7.48 (m, 1H), 8.44 (bs, 3H); MS m/z 270.1

**Intermediate 19: *tert*-Butyl((1*R*,2*R*)-1-{[(2-hydroxyethyl)sulfonyl]methyl}-2,3-dihydro-1*H*-inden-2-yl)carbamate**

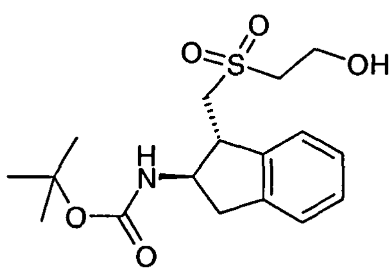

*tert*-Butyl ((1*R*,2*R*)-1-{[(2-hydroxyethyl)thio]methyl}-2,3-dihydro-1*H*-inden-2-yl)carbamate (**Intermediate 24**, 1.9 g, 5.88

mmol) was dissolved in DCM (100 mL) and cooled to 5˚C. 70% *m*-CPBA (3.18g, 12.94 mmol) was added in portions keeping the temperature below 7 ˚C and after the addition was complete the mixture was stirred at 5 ˚C for 1h and then a further 2h at ambient temperature. After washing with saturated sodium bicarbonate (3x50 mL) and water (50 mL) the reaction mixture was dried ($MgSO_4$) and volatiles removed by evaporation under reduced pressure to leave a clear oil. This was further purified by chromatography on silica gel eluting with an EtOAc hexane gradient (0-100%) to give the title compound as a white solid (1.78g, 85%).

$^1$H NMR δ: 1.4 (s, 9H), 2.8 (dd, 1H), 3.1 (dd, 1H), 3.3 (m, 2H), 3.5 (m, 3H), 3.8 (m, 2H), 4.05 (m, 1H), 5.1 (b, 1H), 7.2 (m, 4H), 7.45 (m, 1H); MS m/z 355

The following intermediate was prepared by the method of **Intermediate 19** using *tert*-butyl ((1*R*,2*R*)-1-{([(3-hydroxypropyl)thio]methyl}-2,3-dihydro-1*H*-inden-2-yl)carbamate (**Intermediate 25**) as the thioether.

**Intermediate 20: *tert*-Butyl ((1*R*,2*R*)-1-{[(3-hydroxypropyl)sulfonyl]methyl}-2,3-dihydro-1*H*-inden-2-yl)carbamate**

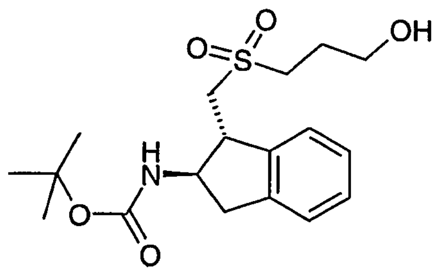

$^1$H NMR δ: 1.38 (s, 9H), 1.85 (m, 2H), 2.80 (m, 1H), 3.14 (m, 3H), 3.46 (m, 5H), 4.07 (m, 1H), 4.67 (t, 1H), 7.20 (m, 4H), 7.44 (m, 1H); MS m/z 392.1

**Intermediate 21: Methyl({(1*R*,2*R*)-2-[(*tert*-butoxycarbonyl)amino]-2,3-dihydro-1*H*-inden-1-yl}thio)acetate**

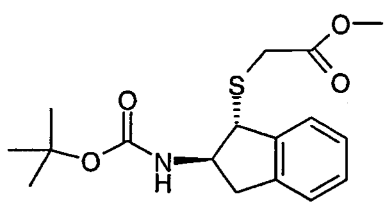

Sodium hexamethyldisilazide (NaHMDS) (10 mL, 1.0 M in THF, 10.00 mmol) was added slowly to a solution of (1*S*,2*S*)-1-[(*tert*-butoxycarbonyl)amino]-2,3-dihydro-1*H*-inden-2-yl methanesulfonate (**Intermediate 22**, 3.00g, 9.17 mmol) in THF (40 mL) ensuring the internal temperature was maintained at <20˚C. After 30 mins methyl thioglycolate (0.90 mL, 10.00 mmol), NaHMDS (1.0 M in THF, 5 mL, 5.00 mmol) and DMF (10 mL) were added and the reaction was stirred at ambient temperature for 3 h. The reaction was quenched by the addition of water (100 mL) and ether (100 mL) and the organic layer was dried ($MgSO_4$), filtered and the volatiles removed *in vacuo*. Purification by column chromatography (eluent gradient: 1:4 to 1:2 EtOAc:hexanes) afforded the title compound (1.73g, 55%) as a brown oil.

$^1$H NMR δ: 1.43 (s, 9H), 2.75 (dd, 1H), 3.40 (d, 1H), 3.55 (m, 2H), 3.73 (s, 3H), 4.36 (m, 2H), 4.84 (s, 1H), 7.22 (m, 3H), 7.39 (m, 1H); MS m/z 360 $[M + Na]^+$.

**Intermediate 22: (1*S*,2*S*)-1-[(*tert*-Butoxycarbonyl)amino]-2,3-dihydro-1*H*-inden-2-yl methanesulfonate**

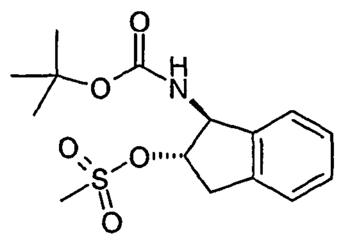

Mesyl chloride (2.24 mL, 30.03 mmol) was added to a cooled (0 ˚C) solution of *tert*-butyl [(1*S*,2*S*)-2-hydroxy-2,3-dihydro-1*H*-inden-1-yl]carbamate (**Intermediate 23**, 6.80 g, 27.3 mmol) and triethylamine (4.01 mL, 30.03 mmol) in DCM (100 mL) ad stirred at 0 ˚C for 1 h. The reaction was quenched by addition of saturated aqueous $NaHCO_3$ (100 mL), the organic layer was dried ($MgSO_4$), filtered and the volatiles removed *in vacuo*. The crude product was triturated with hot ether (40 mL), cooled and filtered to afford the title compound (8.11 g, 91 %) as a white solid.
$^1H$ NMR δ: 1.45 (s, 9H), 3.18 (m, 4H), 3.47 (dd, 1H), 4.78 (s, 1H) 5.19 (m, 2H), 7.28 (m, 4 H); MS m/z 350 $[M + Na]^+$.

**Intermediate 23: *tert*-Butyl [(1*S*,2*S*)-2-hydroxy-2,3-dihydro-1*H*-inden-1-yl]carbamate**

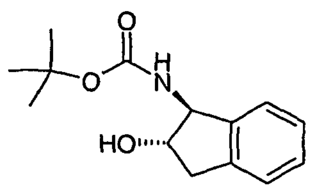

THF (100 mL) followed by 1 M aqueous sodium hydroxide was added to (1*S*,2*S*)(+)-*trans*-1-amino-2-indanol (CAS Reg. No.163061-74-3; 5.00 g, 33.55 mmol). Di-tert-butyl dicarbonate (7.30 g, 33.55 mmol) was then added and stirred for 16 h. The THF was removed *in vacuo* and the remaining aqueous layer was acidified to pH 2 with aqueous citric acid (5% w/v) and diluted with EtOAc (150 mL). The organic layer was dried ($MgSO_4$), filtered and the volatiles removed *in vacuo*. The crude was triturated with hot ether:hexanes (1:1, 40 mL), the suspention cooled and filtered to afford the title compound (6.80 g, 81%) as a white solid.
$^1H$ NMR δ: 1.54 (s, 9H), 2.92 (dd, 1H), 3.28 (dd, 1H), 4.23 (s, 1H), 4.42 (m, 1H), 4.93 (t, 1H), 5.03 (s, 1H), 7.22 (m, 4 H); MS m/z 313 $[M+Na+MeCN]^+$.

**Intermediate 24: *tert*-Butyl((1*R*,2*R*)-1-{[(2-hydroxyethyl)thio]methyl}-2,3-dihydro-1*H*-inden-2-yl)carbamate**

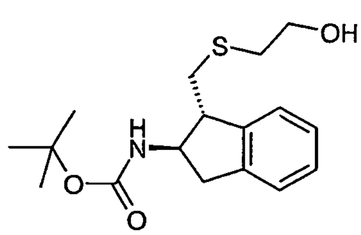

2-Mercaptoethanol (3.95 g, 50.59 mmol) was added to a solution of DBU (3.2 g, 21.1 mmol) in DMF (50 mL) and the mixture heated to 60˚C. A solution of {(1*R*,2*R*)-2-[(*tert*-butoxycarbonyl)amino]-2,3-dihydro-1*H*-inden-1-yl}methyl methanesulfonate (**Intermediate 16**; 2.88 g, 8.43 mmol) in DMF (20 mL) was added and heating at 60˚C continued for a further 2h. The reaction mixture was then cooled to ambient temperature, diluted with EtOAc (200 mL), washed with water (4x50 mL) and brine (50 mL), dried (MgSO4) and evaporated under reduced pressure to give an oil. This was purified by chromatography on silica gel eluting with an EtOAc / hexane gradient (0-50%) to give the title compound (1.9g, 70%) as a clear oil.

$^1H$ NMR δ: 1.4 (s, 9H), 2.6 (t, 2H), 2.7 (m, 2H), 3.15 (m, 2H), 3.55 (m, 2H), 4.05 (m, 1H), 4.7 (t, 1H), 7.1 (m, 4H), 7.45 (m, 1H).. The following intermediate was prepared by the method of **Intermediate 24**, using {(1*R*,2*R*)-2-[(*tert*-butoxycarbonyl) amino]-2,3-dihydro-1*H*-inden-1-yl}methyl methanesulfonate (**Intermediate 16**; as the mesylate and 1-mercapto-3-hydroxypropane.

**Intermediate 25: *tert*-Butyl ((1*R*,2*R*)-1-{[(3-hydroxypropyl)thio]methyl}-2,3-dihydro-1*H*-inden-2-yl)carbamate**

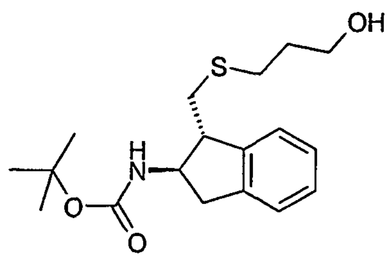

$^1H$ NMR δ: 1.39 (s, 9H), 1.65 (m, 2H), 2.54 (m, 2H), 2.72 (m, 2H), 2.92 (m, 1H), 3.17 (m, 2H), 3.44 (m, 2H), 4.02 (m, 1H), 4.42 (t, 1H), 7.12 (m, 4H), 7.32 (m, 1H); MS m/z 360.1

## Claims

**1.** A compound of formula (1):

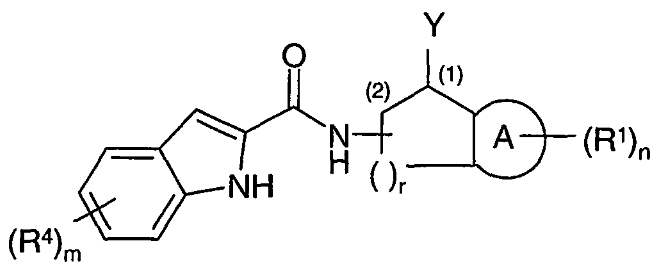

**(1)**

wherein:

A is phenylene or heteroarylene;
n is 0, 1 or 2;
m is 0, 1 or 2;
$R^1$ is independently selected from halo, nitro, cyano, hydroxy, carboxy, carbamoyl, *N*-(1-4C)alkylcarbamoyl, *N,N*-((1-4C)alkyl)$_2$carbamoyl, sulphamoyl, *N*-(1-4C)alkylsulphamoyl, *N*,*N*-((1-4C)alkyl)$_2$sulphamoyl, -S(O)$_b$(1-4C) alkyl (wherein b is 0,1,or 2), -OS(O)$_2$(1-4C)alkyl, (1-4C)alkyl, (2-4C)alkenyl, (2-4C)alkynyl, (1-4C)alkoxy, (1-4C) alkanoyl, (1-4C)alkanoyloxy, hydroxy(1-4C)alkyl, fluoromethyl, difluoromethyl, trifluoromethyl, trifluoromethoxy and -NHSO$_2$(1-4C)alkyl;
or, when n is 2, the two $R^1$ groups, together with the carbon atoms of A to which they are attached, may form a 4 to 7 membered saturated ring, optionally containing 1 or 2 heteroatoms independently selected from O, S and N, and optionally being substituted by one or two methyl groups;
$R^4$ is independently selected from halo, nitro, cyano, hydroxy, fluoromethyl, difluoromethyl, trifluoromethyl, trifluoromethoxy, carboxy, carbamoyl, (1-4C)alkyl, (2-4C)alkenyl, (2-4C)alkynyl, (1-4C)alkoxy and (1-4C)alkanoyl;
r is 1 or 2; and
when r is 1 the group

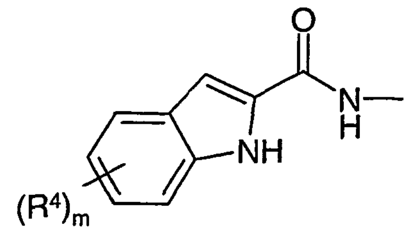

is a substituent on carbon (2) and
when r is 2 (thereby forming a six membered ring) the same group is a substituent on carbon (2) or on carbon (3);
Y is selected from $-C(O)R^2$, $-C(O)OR^2$, $-C(O)NR^2R^3$, -(1-4C)alkyl [optionally substituted by 1 or 2 substituents independently selected from hydroxy, $-C=NR^2$, (1-4C)alkoxy, aryloxy, heterocyclyloxy, $-S(O)_bR^2$ (wherein b is 0, 1 or 2), $-O-S(O)_bR^2$ (wherein b is 0, 1 or 2), $-NR^2R^3$, $-N(OH)R^2$, $-NR^2C(=O)R^2$, $-NHOHC(=O)R^2$, $-SO_2NR^2R^3$, $-N(R^2)SO_2R^2$, aryl and heterocyclyl], -C(O)NOH, -C(O)NSH, -C(N)OH, -C(N)SH, $-SO_2H$, $-SO_3H$, $-SO_2N(OH)R^2$, -(2-4C)alkenyl, $-SO_2NR^2R^3$, -(1-4C)alkylC(O)$R^2$, -(1-4C)alkylC(O)O$R^2$, -(1-4C)alkylSC(O)$R^2$, -(1-4C)alkylOC(O)$R^2$, -(1-4C)alkylC(O)N$R^2R^3$, -(1-4C)alkylOC(O)O$R^2$, -(1-4C)alkylN($R^2$)C(O)O$R^2$, -(1-4C)alkylN($R^2$)C(O)N$R^2R^3$, -(1-4C)alkylOC(O)N$R^2R^3$, (3-6C)cycloalkyl (optionally substituted by 1 or 2 $R^8$), aryl, heterocyclyl (wherein the heterocyclic ring is linked by a ring carbon atom), -(1-4C)alkyl$SO_2$(2-4C)alkenyl and $-S(O)_cR^2$ (wherein c is 0, 1 or 2);
$R^2$ and $R^3$ are independently selected from hydrogen, -O(1-4C)alkyl, -S(1-4C)alkyl, -N(1-4C)alkyl, heterocyclyl, aryl and (1-4C)alkyl [optionally substituted by 1 or 2 $R^8$ groups]; or
wherein $NR^2R^3$ may form a 4 to 7 membered saturated, partially saturated or unsaturated ring, optionally containing 1, 2 or 3 additional heteroatoms independently selected from N, O and S (provided there are no O-O, O-S or S-S bonds), wherein any $-CH_2-$ may optionally be replaced by -C(=O)-, and any N or S atom may optionally be oxidised to form an N-oxide or SO or $SO_2$ group respectively, and wherein the ring is optionally substituted by 1 or 2 substituents independently selected from halo, cyano, (1-4C)alkyl, hydroxy, (1-4C)alkoxy and (1-4C)alkyl$S(O)_b$- (wherein b is 0, 1 or 2);
$R^8$ is independently selected from hydrogen, hydroxy, (1-4C)alkyl, (2-4C)alkenyl, (1-4C)alkoxy, cyano(1-4C)alkyl, amino(1-4C)alkyl [optionally substituted on nitrogen by 1 or 2 groups selected from (1-4C)alkyl, hydroxy, hydroxy(1-4C)alkyl, dihydroxy(1-4C)alkyl, $-CO_2$(1-4C)alkyl, aryl and aryl(1-4C)alkyl], halo(1-4C)alkyl, dihalo(1-4C)alkyl, trihalo(1-4C)alkyl, hydroxy(1-4C)alkyl, dihydroxy(1-4C)alkyl, (1-4C)alkoxy(1-4C)alkoxy, (1-4C)alkoxy(1-4C)alkyl, hydroxy(1-4C)alkoxy, 5- and 6-membered cyclic acetals and mono- and di-methyl derivatives thereof, aryl, heterocyclyl, heterocyclyl(1-4C)alkyl, (3-7C)cycloalkyl (optionally substituted with 1 or 2 hydroxy groups, (1-4C)alkyl or $-CO_2$(1-4C)alkyl), (1-4C)alkanoyl, (1-4C)alkyl$S(O)_b$, (wherein b is 0,1 or 2), (3-6C)cycloalkyl$S(O)_b$- (wherein b is 0, 1 or 2), aryl$S(O)_b$- (wherein b is 0, 1 or 2), heterocyclyl$S(O)_b$- (wherein b is 0, 1 or 2), benzyl$S(O)_b$- (wherein b is 0, 1 or 2), (1-4C)alkyl$S(O)_c$(1-4C)alkyl- (wherein c is 0, 1 or 2), -N(OH)CHO, $-C(=N-OH)NH_2$, -C(=N-OH)NH(1-4C)alkyl, -C(=N-OH)N((1-4C)alkyl$)_2$, -C(=N-OH)NH(3-6C)cycloalkyl, -C(=N-OH)N((3-6C)cycloalkyl$)_2$, $-COCOOR^9$, $-C(O)N(R^9(R^{10})$, $-NHC(O)R^9$, $-C(O)NHSO_2$(1-4C)alkyl, $-NHSO_2R^9$, $(R^9)(R^{10})NSO_2$-, $-COCH_2OR^{11}$, $-COCH_2OH$, $(R^9)(R^{10})N$-, $-COOR^9$, $-CH_2OR^9$, $-CH_2COOR^9$, $-CH_2OCOR^9$, $-CH_2CH(CO_2R^9)OH$, $-CH_2C(O)NR^9R^{10}$, $-(CH2)_wCH(NR^9R^{10})CO_2R^{9'}$ (wherein w is 1, 2 or 3), and $-(CH_2)_wCH(NR^9R^{10})CO(NR^{9'}R^{10'})$ (wherein w is 1, 2 or 3) ;
$R^9$, $R^{9'}$, $R^{10}$ and $R^{10'}$ are independently selected from hydrogen, hydroxy, (1-4C)alkyl (optionally substituted by 1 or 2 $R^{11}$), (2-4C)alkenyl, (3-7C)cycloalkyl (optionally substituted by 1 or 2 hydroxy groups), cyano(1-4C)alkyl, trihalo(1-4C)alkyl, aryl, heterocyclyl, heterocyclyl(1-4Calkyl), $-CO_2$(1-4C)alkyl; or
$R^9$ and $R^{10}$ together with the nitrogen to which they are attached, and/or $R^{9'}$ and $R^{10'}$ together with the nitrogen to which they are attached, form a 4- to 6-membered ring where the ring is optionally substituted on carbon by 1 or 2 substituents independently selected from oxo, hydroxy, carboxy, halo, nitro, cyano, carbonyl, (1-4C)alkoxy and heterocyclyl; or the ring may be optionally substituted on two adjacent carbons by $-O-CH_2-O-$ to form a cyclic acetal wherein one or both of the hydrogens of the $-O-CH_2-O-$group may be replaced by a methyl;
$R^{11}$ is independently selected from (1-4C)alkyl, and hydroxy(1-4C)alkyl;

or a pharmaceutically acceptable salt thereof.

**2.** A compound of the formula (1), or a pharmaceutically acceptable salt thereof, as claimed in claim 1, wherein A is phenylene.

**3.** A compound of the formula (1), or a pharmaceutically acceptable salt or in-vivo hydrolysable ester thereof, as claimed in claim 1 or claim 2, wherein n is 0.

**4.** A compound of the formula (1), or a pharmaceutically acceptable salt or in-vivo hydrolysable ester thereof, as claimed in any one of the preceding claims wherein r is 1.

**5.** A compound of the formula (1), or a pharmaceutically acceptable salt or in-vivo hydrolysable ester thereof, as claimed in any one of the preceding claims wherein m is 1.

**6.** A compound of the formula (1), or a pharmaceutically acceptable salt or in-vivo hydrolysable ester thereof, as claimed in any one of the preceding claims wherein Y is selected from $-C(O)OR^2$, $-C(O)NR^2R^3$, -(1-4C)alkyl [optionally substituted by a substituent selected from hydroxy, (1-4C)alkoxy, $-S(O)_bR^2$ (wherein b is 0, 1 or 2), $-O-S(O)_bR^2$ (wherein b is 0, 1 or 2), $-NR^2R^3$, $-NR^2C(=O)R^2$ and $-SO_2NR^2R^3$], -(1-4C)alkylC(O)$R^2$, -(1-4C)alkylC(O)O$R^2$, -(1-4C)alkylOC(O)$R^2$, -(1-4C)alkylC(O)N$R^2R^3$, -(1-4C)alkylOC(O)O$R^2$, -(1-4C)alkylN($R^2$)C(O)O$R^2$, -(1-4C)alkylN($R^2$)C(O)N$R^2R^3$, -(1-4C)alkylSC(O)$R^2$, -(1-4C)alkylOC(O)N$R^2R^3$, -(1-4C)alkyl$SO_2$(2-4C)alkenyl and $-SO_cR^2$ (wherein c is 0, 1 or 2).

**7.** A compound of the formula (1), or a pharmaceutically acceptable salt or in-vivo hydrolysable ester thereof, as claimed in any one of the preceding claims wherein $R^2$ and $R^3$ are independently selected from hydrogen, heterocyclyl, -O(1-4C)alkyl, -N(1-4C)alkyl, (1-4C)alkyl [optionally substituted by 1 or 2 $R^8$ groups]; or an $NR^2R^3$ group forms a morpholine, thiomorpholine (and oxidised versions thereof), pyrrolidine, or piperidine ring and wherein the ring is optionally substituted by 1 or 2 substituents independently selected from chloro, fluoro, hydroxy and methoxy.

**8.** A compound of the formula (1), or a pharmaceutically acceptable salt or in-vivo hydrolysable ester thereof, as claimed in any one of the preceding claims wherein $R^8$ is independently selected from hydrogen, hydroxy, $-C(O)N(R^9)(R^{10})$, $-NHC(O)R^9$, $-COOR^9$, $-CH_2OR^9$, $-CH_2COOR^9$, $-CH_2OCOR^9$, aryl, heterocyclyl, and 5- and 6-membered cyclic acetals and mono- and di-methyl derivatives thereof.

**9.** A compound of the formula (1), or a pharmaceutically acceptable salt or in-vivo hydrolysable ester thereof, as claimed in any one of the preceding claims wherein $R^9$ and $R^{10}$ are independently selected from hydrogen, hydroxy and (1-4C)alkyl) or $R^9$ and $R^{10}$ together with the nitrogen to which they are attached form a morpholine, thiomorpholine (and oxidised versions thereof), pyrrolidine, or piperidine ring.

**10.** A pharmaceutical composition which comprises a compound of the formula (1), or a pharmaceutically acceptable salt or in-vivo hydrolysable ester thereof, as claimed in claim 1 in association with a pharmaceutically-acceptable diluent or carrier.

**11.** A compound of the formula (1), or a pharmaceutically acceptable salt or in-vivo hydrolysable ester thereof, as claimed in claim 1, for use in a method of treatment of a warm-blooded animal such as man by therapy.

**12.** A compound of the formula (1), or a pharmaceutically acceptable salt or in-vivo hydrolysable ester thereof, as claimed in claim 1, for use as a medicament.

**13.** A compound of the formula (1), or a pharmaceutically acceptable salt or in-vivo hydrolysable ester thereof, as claimed in claim 1, for use as a medicament in the treatment of type 2 diabetes, insulin resistance, syndrome X, hyperinsulinaemia, hyperglucagonaemia, cardiac ischaemia or obesity in a warm-blooded animal such as man.

**14.** The use of a compound of the formula (1), or a pharmaceutically acceptable salt or in-vivo hydrolysable ester thereof, as claimed in claim 1, in the manufacture of a medicament for use in the treatment of type 2 diabetes, insulin resistance, syndrome X, hyperinsulinaemia, hyperglucagonaemia, cardiac ischaemia or obesity in a warm-blooded animal such as man.

**15.** The use of a compound of the formula (1), or a pharmaceutically acceptable salt or in-vivo hydrolysable ester thereof, as claimed in claim 1, in the manufacture of a medicament for use in the treatment of type 2 diabetes in a warm-blooded animal such as man.

**16.** A process for the preparation of a compound of formula (1) as claimed in claim 1, which process comprises:

reacting an acid of the formula (2):

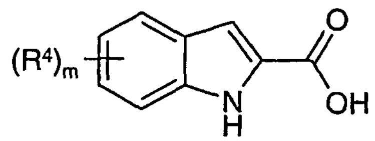

(2)

or an activated derivative thereof; with an amine of formula (3):

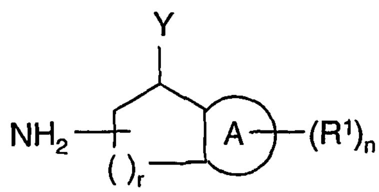

(3)

and thereafter if necessary:

i) converting a compound of the formula (1) into another compound of the formula (1);
ii) removing any protecting groups;
iii) forming a pharmaceutically acceptable salt or *in vivo* hydrolysable ester.

**Patentansprüche**

**1.** Verbindungen der Formel (1):

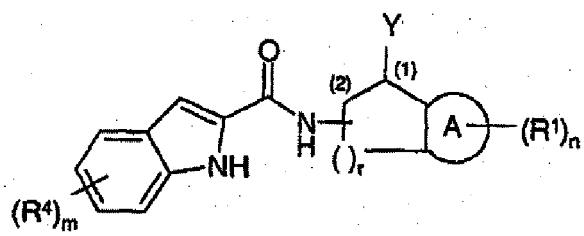

(1)

wobei:

A für Phenylen oder Heteroarylen steht;
n für 0, 1 oder 2 steht;
m für 0, 1 oder 2 steht;
$R^1$ unabhängig ausgewählt ist aus Halogen, Nitro, Cyano, Hydroxy, Carboxyl, Carbamoyl, *N*-(1-4C)-Alkylcarbamoyl, *N,N*-((1-4C)-Alkyl)$_2$carbamoyl, Sulfamoyl, *N*-(1-4C)-Alkylsulphamoyl, *N,N*-((1-4C)-Alkyl)$_2$sulfamoyl, -S(O)$_b$-(1-4C)-Alkyl (wobei b für 0, 1 oder 2 steht), -OS(O)$_2$(1-4C)-Alkyl, (1-4C)Alkyl, (2-4C)-Alkenyl, (2-4C)-Alkinyl, (1-4C)-Alkoxy, (1-4C)-Alkanoyl, (1-4C)-Alkanoyloxy, Hydroxy-(1-4C)-Alkyl, Fluormethyl, Difluormethyl, Trifluormethyl, Trifluormethoxy und -NHSO$_2$-(1-4C)-Alkyl; oder, wenn n für 2 steht, die beiden $R^1$-Gruppen zusammen mit den Kohlenstoffatomen von A, an die sie gebunden sind, einen 4- bis 7-gliedrigen gesättigten Ring bilden können, der gegebenenfalls 1 oder 2 Heteroatome unabhängig voneinander ausgewählt aus 0, S und N enthält

und gegebenenfalls durch eine oder zwei Methylgruppen substituiert ist;
$R^4$ unabhängig ausgewählt ist aus Halogen, Nitro, Cyano, Hydroxy, Fluormethyl, Difluormethyl, Trifluormethyl, Trifluormethoxy, Carboxyl, Carbamoyl, (1-4C)-Alkyl, (2-4C)-Alkenyl, (2-4C)-Alkinyl, (1-4C)-Alkoxy und (1-4C)-Alkanoyl;
r für 1 oder 2 steht; und,
wenn r für 1 steht, die Gruppe

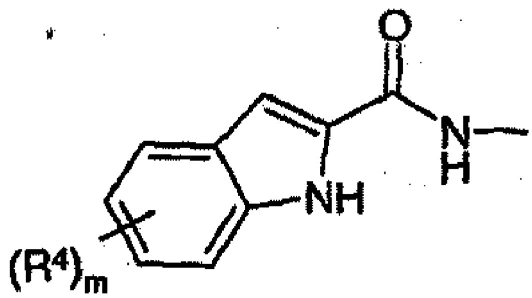

für einen Substituenten an Kohlenstoff(2) steht und,
wenn r für 2 steht (wodurch ein sechsgliedriger Ring gebildet wird), die gleiche Gruppe für einen Substituenten an Kohlenstoff(2) oder an Kohlenstoff(3) steht;
Y ausgewählt ist aus $-C(O)R^2$, $-C(O)OR^2$, $-C(O)NR^2R^3$, -(1-4C)-Alkyl [gegebenenfalls substituiert durch 1 oder 2 Substituenten unabhängig voneinander ausgewählt aus Hydroxy, $-C=NR^2$, (1-4C) -Alkoxy, Aryloxy, Heterocyclyloxy, $-S((O)_bR^2$ (wobei b für 0, 1 oder 2 steht), $-O-S(O)_bR^2$ (wobei b für 0, 1 oder 2 steht), $-NR^2R^3$, -N(OH) $R^2$, $-NR^2C(=O)R^2$, $-NHOHC(=O)R^2$, $-SO^2N\ R^2R^3$, $-N(R^2)\ SO_2R^2$, Aryl und Heterocyclyl], -C(O)NOH, -C(O) NSH, -C(N)OH, -C(N)SH, $-S\ O_2H$, $-SO_3H$, $-SO_2N\ (OH)\ R^2$, -(2-4C) -Alkenyl, $-SO_2NR^2R^3$, -(1-4C) -Alkyl-C (O) $R^2$, -1(-4C) -Alkyl-C (O) $OR^2$, -(1-4C) $-Alkyl-SC(O)R^2$, -(1-4C)-Alkyl-$OC(O)R^2$, -(1-4C)-Alkyl-$C(O)NR^2R^3$, -(1-4C)-Alkyl-$OC(O)OR^2$, -(1-4C)-Alkyl-$N(R^2)C(O)OR^2$, -(1-4C)-Alkyl-$N(R^2)C(O)NR^2R^3$, -(1-4C)-Alkyl-OC(O) $NR^2R^3$, (3-6C)-Cycloalkyl (gegebenenfalls substituiert durch 1 oder 2 $R^8$), Aryl, Heterocyclyl (wobei der heterocyclische Ring über ein Ringkohlenstoffatom gebunden ist), -(1-4C)-Alkyl-$SO_2$-(2-4C)-alkenyl und $-S(O),R^2$ (wobei c für 0,1 oder 2 steht);
$R^2$ und $R^3$ unabhängig voneinander ausgewählt sind aus Wasserstoff, -O-(1-4C)-Alkyl, -S-(1-4C)-Alkyl, -N-(1-4C)-Alkyl, Heterocyclyl, Aryl und (1-4C)-Alkyl [gegebenenfalls substituiert durch 1 oder 2 $R^8$-Gruppen]; oder
wobei $NR^2R^3$ einen 4- bis 7-gliedrigen gesättigten, teilweise gesättigten oder ungesättigten Ring bilden kann, der gegebenenfalls 1, 2 oder 3 zusätzliche Heteroatome unabhängig voneinander ausgewählt aus N, O und S enthält (mit der Maßgabe, daß es keine O-O-, O-S- oder S-S-Bindungen gibt), wobei $-CH_2-$ gegebenenfalls durch -C(=O)- ersetzt sein kann und ein N- oder S-Atom gegebenenfalls unter Bildung eines N-Oxids beziehungsweise einer SO- oder $SO_2$-Gruppe oxidiert sein kann, und wobei der Ring gegebenenfalls substituiert ist durch 1 oder 2 Substituenten unabhängig voneinander ausgewählt aus Halogen, Cyano, (1-4C)-Alkyl, Hydroxy, (1-4C)-Alkoxy und (1-4C)-Alkyl-$(S(O)_b$- (wobei b für 0, 1 oder 2 steht);
$R^8$ unabhängig ausgewählt ist aus Wasserstoff, Hydroxy, (1-4C)-Alkyl, (2-4C)-Alkenyl, (1-4C)-Alkoxy, Cyano-(1-4C)-Alkyl, Amino-(1-4C)-Alkyl [gegebenenfalls am Stickstoff substituiert durch 1 oder 2 Gruppen ausgewählt aus (1-4C)-Alkyl, Hydroxy, Hydroxy-(1-4C)-alkyl, Dihydroxy-(1-4C)-alkyl, $-CO_2$-(1-4C)-Alkyl, Aryl und Aryl-(1-4C)-alkyl], Halogen-(1-4C)-alkyl, Dihalogen-(1-4C)-alkyl, Trihalogen-(1-4C)-alkyl, Hydroxy-(1-4C)-alkyl, Dihydroxy-(1-4C)-alkyl, (1-4C)-Alkoxy-(1-4C)-alkoxy, (1-4C)-Alkoxy-(1-4C)-alkyl, Hydroxy-(1-4C)-alkoxy, 5- und 6-gliedrige cyclische Acetale und Mono- und Dimethylderivate davon, Aryl, Heterocyclyl, Heterocyclyl-(1-4C)-alkyl, (3-7C)-Cycloalkyl (gegebenenfalls substituiert durch 1 oder 2 Hydroxygruppen, (1-4C)-Alkyl oder $-CO_2$-(1-4C)-Alkyl), (1-4C)-Alkanoyl, (1-4C)-Alkyl-$S(O)_b$ (wobei b für 0, 1 oder 2 steht), (3-6C)-Cycloalkyl-$S(O)_b$- (wobei b für 0, 1 oder 2 steht), Aryl-$S(O)_b$- (wobei b für 0, 1 oder 2 steht), Heterocyclyl-$S(O)_b$- (wobei b für 0, 1 oder 2 steht), Benzyl-$S(O)_b$- (wobei b für 0, 1 oder 2 steht), (1-4C)-Alkyl-$S(O)_c$-(1-4C)-alkyl- (wobei c für 0, 1 oder 2 steht), -N(OH))CHO, $-C(=N-OH)NH_2$, -C(=N-OH) NH-(1-4C)-Alkyl, -C(=N-OH)-((1-4C)-$Alkyl)_2$, -C(=N-OH)NH-(3-6C)-Cycloalkyl, -C(=N-OH)N-((3-6C)-$Cycloalkyl)_2$, $-COCOOR^9$, $-C(O)N(R^9)(R^{10})$, -NHC(O) $R^9$, $-C(O)NHSO_2$-(1-4C)-Alkyl, $-NHSO_2R^9$, $(R^9)(R^{10})NSO_2-$, $-COCH_2OR^{11}$, $-COCH_2OH$, $(R^9)\ (R^{10})N-$, $-COOR^9$, $CH_2OR^9$, $-CH_2COOR^9$, $-CH_2OCOR^9$, $-CH_2CH(CO_2R^9)OH$, $-CH_2C(O)NR^9R^{10}$, $-(CH_2)_wCH(NR^9R^{10})CO_2R^9$ (wobei w für 1, 2 oder 3 steht) und $-(CH_2)_wCH(NR^9R^{10})CO(NR^9R^{10})$ (wobei w für 1, 2 oder 3 steht);
$R^9$, $R^{9'}$, $R^{10}$ und $R^{10'}$ unabhängig voneinander ausgewählt sind aus Wasserstoff, Hydroxy, (1-4C)-Alkyl (gegebenenfalls substituiert durch 1 oder 2 $R^{11}$), (2-4C)-Alkenyl, (3-7C)-Cycloalkyl (gegebenenfalls substituiert durch 1 oder 2 Hydroxylgruppen), Cyano-((1-4C))-alkyl, Trihalogen(1-4C)alkyl, Aryl, Heterocyclyl, Heterocyclyl-(1-4C)-alkyl, -$CO_2$-(1-4C)-Alkyl; oder
$R^9$ und $R^{10}$ zusammen mit dem Stickstoff, an den sie gebunden sind, und/oder $R^{9'}$ und $R^{10'}$ zusammen mit dem Stickstoff, an den sie gebunden sind, einen 4- bis 6-gliedrigen Ring bilden, wobei der Ring gegebenenfalls am Kohlenstoff durch 1 oder 2 Substituenten unabhängig voneinander ausgewählt aus Oxo, Hydroxy, Carboxyl, Halogen, Nitro, Cyano, Carbonyl, (1-4C)-Alkoxy und Heterocyclyl substituiert ist; oder der Ring gegebenenfalls an zwei benachbarten Kohlenstoffen durch
-O-$CH_2$-O- substituiert sein kann, unter Bildung eines cyclischen Acetals, wobei einer oder beide Wasserstoffe der -O-$CH_2$-O-Gruppe durch Methyl ersetzt sein können;
$R^{11}$ unabhängig ausgewählt ist aus (1-4C)-Alkyl und Hydroxy-(1-4C)-Alkyl;

und deren pharmazeutisch annehmbare Salze.

2. Verbindungen der Formel (1) und deren pharmazeutisch annehmbare Salze nach Anspruch 1, wobei A für Phenylen steht.

3. Verbindungen der Formel (1) und deren pharmazeutisch annehmbare Salze und in vivo hydrolysierbare Ester nach Anspruch 1 oder 2, wobei n für 0 steht.

4. Verbindungen der Formel (1) und deren pharmazeutisch annehmbare Salze und in vivo hydrolysierbare Ester nach einem der vorhergehenden Ansprüche, wobei r für 1 steht.

5. Verbindungen der Formel (1) und deren pharmazeutisch annehmbare Salze und in vivo hydrolysierbare Ester nach einem der vorhergehenden Ansprüche, wobei m für 1 steht.

6. Verbindungen der Formel (1) und deren pharmazeutisch annehmbare Salze und in vivo hydrolysierbare Ester nach einem der vorhergehenden Ansprüche, wobei Y ausgewählt ist aus -C(O)O$R^2$, -C(O)N$R^2R^3$, -(1-4C)-Alkyl [gegebenenfalls substituiert durch einen Substituenten ausgewählt aus Hydroxy, (1-4C)-Alkoxy,- $S(O)_bR^2$ (wobei b für 0, 1 oder 2 steht), -O-$S(O)_bR^2$ (wobei b für 0, 1 oder 2 steht) -N$R^2R^3$, -N$R^2$C (=O)$R^2$ und -$SO_2$N$R^2R^3$], -(1-4C)-Alkyl-C(O)$R^2$, -(1-4C)-Alkyl-C(O)O$R^2$, -(1-4C)-Alkyl-OC(O)$R^2$, -(1-4C)-Alkyl-C(O)N$R^2R^3$, -(1-4C)-Alkyl-OC(O)O$R^2$, -(1-4C)-Alkyl-N($R^2$)C(O)O$R^2$, -(1-4C)-Alkyl-N($R^2$)C(O)N$R^2R^3$, -(1-4C)-Alkyl-SC(O)$R^2$, -(1-4C)-Alkyl-OC(O)N$R^2R^3$, -(1-4C)-Alkyl-$SO_2$-(2-4C)-alkenyl und -$SO_cR^2$ (wobei c für 0, 1 oder 2 steht) .

7. Verbindungen der Formel (1) und deren pharmazeutisch annehmbare Salze und in vivo hydrolysierbare Ester nach einem der vorhergehenden Ansprüche, wobei $R^2$ und $R^3$ unabhängig voneinander ausgewählt sind aus Wasserstoff, Heterocyclyl, -O-(1-4C)-Alkyl, -N-(1-4C)-Alkyl, (1-4C)-Alkyl [gegebenenfalls substitiuert durch 1 oder 2 $R^8$-Gruppen]; oder eine N$R^2R^3$-Gruppe einen Morpholin-, Thiomorpholin-(und oxidierte Versionen davon), Pyrrolidin- oder Piperidinring bildet und wobei der Ring gegebenenfalls durch 1 oder 2 Substituenten unabhängig voneinander ausgewählt aus Chlor, Fluor, Hydroxy und Methoxy substituiert ist.

8. Verbindungen der Formel (1) und deren pharmazeutisch annehmbare Salze und in vivo hydrolysierbare Ester nach einem der vorhergehenden Ansprüche, wobei
$R^8$ unabhängig ausgewählt ist aus Wasserstoff, Hydroxy, -C(O)N($R^9$) ($R^{10}$), -NHC(O)$R^9$, -COO$R^9$, -$CH_2$O$R^9$, -$CH_2$ COO$R^9$, $CH_2$OCO$R^9$, Aryl, Heterocyclyl und 5- und 6-gliedrigen cyclischen Acetalen und Mono- und Dimethylderivaten davon.

9. Verbindungen der Formel (1) und deren pharmazeutisch annehmbare Salze und in vivo hydrolysierbare Ester nach einem der vorhergehenden Ansprüche, wobei
$R^9$ und $R^{10}$ unabhängig voneinander ausgewählt sind aus Wasserstoff, Hydroxy und (1-4C)-Alkyl oder $R^9$ und $R^{10}$ zusammen mit dem Stickstoff, an dem sie gebunden sind, einen Morpholin-, Thiomorpholin- (und oxidierte Versionen davon), Pyrrolidin- oder Piperidinring bilden.

10. Pharmazeutische Zusammensetzung, enthaltend eine Verbindung der Formel (1) oder ein pharmazeutisch annehmbares Salz oder einen in vivo hydrolysierbaren Ester davon nach Anspruch 1 in Assoziation mit einem pharmazeutisch annehmbaren Verdünnungsmittel oder einem pharmazeutisch annehmbaren Träger.

11. Verbindungen der Formel (1) und deren pharmazeutisch annehmbare Salze und in vivo hydrolysierbare Ester nach

Anspruch 1 zur Verwendung zur therapeutischen Behandlung eines Warmblüters wie dem Menschen.

**12.** Verbindungen der Formel (1) und deren pharmazeutisch annehmbare Salze und in vivo hydrolysierbare Ester nach Anspruch 1 zur Verwendung als Medikament.

**13.** Verbindungen der Formel (1) und deren pharmazeutisch annehmbare Salze und in vivo hydrolysierbare Ester nach Anspruch 1 zur Verwendung als Medikament bei der Behandlung von Typ-2-Diabetes, Insulinresistenz, Syndrom X, Hyperinsulinämie, Hyperglucagonämie, kardialer Ischämie oder Obesitas in einem Warmblüter wie dem Menschen.

**14.** Verwendung einer Verbindung der Formel (1) oder eines pharmazeutisch annehmbaren Salzes oder eines in vivo hydrolysierbaren Esters davon nach Anspruch 1 bei der Herstellung eines Medikaments zur Verwendung bei der Behandlung von Typ-2-Diabetes, Insulinresistenz, Syndrom X, Hyperinsulinämie, Hyperglucagonämie, kardialer Ischämie oder Obesitas in einem Warmblüter wie dem Menschen.

**15.** Verwendung einer Verbindung der Formel (1) oder eines pharmazeutisch annehmbaren Salzes oder eines in vivo hydrolysierbaren Esters davon nach Anspruch 1 bei der Herstellung eines Medikaments zur Verwendung bei der Behandlung von Typ-2-Diabetes in einem Warmblüter wie dem Menschen.

**16.** Verfahren zur Herstellung einer Verbindung der Formel (1) nach Anspruch 1, bei dem man:

eine Säure der Formel (2):

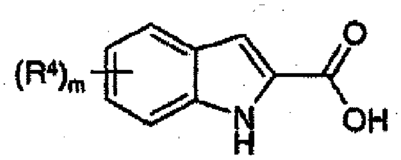

(2)

oder ein aktiviertes Derivat davon mit einem Amin der Formel (3):

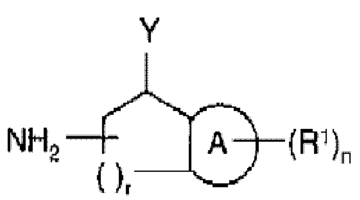

(3)

umsetzt und anschließend, falls erforderlich:

i) eine Verbindung der Formel (1) in eine andere Verbindung der Formel (1) umwandelt;
ii) gegebenenfalls vorhandene Schutzgruppen entfernt;
iii) ein pharmazeutisch annehmbares Salz oder einen in vivo hydrolysierbaren Ester bildet.

**Revendications**

**1.** Composé de formule (I) :

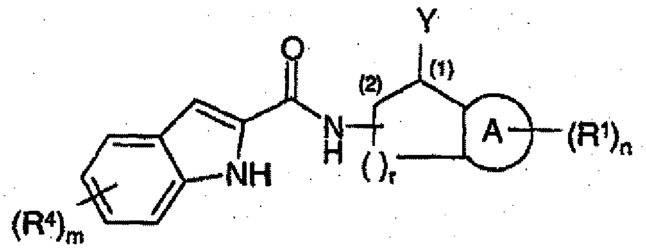

(1)

dans laquelle :

A est phénylène ou hétéroarylène ;
n vaut 0, 1 ou 2 ;
m vaut 0, 1 ou 2 ;
$R^1$ est choisi indépendamment parmi halogéno, nitro, cyano, hydroxy, carboxy, carbamoyle, N-(C1-4)-alkylcarbamoyle, N,N-((C1-4)alkyl)$_2$carbamoyle, sulfamoyle, N-(C1-4)alkylsulfamoyle, N,N-((C1-4)-alkyl)$_2$sulfamoyle, -S(O)$_b$(C1-4)alkyle (où b vaut 0, 1, ou 2), -OS(O)$_2$(C1-4)alkyle, (C1-4)alkyle, (C2-4)alcényle, (C2-4)alcynyle, (C1-4)alcoxy, (C1-4)alcanoyle, (C1-4)alcanoyloxy, hydroxy(C1-4)alkyle, fluorométhyle, difluorométhyle, trifluorométhyle, trifluorométhoxy et -NHSO$_2$(C1-4)alkyle ;
ou, lorsque n vaut 2, les deux groupements $R^1$, conjointement avec les atomes de carbone de A auxquels ils sont fixés, peuvent former un cycle de 4 à 7 chaînons saturé, contenant éventuellement 1 ou 2 hétéroatomes choisis indépendamment parmi 0, S et N, et étant éventuellement substitué par un ou deux groupements méthyle ;
$R^4$ est choisi indépendamment parmi halogéno, nitro, cyano, hydroxy, fluorométhyle, difluorométhyle, trifluorométhyle, trifluorométhoxy, carboxy, carbamoyle, (C1-4)alkyle, (C2-4)alcényle, (C2-4)alcynyle, (C1-4)alcoxy et (C1-4)alcanoyle ;
r vaut 1 ou 2 ; et
lorsque r vaut 1, le groupement

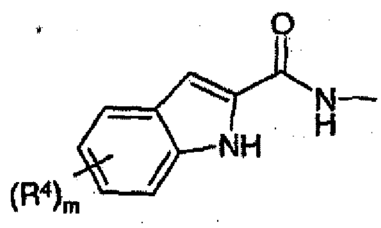

est un substituant sur le carbone (2) et
lorsque r vaut 2 (formant ainsi un cycle à six chaînons), le même groupement est un substituant sur le carbone (2) ou sur le carbone (3) ;
Y est choisi parmi -C(O)$R^2$, -C(O)O$R^2$, -C(O)N$R^2R^3$, -(C1-4)alkyle [éventuellement substitué par 1 ou 2 substituants choisis indépendamment parmi hydroxy, -C=N$R^2$, (C1-4)alcoxy, aryloxy, hétérocyclyloxy, -S(O)$_b R^2$ (où b vaut 0, 1 ou 2), -O-S(O)$_b R^2$ (où b vaut 0, 1 ou 2), -N$R^2R^3$, -N(OH)$R^2$, -N$R^2$C(=O)$R^2$, -NHOHC(=O)$R^2$, -SO$_2$N$R^2R^3$, -N($R^2$) SO$_2R^2$, aryle et hétérocyclyle], -C(O)NOH, -C(O)NSH, -C(N)OH, -C(N)SH, -SO$_2$H, -SO$_3$H, -SO$_2$N(OH)$R^2$, -(C2-4)alcényle, -SO$_2$N$R^2R^3$,-(C1-4)alkylC(O)$R^2$,-(C1-4)alkylC(O)O$R^2$, -(C1-4)alkylSC(O)$R^2$, -(C1-4)alkylOC(O)$R^2$, -(C1-4)alkylC(O)N$R^2R^3$, -(C1-4) alkylOC(O)O$R^2$, -(C1-4)alkylN($R^2$) C(O)O$R^2$, -(C1-4) alkylN($R^2$)C(O)N$R^2R^3$, -(C1-4)alkylOC(O)N$R^2R^3$, (C3-6) cycloalkyle (éventuellement substitué par 1 ou 2 $R^8$), aryle, hétérocyclyle (où le cycle hétérocyclique est lié par un atome de carbone de cycle), -(C1-4)alkylSO$_2$ (C2-4)alcényle et -S(O)$_c R^2$ (où C vaut 0, 1 ou 2) ;
$R^2$ et $R^3$ sont choisis indépendamment parmi hydrogène, -O(C1-4)alkyle, -S(C1-4)alkyle, -N(C1-4) alkyle, hétérocyclyle, aryle, et (C1-4)alkyle [éventuellement substitué par 1 ou 2 groupements $R^8$] ; ou
où N$R^2R^3$ peut former un cycle de 4 à 7 chaînons saturé, partiellement saturé ou insaturé, contenant éventuellement 1, 2 ou 3 hétéroatomes supplémentaires choisis indépendamment parmi N, O et S (à condition qu'il n'y ait aucune liaison O-O, O-S ou S-S), où tout -CH$_2$- peut éventuellement être remplacé par -C(=O)-, et tout

atome de N ou S peut éventuellement être oxydé pour former un N-oxyde ou un groupement SO ou $SO_2$ respectivement, et où le cycle est éventuellement substitué par 1 ou 2 substituants choisis indépendamment parmi halogéno, cyano, (C1-4)alkyle, hydroxy, (C1-4)alcoxy et (C1-4)alkyl$S(O)_b$- (où b vaut 0, 1 ou 2) ;

$R^8$ est choisi indépendamment parmi hydrogène, hydroxy, (C1-4)alkyle, (C2-4)alcényle, (C1-4)alcoxy, cyano (C1-4)alkyle, amino(C1-4)alkyle [éventuellement substitué sur l'azote par 1 ou 2 groupements choisis parmi (C1-4)alkyle, hydroxy, hydroxy(C1-4)alkyle, dihydroxy(C1-4)alkyle, $-CO_2$(C1-4)alkyle, aryle et aryl(C1-4)alkyle], halogéno(C1-4)alkyle, dihalogéno(C1-4)alkyle, tri-halogéno(C1-4)alkyle, hydroxy(C1-4)alkyle, dihydroxy-(C1-4)alkyle, (C1-4)alcoxy(C1-4)alcoxy, (C1-4)alcoxy-(C1-4)alkyle, hydroxy(C1-4)alcoxy, des acétals cycliques de 5 et 6 chaînons et des dérivés mono- et di-méthyliques de ceux-ci, aryle, hétérocyclyle, (hétérocyclyl) (C1-4)alkyle, (C3-7)cycloalkyle (éventuellement substitué par 1 ou 2 groupements hydroxy, (C1-4)alkyle ou $-CO_2$(C1-4)alkyle), (C1-4)alcanoyle, (C1-4)alkyl$S(O)_b$- (où b vaut 0, 1 ou 2), (3-6C)cycloalkyl$S(O)_b$- (où b vaut 0, 1 ou 2), aryl$S(O)_b$-(où b vaut 0, 1 ou 2), hétérocyclyl$S(O)_b$- (où b vaut 0, 1 ou 2), benzyl$S(O)_b$- (où b vaut 0, 1 ou 2), (C1-4)alkyl$S(O)_c$(C1-4)alkyl- (où c vaut 0, 1 ou 2), -N(OH)CHO, $-C(=N-OH)NH_2$, -C(=N-OH)NH(C1-4) alkyle, $-C(=N-OH)N((C1\text{-}4)alkyl)_2$, -C(=N-OH)NH(C3-6)cycloalkyle, $-C(=N-OH)N((C3\text{-}6)cycloalkyl)_2$, $-COCOOR^9$, $-C(O)N(R^9)(R^{10})$, $-NHC(O)R^9$, $-C(O)NHSO_2$(C1-4)alkyle, $-NHSO_2R^9$, $(R^9)$ $(R^{10})NSO_2$-, $-COCH_2OR^{11}$, $-COCH_2OH$, $(R^9)(R^{10})N$-, $-COOR^9$, $-CH_2OR^9$, $-CH_2COOR^9$, $-CH_2OCOR^9$, $-CH_2CH(CO_2R)OH$, - $CH_2C(O)NR^9R^{10}$, $-(CH_2)_wCH(NR^9R^{10})CO_2R^{9'}$ (où w vaut 1, 2 ou 3), et $-(CH_2)_wCH(NR^9R^{10})CO(NR^{9'}R^{10'})$ (où w vaut 1, 2 ou 3) ;

$R^9$, $R^9$, $R^{10}$ et $R^{10'}$ sont choisis indépendamment parmi hydrogène, hydroxy, (C1-4)alkyle (éventuellement substitué par 1 ou 2 $R^{11}$) , (C2-4)alcényle, (C3-7)cycloalkyle (éventuellement substitué par 1 ou 2 groupements hydroxy), cyano(C1-4)alkyle, trihalogéno-(C1-4)alkyle, aryle, hétérocyclyle, hétérocyclyl(C1-4)-alkyle, $-CO_2$ (C1-4)alkyle ; ou

$R^9$ et $R^{10}$, conjointement avec l'azote auxquels ils sont fixés, et/ou $R^{9'}$ et $R^{10'}$, conjointement avec l'azote auxquels ils sont fixés, forment un cycle de 4 à 6 chaînons, où le cycle est éventuellement substitué sur le carbone par 1 ou 2 substituants choisis indépendamment parmi oxo, hydroxy, carboxy, halogéno, nitro, cyano, carbonyle, (C1-4)alcoxy et hétérocyclyle ; ou le cycle peut éventuellement être substitué sur deux carbones adjacents par $-O-CH_2-O-$ pour former un acétal cyclique où l'un des hydrogènes, ou les deux, du groupement $-O-CH_2-O-$ peuvent être remplacés par méthyle ;

$R^{11}$ est choisi indépendamment parmi (C1-4)alkyle et hydroxy(C1-4)alkyle ;

ou un sel pharmaceutiquement acceptable de celui-ci.

**2.** Composé de formule (1), ou un sel pharmaceutiquement acceptable de celui-ci, selon la revendication 1, dans lequel A est phénylène.

**3.** Composé de formule (1), ou un sel pharmaceutiquement acceptable ou un ester hydrolysable *in vivo* de celui-ci, selon la revendication 1 ou la revendication 2, dans lequel n vaut 0.

**4.** Composé de formule (1), ou un sel pharmaceutiquement acceptable ou un ester hydrolysable *in* vivo de celui-ci, selon l'une quelconque des revendications précédentes, dans lequel r vaut 1.

**5.** Composé de formule (1), ou un sel pharmaceutiquement acceptable ou un ester hydrolysable *in vivo* de celui-ci, selon l'une quelconque des revendications précédentes, dans lequel m est 1.

**6.** Composé de formule (1), ou un sel pharmaceutiquement acceptable ou un ester hydrolysable *in vivo* de celui-ci, selon l'une quelconque des revendications précédentes, dans lequel Y est choisi parmi $-C(O)OR^2$ $-C(O)NR^2R^3$, -(C1-4 )alkyle [éventuellement substitué par un substituant choisi parmi hydroxy, (C1-4)alcoxy, $-S(O)_bR^2$ (où b vaut 0, 1 ou 2), $-O-S(O)_bR^2$(où b vaut 0, 1 ou 2), $-NR^2R^3$, $-NR^2C(=O)R^2$ et $-SO_2NR^2R^3$] , -(C1-4)alkyl$C(O)R^2$, -(C1-4) alkyl$C(O)OR^2$, -(C1-4)alkylo$C(O)R^2$, -(C1-4)alkyl$C(O)NR^2R^3$, -(C1-4)alkyl$OC(O)OR^2$, -(C1-4)alkyl$N(R^2)C(O)OR^2$, -(C1-4)alkyl-$N(R^2)C(O)NR^2R^3$, -(C1-4)alkyl$SC(O)R^2$, -(C1-4)alkyl-$OC(O)NR^2R^3$, -(C1-4)alkyl$SO_2(C_2$-4)alcényle et $-SO_cR^2$ (où C vaut 0, 1 ou 2).

**7.** Composé de formule (1), ou un sel pharmaceutiquement acceptable ou un ester hydrolysable *in vivo* de celui-ci, selon l'une quelconque des revendications précédentes, dans lequel $R^2$ et $R^3$ sont choisis indépendamment parmi hydrogène, hétérocyclyle, -O(C1-4)alkyle, -N(C1-4)alkyle, (C1-4)alkyle [éventuellement substitué par 1 ou 2 groupements $R^8$] ; ou un groupement $NR^2R^3$ forme un cycle morpholine, thiomorpholine (et leurs versions oxydées), pyrrolidine ou pipéridine, et dans lequel le cycle est éventuellement substitué par 1 ou 2 substituants choisis indépendamment parmi chloro, fluoro, hydroxy et méthoxy.

**8.** Composé de formule (1), ou un sel pharmaceutiquement acceptable ou un ester hydrolysable *in vivo* de celui-ci, selon l'une quelconque des revendications précédentes, dans lequel $R^8$ est choisi indépendamment parmi hydrogène, hydroxy, $-C(O)N(R^9)(R^{10})$, $-NHC(O)R^9$, $-COOR^9$, $-CH_2OR^9$, $-CH_2COOR^9$, $-CH_2OCOR^9$, aryle, hétérocyclyle, et des acétals cycliques à 5 et 6 chaînons et les dérivés diméthyliques de ceux-ci.

**9.** Composé de formule (1), ou un sel pharmaceutiquement acceptable ou un ester hydrolysable *in vivo* de celui-ci, selon l'une quelconque des revendications précédentes, dans lequel $R^9$ et $R^{10}$ sont choisis indépendamment parmi hydrogène, hydroxy et (C1-4)alkyle ou $R^9$ et $R^{10}$, conjointement avec l'azote auxquels ils sont fixés, forment un cycle morpholine, thiomorpholine (et leurs versions oxydées), pyrrolidine ou pipéridine.

**10.** Composition pharmaceutique comprenant un composé de formule (1), ou un sel pharmaceutiquement acceptable ou un ester hydrolysable *in vivo* de celui-ci, selon la revendication 1, en association avec un diluant ou véhicule pharmaceutiquement acceptable.

**11.** Composé de formule (1), ou un sel pharmaceutiquement acceptable ou un ester hydrolysable *in vivo* de celui-ci, selon la revendication 1, pour une utilisation dans une méthode de traitement d'un animal à sang chaud, tel que l'homme, par thérapie.

**12.** Composé de formule (1), ou un sel pharmaceutiquement acceptable ou un ester hydrolysable *in vivo* de celui-ci, selon la revendication 1, pour une utilisation comme médicament.

**13.** Composé de formule (1), ou un sel pharmaceutiquement acceptable ou un ester hydrolysable *in vivo* de celui-ci, selon la revendication 1, pour une utilisation comme médicament dans le traitement du diabète de type 2, de l'insulinorésistance, du syndrome X, de l'hyperinsulinémie, de l'hyperglucagonémie, de l'ischémie cardiaque ou de l'obésité chez un animal à sang chaud, tel que l'homme.

**14.** Utilisation d'un composé de formule (1), ou d'un sel pharmaceutiquement acceptable ou un ester hydrolysable *in vivo* de celui-ci, selon la revendication 1, dans la fabrication d'un médicament destiné à être utilisé dans le traitement du diabète de type 2, de l'insulinorésistance, du syndrome X, de l'hyperinsulinémie, de l'hyperglucagonémie, de l'ischémie cardiaque ou de l'obésité chez un animal à sang chaud, tel que l'homme.

**15.** Utilisation d'un composé de formule (1), ou d'un sel pharmaceutiquement acceptable ou un ester hydrolysable *in vivo* de celui-ci, selon la revendication 1, dans la fabrication d'un médicament destiné à être utilisé dans le traitement du diabète de type 2 chez un animal à sang chaud, tel que l'homme.

**16.** Procédé de préparation d'un composé de formule (1) selon la revendication 1, lequel procédé comprend :

la réaction d'un acide de formule (2) :

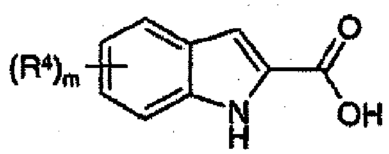

(2)

ou d'un dérivé activé de celui-ci ; avec une amine de formule (3) :

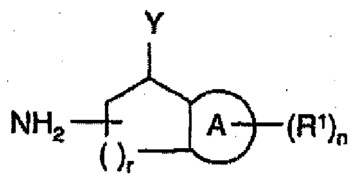

(3)

puis, si nécessaire :

i) la conversion d'un composé de formule (1) en un autre composé de formule (1) ;
ii) l'élimination de tout groupement protecteur ;
iii) la formation d'un sel pharmaceutiquement acceptable ou d'un ester hydrolysable *in vivo.*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0846464 A2

### Non-patent literature cited in the description


- **WEYER et al.** *J Clin Invest,* 1999, vol. 104, 787-794
- **CLORE ; BLACKGARD.** *Diabetes,* 1994, vol. 43, 256-262
- **DE FRONZO, R. A. et al.** *Diabetes Care,* 1992, vol. 15, 318-355
- **REAVEN, G.M.** *Diabetologia,* 1995, vol. 38, 3-13
- **CHARLES, M.A. et al.** *Lancet,* 1996, vol. 348, 1657-1658
- **COUTINHO, M. et al.** *Diabetes Care,* 1999, vol. 22, 233-240
- **SHAW, J.E. et al.** *Diabetes Care,* 2000, vol. 23, 34-39
- DCCT Research Group. *New. Eng. J. Med.,* 1993, vol. 329, 977-986
- **HELLERSTEIN et al.** *Am J Physiol,* 1997, vol. 272, E163
- **AISTON S et al.** *Diabetalogia,* 2000, vol. 43, 589-597
- **HOOVER et al.** *J Med Chem,* 1998, vol. 41, 2934-8
- **MARTIN et al.** *PNAS,* 1998, vol. 95, 1776-81
- **SHIOTA et al.** *Am J Physiol,* 1997, vol. 273, E868
- Design of Prodrugs. Elsevier, 1985
- Methods in Enzymology. Academic Press, 1985, vol. 42, 309-396
- A Textbook of Drug Design and Development. **H. BUNDGAARD.** Design and Application of Prodrugs. 1991, 113-191
- **H. BUNDGAARD.** *Advanced Drug Delivery Reviews,* 1992, vol. 8, 1-38
- **H. BUNDGAARD et al.** *Journal of Pharmaceutical Sciences,* 1988, vol. 77, 285
- **N. KAKEYA et al.** *Chem Pharm Bull,* 1984, vol. 32, 692
- **T.W. GREEN.** Protective Groups in Organic Synthesis. John Wiley and Sons, 1991
- **PESCE, M A ; BODOURIAN, S H ; HARRIS, R C ; NICHOLSON, J F.** *Clinical Chemistry,* 1977, vol. 23, 1171-1717
- **P.O. SEGLEN.** *Methods Cell Biology,* 1976, vol. 13, 29-83
- Comprehensive Medicinal Chemistry. Pergamon Press, 1990, vol. 5
- **DIDIER, E et al.** *Tetrahedron,* 1991, vol. 47 (27), 4941-4958